(19) Europäisches Patentamt
European Patent Office
Office européen des brevets

(11) Publication number: **0 010 317**
**B1**

(12) **EUROPEAN PATENT SPECIFICATION**

(45) Date of publication of patent specification: **21.12.83**

(21) Application number: **79104106.4**

(22) Date of filing: **23.10.79**

(51) Int. Cl.³: **C 07 D 487/04,**
A 61 K 31/40 //C07D205/08,
C07D498/04, C07F9/65,
C07F7/18, C07D405/14,
C07C69/007, C07C79/22,
C07C149/24, (C07D487/04,
209/00, 205/00)

(54) **6-, 1- and 2-substituted-1-carbapen-2-em-3-carboxylic acids, processes for the preparation of such compounds and pharmaceutical composition comprising such compounds.**

(30) Priority: **24.10.78 US 954273**
**24.10.78 US 954274**
**24.10.78 US 954270**
**24.10.78 US 954272**

(43) Date of publication of application:
**30.04.80 Bulletin 80/9**

(45) Publication of the grant of the patent:
**21.12.83 Bulletin 83/51**

(84) Designated Contracting States:
**AT BE CH DE FR GB IT LU NL SE**

(56) References cited:
**EP - A - 0 001 628**
**LU - A - 79 677**
**US - A - 4 153 714**

(73) Proprietor: **MERCK & CO. INC.**
**126, East Lincoln Avenue P.O. Box 2000**
**Rahway New Jersey 07065 (US)**

(72) Inventor: **Christensen, Burton G.**
**195 Watchung Terrace**
**Scotch Plains, N.J. 07076 (US)**
Inventor: **Shih, David H.**
**9 Hazel Avenue**
**Edison, N.J. 08817 (US)**

(74) Representative: **Abitz, Walter, Dr.-Ing. et al,**
**Abitz, Morf, Gritschneder P.O. Box 86 01 09**
**D-8000 München 86 (DE)**

**EP 0 010 317 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# 6-, 1- and 2-substituted-1-carbapen-2-em-3-carboxylic acids, processes for the preparation of such compounds and pharmaceutical composition comprising such compounds

## BACKGROUND OF THE INVENTION

This invention relates to 6-, 1- and 2-substituted-1-carbapen-2-em-3-carboxylic acids and derivatives thereof which are useful as antibiotics and which may be represented by the following generic structural formula (I):

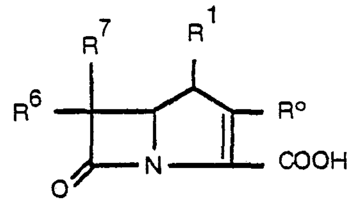

wherein: R° is H or —$SR^8$; $R^1$, $R^6$, $R^7$ and $R^8$ are indpendently selected from hydrogen ($R^1$ is not hydrogen), substituted and unsubstituted: alkyl, alkenyl, and alkynyl, having from 1—10 carbon atoms; cycloalkyl, cycloalkylalkyl, and alkylcycloalkyl, having 3—6 carbon atoms in the cycloalkyl ring and 1—6 carbon atoms in the alkyl moieties; phenyl; aralkyl, aralkenyl, and aralkynyl wherein the aryl moiety is phenyl and the linear chain has 1—6 carbon atoms; heterocyclyl and heterocyclylalkyl; wherein the substituent or substituents relative to the above-named radicals are selected from: amino, mono, di- and trialkylamino, hydroxyl, alkoxyl, mercapto, alkylthio, phenylthio, sulfamoyl, amidino, guanidino, nitro, chloro, bromo, fluoro, cyano and carboxy; and wherein the hetero atom or atoms in the above-named heterocyclic moieties are selected from 1—4 oxygen, nitrogen or sulphur atoms; and wherein the alkyl moieties of the above-recited substituents have 1—6 carbon atoms.

This invention also relates to the carboxyl derivatives of I which are antibiotics and which may be represented by the following generic structure (I):

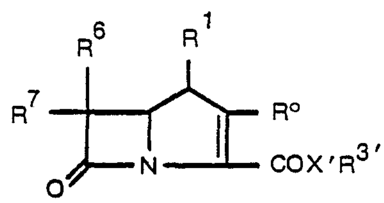

wherein X′ is oxygen; and $R^{3'}$ is, selected from hydrogen, pharmaceutically acceptable salt and ester moieties known in the bicyclic β-lactam antibiotic art.

This invention also relates to processes for the preparation of such compounds (I) and pharmaceutical compositions comprising such compounds.

Preferred compounds of formula I are those wherein $R^6$ is H or methyl and $R^7$ is alkyl, phenyl, aralkyl or hydroxyl-substituted alkyl, phenyl or aralkyl.

Further preferred compounds of formula I are those wherein: $R^6$ is hydrogen or methyl and $R^8$ is selected from:

H, $CH_3$, $(CH_2)_2NH_2$, $C(CH_3)_2CH_2NH_2$, 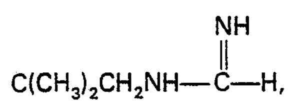, 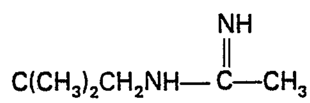

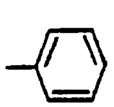, 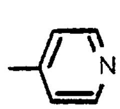, 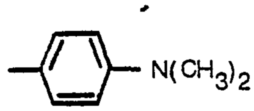 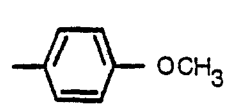 $CH_2CH_2CH_2NH_2$,

$CH_2CH(CH_3)NH_2$, 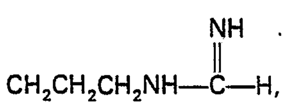, 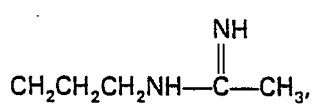, 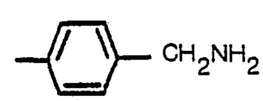

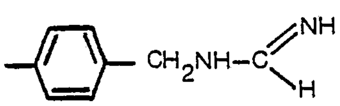 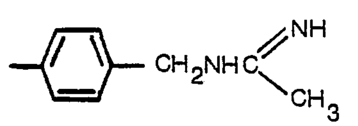 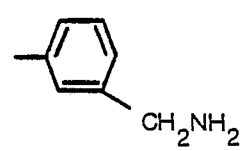

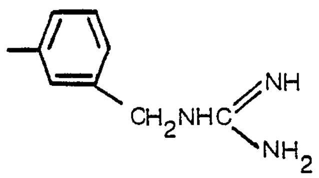 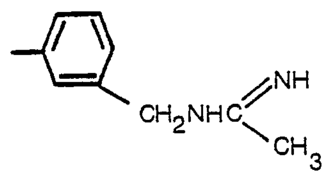 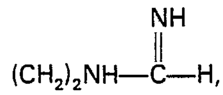, 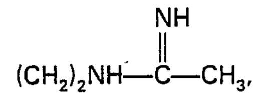,

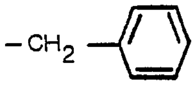 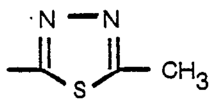 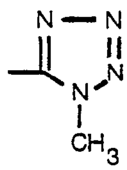 $CH(CH_3)CH_2NH_2$,

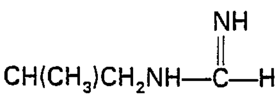 or 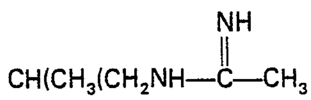

and $R^7$ is selected from: $—CH_2OH$ 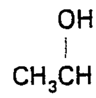 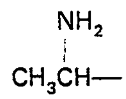 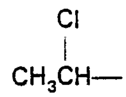 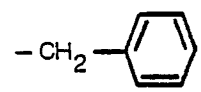

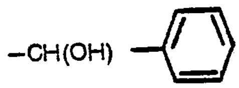 $—CH_3$ $—CH_2CH_3$ 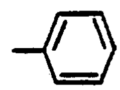

There is a continuing need for new antibiotics. For unfortunately, there is no static effectiveness of any given antibiotic because continued wide scale usage selectively gives rise to resistant strains of pathogens. In addition, the known antibiotics suffer from the disadvantage of being effective only against certain types of microorganisms. Accordingly the search for new antibiotics continues.

Thus, it is an object of the present invention to provide a novel class of antibiotics which are useful in animal and human therapy and in inanimate systems. These antibiotics are active against a broad range of pathogens which representatively include both gram positive bacteria such as *S. aureua, Strep, pyogenes,* and *B. subtilis,* and gram negative bacteria such as *E. coli, Pseudomonas,* Proteus morganii, Serratia, and *Klebsiella.* Further objects of this invention are to provide chemical processes for the preparation of such antibiotics and their non-toxic pharmaceutically acceptable salts and pharmaceutical compositions comprising such antibiotics.

1-Corbapen-2-em-3-carboxylic acids are disclosed by DE—A—2 751 624, however nothing in this prior-art would suggest the present 1-substituted derivatives.

DETAILED DESCRIPTION OF THE INVENTION

The compounds of the present invention (I, above) are conveniently prepared by the following scheme:

DIAGRAM I

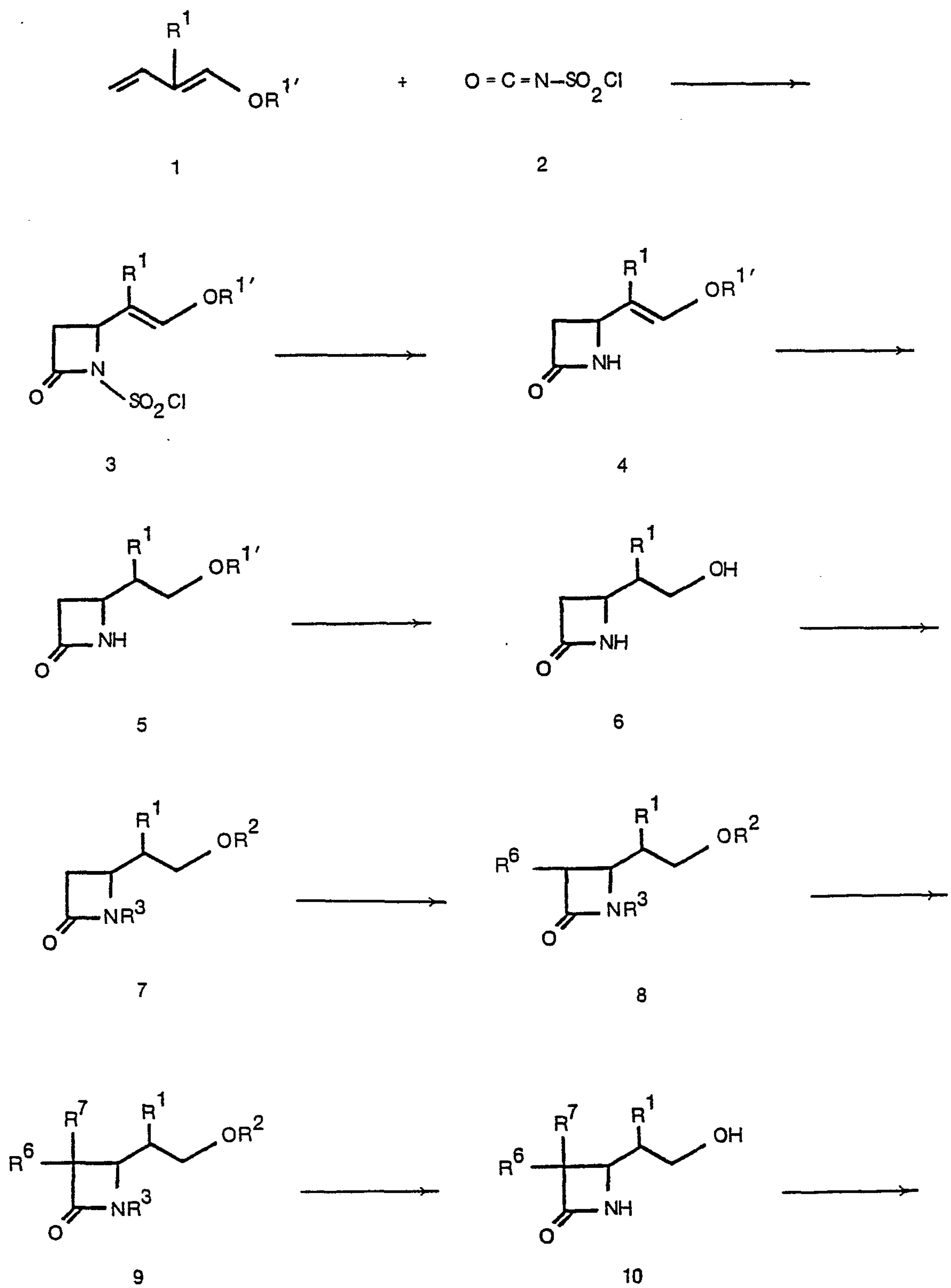

1

2

3

4

5

6

7

8

9

10

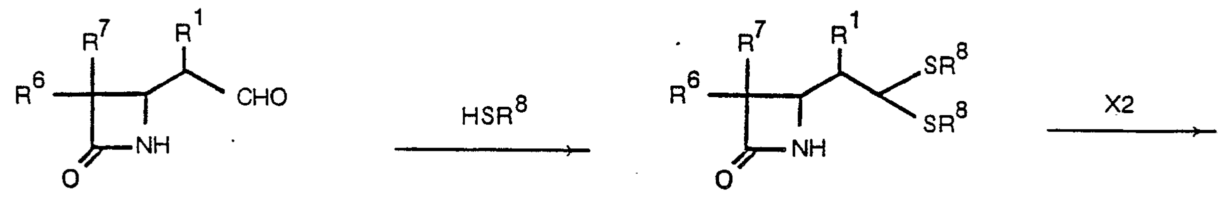


11 12

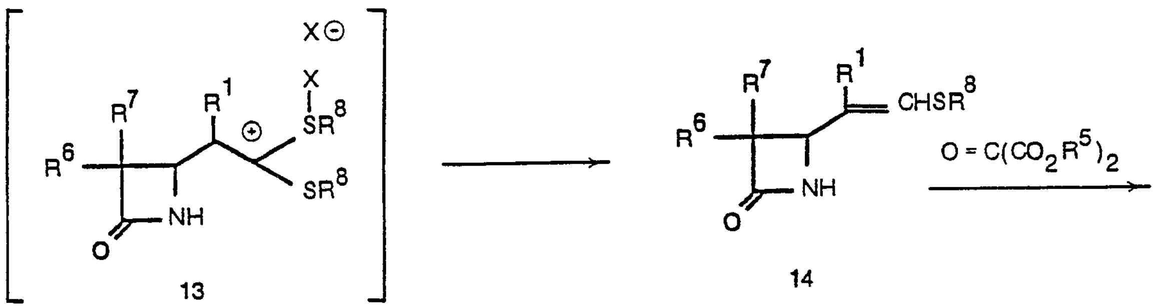


13 14

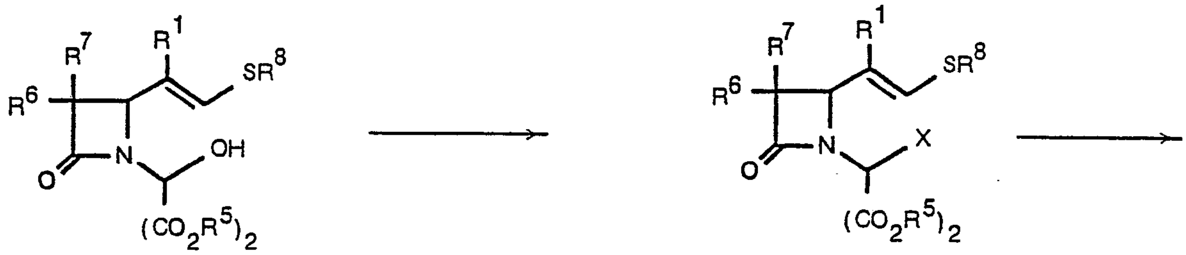

15 16

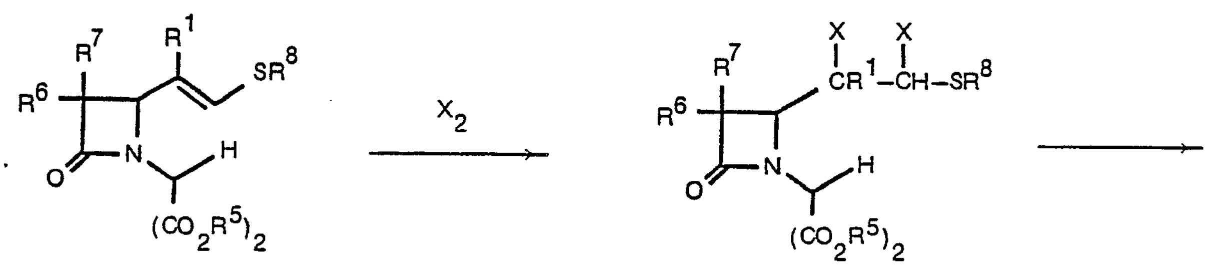


17 18

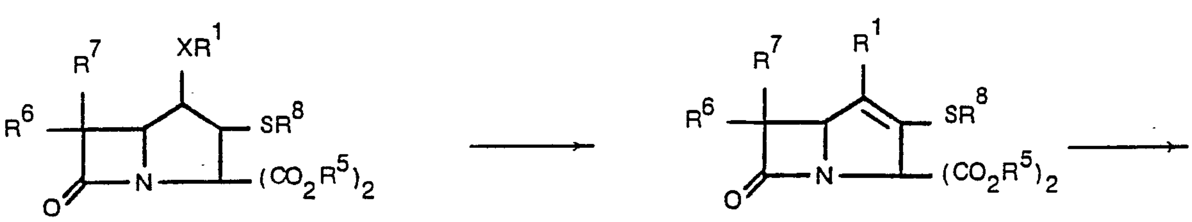

19 20

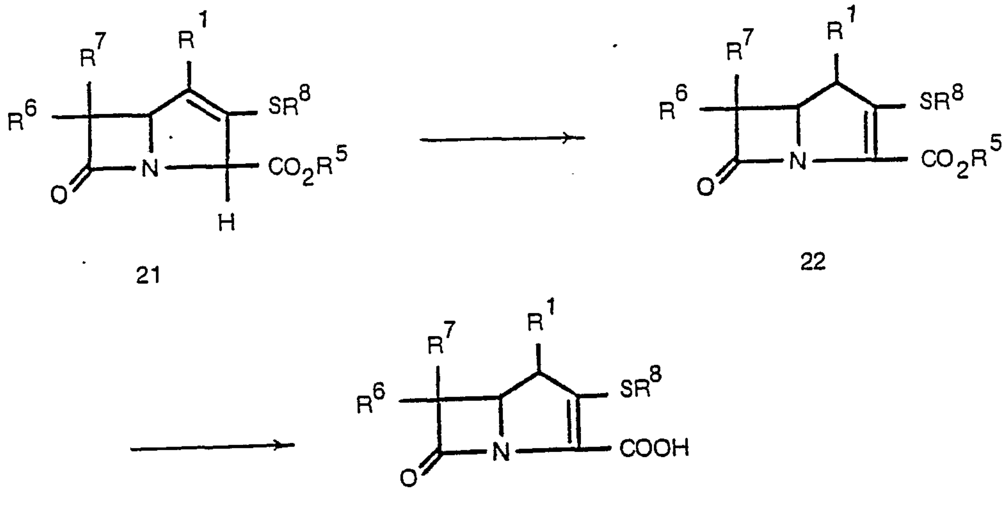

21

22

1

In words relative to the above diagram, the 4-(1,2-substituted-vinyl)azetidine-2-one, 4 starting material is prepared by reacting an $R^1$- and $R^{1'}$-substituted-oxybutadiene, 1 with chlorosulfonylisocyanate 2. The rection is conducted without solvent or may be run in solvent such as diethyl ether, ethyl acetate, chloroform or methylene chloride; at a temperature of from −78°C to 25°C for from a few minutes to 1 hour to provide 3. The radical $R^{1'}$ is an easily removable acyl blocking group such as an alkanoyl or aralkanoyl which bears no functional group or groups which might interfere with the desired course of reaction (1 + 2 → 3 → 4). Intermediate species 3 is converted to the sulfinamide by reduction which is then hydrolyzed to 4 at pH 6—8. Typically the reaction solution comprising 3 is contacted (5—30 minutes) with an aqueous solution (at 0—25°C) of a reducing agent such as sodium sulfite or thiophenol, at pH 6—8 to provide 4.

The reaction 4 → 5 is a reduction, and is preferably achieved by hydrogenation in a solvent such as ethyl acetate, ether, dioxane, tetrahydrofuran (THF) or ethanol, at 0 to 25°C for from 5 minutes to 2 hours under 1 to 10 atmospheres of hydrogen in the presence of a hydrogenation catalyst such as a platinum metal or oxide thereof such as 10% Pd/C.

The de-blocking reaction 5 → 6 is usually desirable when $R^{1'}$ is acyl to permit the later alkylation, 7 → 8. The preferred de-blocking procedure is by alcoholysis wherein the solvent is a lower alkanol such as methanol or ethanol, in the presence of the corresponding alkali metal alkoxide, such as sodium methoxide. Typically, the reaction is conducted for from 5 minutes to 1 hour at a temperature of from −10° to 25°C.

Blocking groups $R^3$ and $R^2$ are established (6 → 7) to provide a suitably protected species for alkylation (7 → 8 → 9). There is no criticality in the choice of blocking groups, provided only that they do not interfere with the intended alkylation. $R^3$ may be hydrogen, a triorganosilyl group such as trimethylsilyl or t-butyldimethylsilyl; or a cyclic ether such as 2-tetrahydropyranyl; $R^2$ may also be a cyclic ether such as 2-tetrahydropyranyl; alternatively, $R^3$ and $R^2$ may be joined together to form protected species such as 7a:

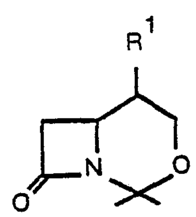

7a

For example, species such as 7a are conveniently prepared by treating 6 with 2,2-dimethoxypropane in the presence of a catalyst such as boron trifluoride etherate or, toluene sulphonic acid, in a solvent such as methylene chloride, ether, chloroform or dioxane at a temperature of from −10°C to 35°C for from a few minutes to 1 hour.

Species 7 can be mono- or dialkylated at ring position 6. Alkylation of 7 provides 8. Typically, 7 is treated with a strong base such as lithium diisopropylamide, sodium hydride, phenyl lithium or butyl lithium in a solvent such as tetrahydrofuran (THF), ether or dimethoxyethane at a temperature of from −80°C to 0°C., whereupon the alkylation agent of choice, $R^6X$, is added ($R^6$ is as described above and X is chloro, iodo or bromo; alternatively the alkylating agent may be $R^6$-tosylate, $R^6$-mesylate or an alde-

hyde or ketone such as acetaldehyde) to provide monoalkylated species 8. When desired dialkylated species 9 may be obtained from 8 by repeating the alkylating procedure 7 → 8.

The de-blocking reaction 9 → 10 is typically conducted by acid hydrolysis such as aqueous acetic acid at a temperature of from 25°C to 75°C for from 5 minutes to 3 hours.

The aldehyde intermediate 11 is prepared by treating 10 with an oxidizing agent such as $CrO_3$.2(pyridine) in $CH_3CN$, 1:1 mixture of dimethylsulfoxide and acetic anhydride, cyclohexylcarbodiimide in DMSO or pyridinium chlorochromate in $CH_2Cl_2$ at a temperature of from 0—25°C for from 5 minutes to 1 hour. The resulting species 11 in a solvent such as acetonitrile, methylene chloride or chloroform at a temperature of from −10 to 25°C is treated with an excess of the reagent $HSR^8$ in the presence of an acid catalyst such as boron trifluoride etherate or toluene sulphonic acid to provide 12. Typically, the reaction requires from 1 to 60 minutes.

The vinyl sulphide 14 is obtained *via* intermediate 13 by treating 12 with a halogen such as chlorine or bromine (X = Cl or Br) in a solvent such as ether, methylene chloride, tetrahydrofuran or glyme at a temperature of from −78° to 30°C for from 1 to 30 minutes followed immediately by treating with an olefin such as cyclohexene or isobutylene in the presence of base such as triethylamine, DBU or sodium hydride in a solvent such as DMF, glyme, THF, HMPA. The solution is held at −20° to 25°C for from 1 to 8 hours to yield 14.

The vinyl sulphide species 14 is reacted with a diester of oxomalonic acid (or its monohydrate) to provide 15. There is no criticality as to the identity of the ester moiety, $R^5$, of the oxomalonic acid. $R^5$ may be a conventional, easily removable blocking group or it may be a pharmaceutically acceptable ester moiety. Suitable ester radicals $R^5$ are *p*-nitrobenzyl, benzyl, *o*-nitrobenzyl, t-butyl, 2,2,2-trichloroethyl. The reaction 14 → 15 is typically conducted in a high boiling organic solvent such as benzene, toluene or cyclohexane at a temperature of from about 50°C to reflux for from 0.5 to 6 hours.

The halogenation reaction 15 → 16 is typically conducted in a solvent such as THF, glyme, ether, methylene chloride or chloroform in the presence of a halogenating agent such as thionyl chloride or phosphorus pentachloride in the presence of base such as pyridine at a temperature of from −20°C to 25°C for from 5 minutes to 3 hours. The selective reduction of 15 → 17 via 16 is completed by treating 16 with tributylphosphine, triphenylphosphine, or the like in aqueous DMF or similar aqueous systems involving dioxane, THF, glyme DMSO, or acetone at a temperature of from about 0—50°C for from 10 minutes to 5 hours.

Species 17 is halogenated by the previous procedure (12 → 13), but omitting the addition of the cyclohexene or other olefin, to provide the dihalo species 18. Species 18 is treated with a base such as triethylamine, sodium hydride or potassium hydride in a solvent such as DMF, acetonitrile, methylene chloride, chloroform or glyme at a temperature of from about −78° to 25°C for 1 to 5 hours to provide 19. Species 19 is converted to 20 on treatment with a strong base such as 1,5-diazabicyclo[5.4.0]-undec-5-ene(DBU) or 1,5-diazabicyclo[3.4.0]non-5-ene(DBN), in a solvent such as DMSO, acetone, chloroform, DMF, THF or glyme or on treatment with AgF in pyridine at a temperature of from 0—40°C for from 1/4 to 24 hours. The reaction 20 → 21 is conducted by treating 20 with an aromatic base such as pyridine, aqueous dimethylsulfoxide, s-collidine or lutidine, in the presence of a displacing agent such as lighium iodide, sodium chloride, lithium bromide or sodium bromide, at a temperature of from about 80—150°C for from 15 minutes to 2 hours. An aqueous work up to the resulting reaction mixture provides 21. Isomerization of the double bond 21 → 22 is accomplished by treating 21 in a solvent such as DMF, DMSO, ethyl ether, THF, glyme, methylene chloride with a strong base such as diisoprypylamine, DBU or DBN, at a temperature of from 0° to about 25°C for from a few minutes to 2 hours or until equilibrium has been established as determined by examination of sample aliquots by ultraviolet absorption or by thin layer chromatography. The final reaction 22 → I (hydrogenolysis of the blocking group) is accomplished by treating 22 in a solvent such as dioxane, ethanol or THF or an aqueous mixture thereof in the presence of a platinum metal catalyst such as Pd/C under a hydrogen pressure of from 1—4 atmospheres for from 0.5 to 8 hours at a temperature of from about 0—25°C.

In the foregoing description of the invention, suitable reagents $HSR^8$ (11 → 12) are representatively illustrated by the following list:

$HSCH_2CH_2CH_2NHCO_2PNB$, $PNBO_2CNHCH_2.CH_2CH_2SX$, 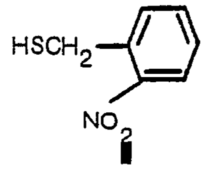

$HSC(CH_3)_2CH_2NHCO_2PNB$, HSØ, $HSCH_2Ø$, $HSC(CH_3)_3$, $HSCØ_3$, 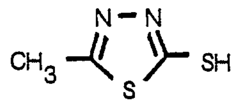

(Ø = phenyl; and PNB = p-nitrobenzyl), $CH_3SH$, $CH_3CH_2SH$, $CH_3(CH_2)_2SH$, $(CH_3)_2CHSH$,

$CH_3(CH_2)_3SH$, $(CH_3)_2CH(CH_2)_2SH$, $CH_2{\equiv}CHCH_2SH$, $CH{\equiv}CCH_2SH$, 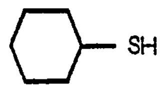

$Ø(CH_2)_3SH$(Ø = phenyl), $Ø(CH_2)_2SH$, $HO(CH_2)_2SH$, $H_2(CH_2)_2SH$, $H_2N(CH_2)_3SH$,

$CH_3(CH_2)_2NH(CH_2)_2SH$, 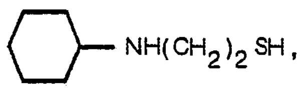 , $(CH_3)_2N(CH_2)_2SH$, $(CH_3CH_2)_2N(CH_2)_2SH$,

$HO_2C(CH_2)_2SH$, $ØCH_2SH$, 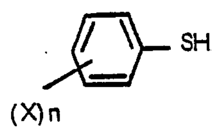

(n = 0, 1 or 2; X = Cl, Br, F, Cl, $OCH_3$, $CH_3NH_2$, $NH\overset{O}{\overset{\|}{C}}CH_3$). 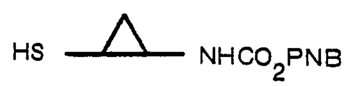

Similarly, suitable alkylating agents for establishing $R^6$ and/or $R^7$ at ring position 6 (7 → 8 → 9) are:

$ØCH_2CHO$, $ØCH_2CH_2CHO$, $CH_2O$, $CH_3I$, ØCH Br, $CH_3COCH_3$.

Relative to the compounds of the present invention I:

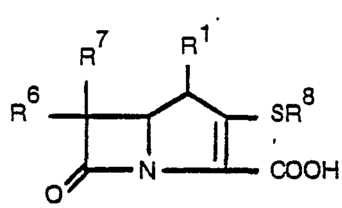

the most preferred values for $R^1$ include:

methyl,
ethyl,
propyl,
isopropyl,
cyclopropyl,
phenyl,
benzyl.

The most preferred radicals for $R^6$ and $R^7$ are: $R^6$ = H and $R^7$ is selected from hydroxymethyl, 1-hydroxyethyl, 1-hydroxypropyl, 2-hydroxypropyl, 2-hydroxyethyl; the most preferred values for $R^8$ are: aminoethylthio, aminopropylthio, aminocyclopropylthio, aminoisopropylthio, amidinoisopropylthio.

Especially preferred embodiments of the present invention are those wherein $R^1$ is selected from methyl, ethyl, propyl, isopropyl, cyclopropyl, phenyl, benzyl, and 2-bromoethyl.

The compounds of the present invention (I, above) wherein R° is H are conveniently prepared by the following scheme:

DIAGRAM II

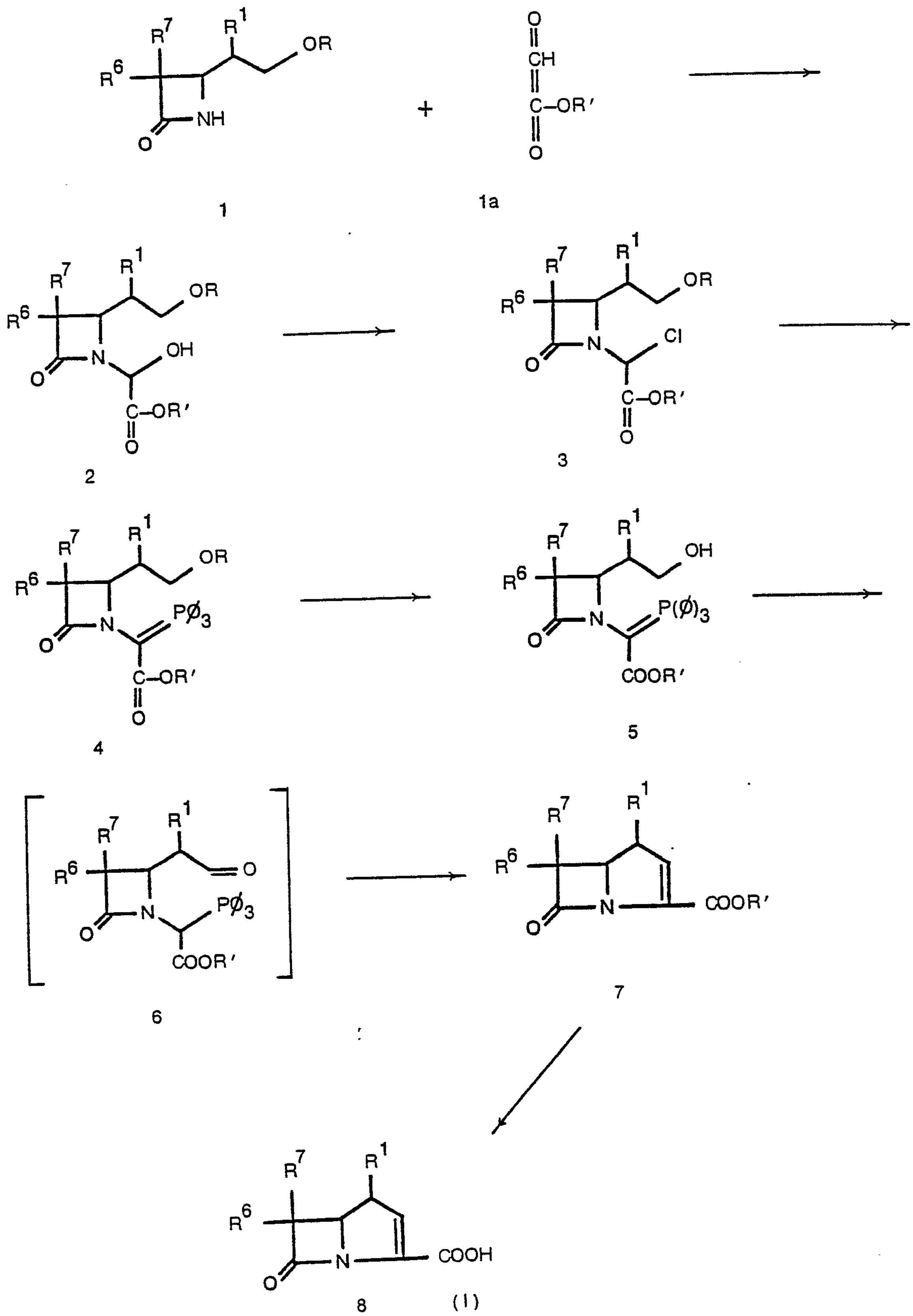

1 1a

2 3

4 5

6 7

8 (I)

wherein $R^6$, $R^7$ and $R^1$ are as hereinbefore defined;

R and R′ are readily removable blocking groups; R′ may also be a pharmaceutically acceptable ester moiety. Typically, the blocking group R is an acyl such as a lower alkanoyl or aralkylcarbonyl such as acetyl, bromo-*t*-butoxycarbonyl, benzyloxycarbonyl, formyl or trifluoroacetyl or a trialkylsilyl such as a trimethylsilyl or *t*-butyl dimethylsilyl group; and typically the blocking group R′ is substituted or unsubstituted alkyl, aralkyl or alkenyl, such as benzyl, *p*-nitrobenzyl, o-nitrobenzyl, pivaloyloxymethyl or bromo-*t*-butyl.

In words relative to the above reaction diagram, a suitably substituted azetidinone (1) is reacted with a glyoxylate ester such as benzyl glyoxylate to form the corresponding 1-(benzyloxycarbonylhydroxymethyl)azetidinone (2). The reaction 1 → 2 is conveniently carried out in a solvent such as benzene, toluene or xylene at a temperature of from about 25°C to reflux for from 2 to 10 hours. There is no criticality as to the precise identity of the solvent, provided only that it adequately solubilizes the reactants and be inert or substantially inert to the desired course of reaction. The halogenation reaction 2 → 3 may be conducted by any of a variety of well-known halogenation means. Suitable reagents include: $SOCl_2$, $POCl_3$ or oxalyl chloride. A preferred means of chlorination involves treating 2 in a solvent such as tetrahydrofuran (THF), ether or $CH_2Cl_2$ with thionylchloride in the presence of 1 to 2 equivalents (relative to the thionylchloride) of a base such as pyridine, triethylamine or quinoline. Typically, the reaction is conducted at a temperature of from −30 to 25°C for from 0.5 to 1 hour. The resulting 1-(benzyloxycarbonylchloromethyl)-azetidinone species, 3, is isolated, if desired, by conventional procedures for later reaction 3 → 4. The intermediate 4 is prepared from 3 by treating 3 in a solvent such as dimethylformamide (DMF), dimethylsulfoxide (DMSO), THF or dimethoxyethane (DME) with 1 to 1.5 equivalents of a phosphine such as triphenylphosphine, tributylphosphine, triethylphosphine, tris-(2-cyanoethyl)phosphine. Typically the reaction is conducted under a nitrogen atmosphere at a temperature of from −20° to 25°C., for from 0.5 to 2 hours. The reaction 4 → 5 may be achieved by any of a variety of well-known deblocking procedures such as hydrolysis or hydrogenolysis. A particularly convenient means for the deblocking 4 → 5, when R = acyl, is by alcoholysis procedure comprising treating 4 in a lower alkanol such as methanol or ethanol, in the presence of 0.1 to 1.4 equivalents of the corresponding alkali metal alkoxide such as sodium methoxide; typically the reaction is conducted at a temperature of from 0 to 25°C., for from 0.5 to 2 hours or is by an acid hydrolysis by treating 4 (R = triorganosilyl) with HCl in THF or DMF, at 25°C for 1 to 15 minutes. The ring closure reaction 5 → 7 proceeds *via* the oxo intermediate 6 and is achieved by treating 5 with an equivalent of an oxidizing system such as 1:1 mixture of dimethylsulfoxide (DMSO) and acetic anhydride ($Ac_2O$); other oxidizing systems include cyclohexylcarbodiimide in DMSO, $CrO_3$.2 (pyridine) in $CH_2Cl_2$, and pyridinium chlorochromate in $CH_2Cl_2$ for example. Typically, the closure step 5 → 7 is conducted at a temperature of from about 0 to 100°C for from 0.25 to 24 hours in the oxidative system (DMSO/$Ac_2O$) described above or by heating from 100—160°C (after isolation of the oxo compound 6) in a solvent such as benzene, toluene, dioxane, xylene, or DMF. The carboxyl deblocking step 7 → 8 may be achieved by a number of well-known procedures such as hydrolysis, hydrogenation, or photolysis of a suitable R′ group. Suitable hydrogenation catalysts for deblocking include the platinum metals and their oxides such as palladium on carbon; suitable solvents for the hydrogenation include methanol, dioxane/$H_2O$ or ethanol/$H_2O$ in the presence of hydrogen at a pressure of from 1 to 50 atmospheres; the hydrogenation is typically conducted for from 5 min. to 4 hours at a temperature of about 25°C in the optional presence of a mild base such as sodium bicarbonate. The carboxyl deblocking can also be achieved by photolysis of 7 in dioxane/$H_2O$, methanol/$H_2O$ or ethanol/$H_2O$ under u.v. radiation ($\lambda = 350$ nm) for 1 to 4 hr. at 25°C.

The glyoxylate esters 1a used to react with 1 can be prepared by oxidation of the corresponding tartaric acid diesters with oxidants such as periodic acid or lead tetracetate in a solvent such as THF, benzene, or methylene chloride at −20 to 25° for 1/2 to 4 hours. The tartarate esters are prepared from dilithio tartarate or disodio tartarate by reaction with R′X wherein X is chloro, bromo or iodo and R′ is as defined above in a solvent such as DMF or DMSO at 25° to 70°C. for from 4 to 48 hrs. As noted above, R′ may be a pharmaceutically acceptable ester moiety. Such pharmaceutically acceptable esters and amides, however, may also be prepared from the free acid of I.

The following diagram summarizes the synthesis of substituted azetidinone material 1.

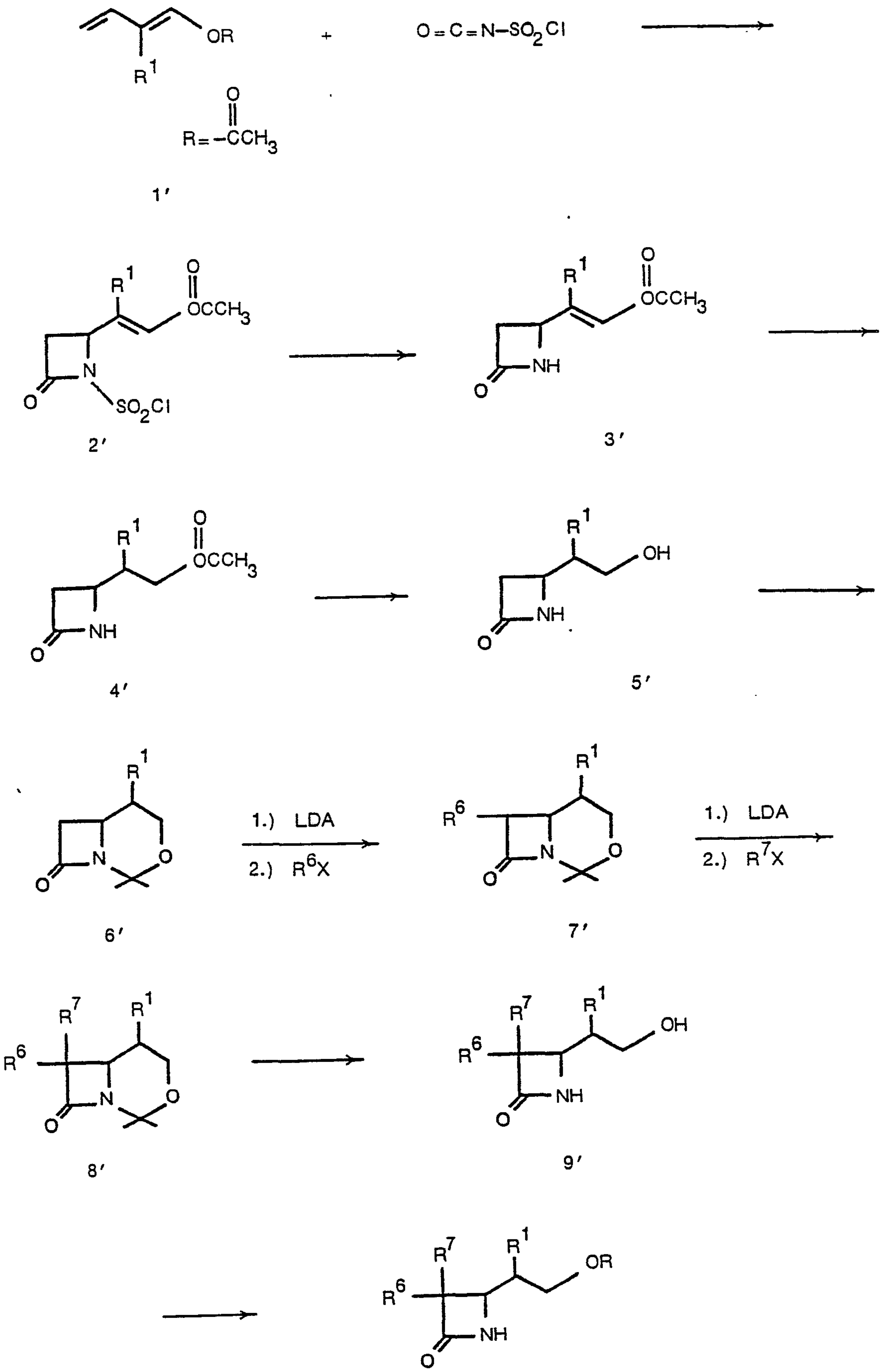


1′ 2′ 3′ 4′ 5′ 6′ 7′ 8′ 9′

In words relative to the above diagram for the preparation of 1, the 4-(1-methyl-2-acetoxyvinyl)-azetidinone-2-one (3′) is prepared by reacting chlorosulphonyl isocyanate and an acyloxybutadiene (1′) such as 2-acetoxy-2-methylbutadiene in a solvent such as anhydrous diethyl ether at a temperature of from about −30°C to 0°C under a nitrogen atmosphere. The reaction intermediate 2′ is converted to 3′ by hydrolysis. The reduction of 3′ to provide the 4-(1-methyl-2-acetoxyethyl)-2-azetidinone (4′) is conducted by any convenient means such as hydrogenation in the presence of a catalyst such as platinum, palladium or oxides thereof under a hydrogen pressure of from 1 to 20 atmospheres in a solvent such as ethanol or ethyl acetate, at a temperature of from 0° to 25°C. for from 5 minutes to 1 hour. The 4-(2-hydroxy-1-methyl-ethyl-2-azetidinone species 5′ is obtained from 4′ by hydrolysis. The 8-oxo-2,2-dimethyl-5-methyl-3-oxa-1-azabicyclo[4.2.0]octane species 6′ is obtained on treatment of 5′ with 2,2-dimethoxypropane in the presence of a catalyst such as boron trifluoride etherate in a solvent such as methylene chloride at a temperature of from 0° to 40°C. for from 1 to 40 minutes. Alternatively 5′ can be treated with boron trifluoride etherate and trimethylorthoformate to give 8-oxo-2-methoxy-5-methyl-3-oxa-1-azabicyclo[4.2.0]octane which can be mono- or dialkylated following the procedures for 6′ → 7′ or 8′. Alkylation of 6′ provides 7′. Typically, 6′ is treated with a strong base such as lithium diisopropylamide, sodium hydride, phenyl lithium or butyl lithium in a solvent such as tetrahydrofuran (THF), ether or dimethoxyethane at a temperature of from −80°C to 0°C., whereupon the alkylating agent of choice, $R^6X$ is added ($R^6$ is as described above and X is chloro or bromo; alternatively the alkylating agent may be $R^6$-tosylate $R^6$-mesylate or an aldehyde or ketone such as acetaldehyde) to provide mono- alkylated species 7′. When desired dialkylated species 8′ may be obtained from 7′ by repeating the alkylating procedure, 6′ → 7′. Species 9′ is obtained from 7′ or 8′ by acid hydrolysis.

The desired blocked-species 1 is obtained by treating 9 with an silylating agent such as t-butyldimethylchlorosilane or trimethylchlorosilane in a solvent such as DMF, $CH_2Cl_2$ or THF in the presence of a base such as imidazole at 0°C to 25°C for from 0.5 hr to 6 hr or with an acylating agent such as acetyl chloride, formic acetic anhydride or trifluoroacetic anhydride in a solvent such as $CH_2Cl_2$, $CHCl_3$ or THF at a temperature of from −20° to about 25°C. for from 0.5 to about 4 hours. The starting material 1 may be isolated for later reaction in accordance with the procedures of the present invention for the preparation of the compounds of the present invention.

It should be noted that in the establishment of R (9′ → 1), the ring nitrogen may be protected by an easily removable blocking group R″:

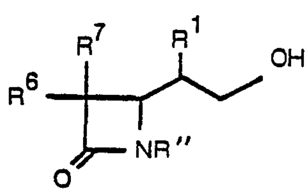

wherein R″ is acyl or triorganosilyl such as trimethylsilyl, t-butyldimethylsilyl, trifluoroacetyl or formyl. Removal of R″ is accomplished by hydrolysis to provide 1 according to well-known procedures.

Starting material 1 may alternatively be prepared by the following scheme:

5′

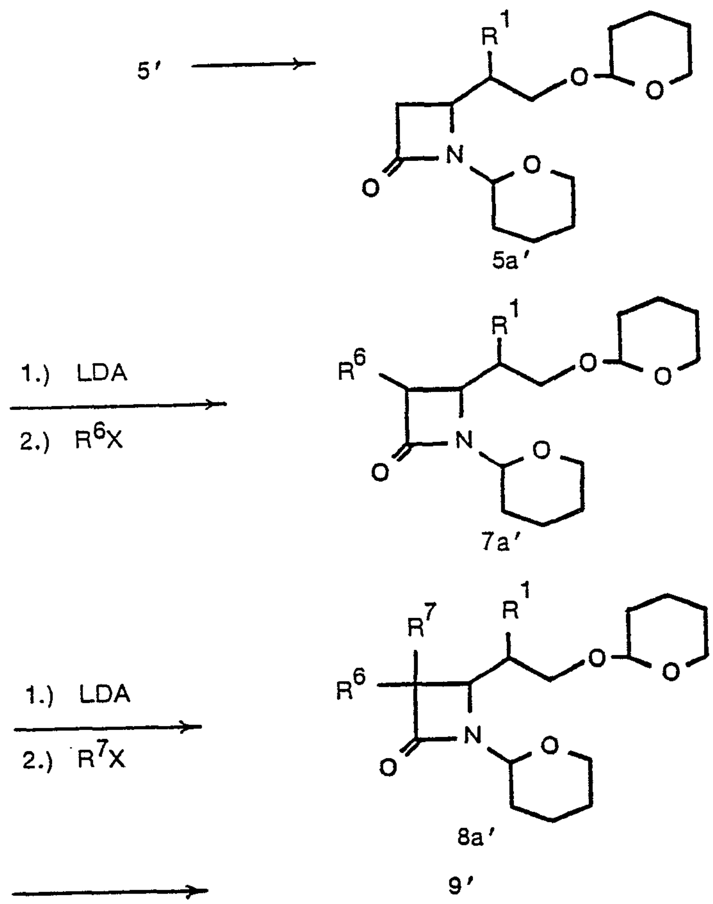


5a′

7a′

8a′

9′

wherein all symbolism is as previously defined.

Reaction 5 → 5a′ is accomplished by treating 5′ with 2,3-dihydropyran in a solvent such as p-dioxane or benzene, in the presence of for example p-toluene-sulfonic acid or perchloric acid, at a temperature of from 0° to about 30°C. The intermediate 5a′ may be isolated for later alkylation to obtain 7a′ and 8a′ by procedures analogous to previously described reactions 6′ → 7′ → 8′. Intermediate species 9′ is obtained from 7a′ or 8a′ by mild acid hydrolysis.

Finally, it should be noted that intermediate species 9a′ may conveniently be prepared for later reaction in the above scheme by internal acylation according to the following reaction:

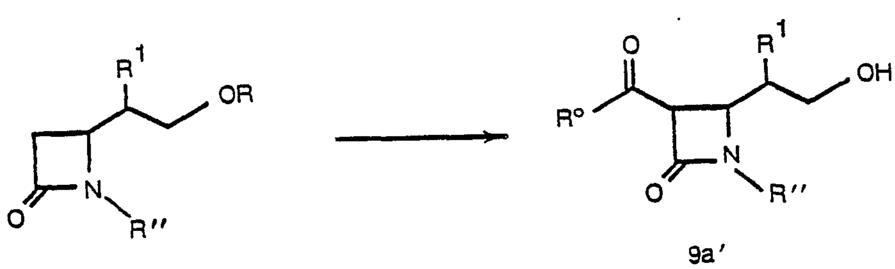

9a′

wherein R is acyl, 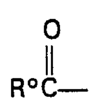

is $R^1$ and R° is for example, lower alkyl or acyl. Typically, the above reaction is conducted in a solvent such as tetrahydrofuran, ether or dimethoxyethane, in the presence of 1 to 2 equivalents of a strong base such as lithium diisopropylamide, sodium hydride or potassium hydride at a temperature of from −78 to 25°C., for from 0.5 to 24 hours.

It will be recognized that the compounds of the present invention, structure I, above, exhibit stereoisomerism. The nature of which depends upon the identity of ring substituents $R^6$, $R^7$ and $R^1$. The total synthesis of I is capable of stereo-selectivity. Preferably the paths leading the ultimate diastereomers of I diverge at intermediate level 6′, 7′ or 8′ (see above reaction diagram). Fractional

crystallization or practically any chromatographic technique is suitable for resolving the diastereoisomers at the preferred intermediate level. Completion of the synthesis after such resolution provides, if desired, the diastereoisomers of I in substantially pure form. A representative example of such resolution is presented below in the Examples section.

Preparation of the Substituted Acyloxy-butadiene, 1′

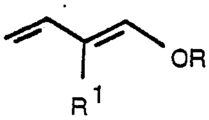

1′

The substituted 1,3-butadiene reagent 1′ is prepared according to known procedures, which may be summarized:

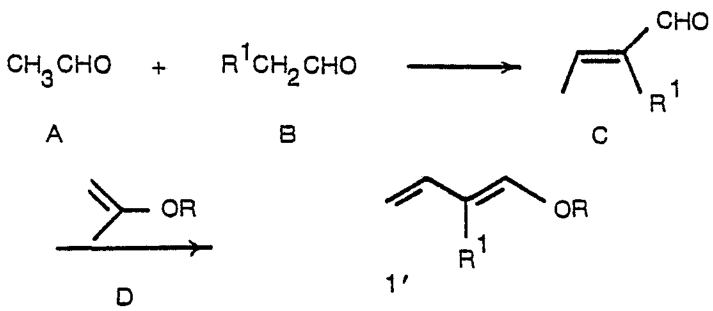

A B C

D 1′

wherein $R^1$ and R are as defined above.

The $\alpha,\beta$-unsaturated aldehyde intermediate C is prepared on condensation of A and B in the presence of a base such as NaOH, at a temperature of from 0 to 100°C for from 10 min. to 2 hours. Condensation of C with the isopropenyl ester D in the presence of cupric acetate (1 to 10 mole percent relative to C) and a strong acid such as p-toluene sulfonic acid, perchloric acid or sulfuric acid (1 to 10 mole percent relative to C) provides 1′. Typically the reaction is conducted at 90 to 120°C for from 1 to 8 hours. Representative examples illustrating this preparation of 1′ are given below.

In the generic description of the present invention (I, above; when $R^o$=H), the substituents $R^6$, $R^7$ and $R^1$ are preferably selected from hydrogen (R′ is not H); substituted and unsubstituted: straight and branched loweralkyl having from 1 to 10 carbon atoms; cycloalkyl having from 3 to 6 carbon atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; phenyl and naphthyl; phenylalkyl such as benzyl and phenethyl; heterocyclyl (saturated and unsaturated) comprising mono- and bicyclic structures having from 5 to 10 ring atoms wherein one or more of the hetero atoms is selected from oxygen, nitrogen or sulphur, such as thiophene, imidazolyl, tetrazolyl and furyl; heterocyclylalkyl which comprises the immediately preceding heterocyclyl moieties and the alkyl moiety comprises from 1 to 10 carbon atoms; the substituent (or substituents) relative to the above-named radicals is selected from amino, hydroxyl, cyano, carboxyl, nitro, chloro, bromo, fluoro, lower alkoxy having from 1 to 6 carbon atoms, mercapto, perhaloloweralkyl such as trifluoromethyl, loweralkylthio, guanidino, amidino, sulfamoyl, and N-substituted: sulfamoyl, amidino and guanidino wherein the N-substituent is loweralkyl having from 1 to 6 carbon atoms or aryl having 6—10 carbon atoms. In a preferred class of such compounds R′ is alkyl having from 1—6 carbon atoms, cyclopropyl, benzyl or phenyl; $R^6$ is hydrogen and $R^7$ is alkyl having from 1—6 carbon atoms or phenylalkyl wherein the alkyl moiety has 1—6 carbon atoms substituted by hydroxyl or amino. Preferably R′ is methyl, ethyl, isopropyl, t-butyl or phenyl and $R^7$ is 1-hydroxyethyl, methyl, or hydroxymethyl.

In a preferred compound of formula I $R^o$, $R^6$ and $R^7$ are hydrogen and $R^1$ is methyl, phenyl, ethyl, cyclopropyl, propyl and isopropyl.

A preferred compound of formula I is a compound having the formula:

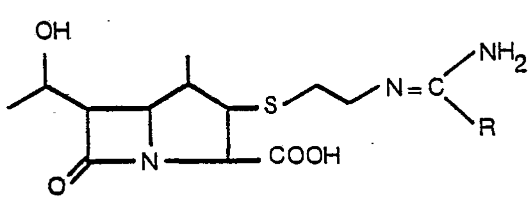

wherein R is H or $CH_3$.

A particularly preferred class of compounds (I, above) are those wherein $R^7$ is hydrogen, $R^1$ is selected from (I, above) substituted and unsubstituted: loweralkyl having from 1 to 6 carbon atoms, cyclopropyl, benzyl and phenyl; and $R^6$ is an $\alpha$-substituted alkyl wherein the $\alpha$-substituent is hydroxyl, amino or mercapto and wherein the alkyl moiety is straight or branched and comprises 1 to 6 carbon atoms; the substituents relative to the above-named preferred radicals $R^1$ are selected from hydroxyl, bromo, fluoro, chloro, amino, amidino, guanidino, phenyl, mercapto, carboxyl, trifluoromethyl, loweralkylthio and loweralkoxyl wherein the alkyl moiety of the loweralkylthio and loweralkoxyl comprises 1 to 6 carbon atoms.

The preferred esters used as protecting groups are those where R′ is benzyl, p-nitrobenzyl, o-nitrobenzyl, t-butyl, bromo-t-butyl, t-butyl-dimethylsilyl, trimethylsilyl, trichloroethyl; or R′ represents pharmaceutically acceptable ester moieties such as pivaloyloxymethyl, allyl, methallyl, (2-methylthio)-ethyl, 3-methyl-2-butenyl, p-t-butylbenzyl, 5-indanyl, 3-phthalidyl.

The compounds made available by the present invention are valuable antibiotics active against various grampositive and gram-negative bacteria and, accordingly, find utility in human and veterinary medicine. Such sensitive bacteria representatively include: *Staphylococcus aureus, Escherichia coli, Klebsiella pneumoniae, Serratia, Salmonella typhosa, Pseudomonas* and *Bacterium proteus.* The resulting compounds may further be utilized as additives to animal feed, for preserving foodstuffs, and as disinfectants. For example, they may be employed in aqueous compositions in concentrations ranging from 0.1 to 100 parts of antibiotic per million parts of solution in order to destroy and inhibit the growth of harmful bacteria on medical and dental equipment and as bactericides in industrial applications, for example, in waterbased paints and in the white water of paper mills to inhibit the growth of harmful bacteria.

These antibiotics may be used alone or in combination as an active ingredient in any one of a variety of pharmaceutical preparations. These antibiotics and their corresponding salts may be employed in capsule form or as tablets, powders or liquid solutions or as suspensions or elixirs or may be administered orally, intravenously or intramuscularly.

Tablets and capsules for oral administration may be in unit dose presentation form, and may contain conventional excipients such as binding agents, for example, syrup, acacia, gelatin, sorbitol, tragacanth, or polyvinylpyrrolidone; fillers for example, lactose, sugar, maize-starch, calcium phosphate, sorbitol or glycine; lubricants, for example, magnesium stearate, talc, polyethylene glycol, silica; disintegrants, for example, potato starch or acceptable wetting agents such as sodium lauryl sulphate. The tablets may be coated according to methods well known in the art. Oral liquid preparations may be in the form of aqueous or oily suspension, solution, emulsions, or syrups; or may be presented as a dry product, for reconstitution with water or other suitable vehicles before use. Such liquid preparations may contain conventional additives such as suspending agents, for example, sorbitol, syrup, methyl cellulose, glucose/sugar syrup, gelatin, hydroxyethylcellulose, carboxymethyl cellulose, aluminum stearate gel or hydrogenated edible oils, for example almond oil, fractonated coconut oil, oily esters, propylene glycol, or ethyl alcohol; preservatives, for example methyl or propyl *p*-hydroxybenzoates or sorbic acid.

Compositions for injection may be presented in unit dose form in ampules, or in multidose container. The compositions may take such forms as suspensions, sulutions, or emulsions in oily or aqueous vehicles, and may contain formulatory agents such as suspending, stabilizing and/or dispersing agents. Alternatively, the active ingredient may be in powder form for reconstitution with a suitable vehicle, e.g., sterile, pyrogen-free water, before use.

The compositions may also be prepared in suitable forms for absorption through the mucous membranes of the nose and throat or bronchial tissues and may conveniently take the form of powder or liquid sprays or inhalants, lozenges, throat paints. For medication of the eyes or ears, the preparations may be presented as individual capsules, in liquid or semi-solid form, or may be used as drops. Topical applications may be formulated in hydrophobic or hydrophilic bases as ointments, creams, or lotions.

Also, in addition to a carrier, the instant compositions may include other ingredients such as stabilizers, binders, antioxidants, preservatives, lubricators, suspending agents, viscosity agents or flavoring agents. In addition, there may also be included in the compositions other active ingredients to provide a broader spectrum of antibiotic activity.

For veterinary medicine the composition may, for example, be formulated as an intramammary preparation in either long or quick-release bases.

The dosage to be administered depends to a large extent upon the general health and weight of the subject being treated, and the route and frequency of administration — the parenteral route being preferred for generalized infections and the oral route for intestinal infections. In general, a daily oral dosage consists of from about 2 to about 600 mg. of active ingredient per kg. of body weight of the subject in one or more applications per day.

A preferred daily dosage for adult humans lies in the range of from about 15 to 150 mg. of active ingredient per kg. of body weight.

The instant compositions may be administered in several unit dosage forms as, for example, in solid or liquid orally ingestible dosage form. The compositions per unit dosage, whether liquid or solid

may contain from 0.1% to 99% of active material, the preferred range being from about 10—60%. The composition will generally contain from about 15 mg. to about 1500 mg. of the active ingredient; however, in general, it is preferably to employ a dosage amount in the range of from about 100 mg. to 1000 mg. In parenteral administration the unit dosage is usually the pure compound in a slightly acidified sterile water solution or in the form of a soluble powder intended for solution.

Especially preferred pharmaceutically acceptable salts and esters involving the carboxyl group of compounds of the present invention (I) include sodium potassium and ammonium salts and pivaloxymethyl, p-t-butylbenzyl, 5-indanyl, 3-phthalidyl and 3-methyl-2-butenyl esters. One should note that when, in the total synthesis outlined above, $R^2$ is a pharmaceutically acceptable ester moiety, there is no need for the final deblocking step if it is desired to have the final product I in the form of a pharmaceutically acceptable ester.

Especially preferred embodiments of the present invention are those, as defined above, except that any unsubstituted amino group borne on radical $R^8$ of Structure I is derivatized according to the teachings of Belgium Patent 848,545 (issued 5—20—77); the resulting amino group being represented thus (partial structure):

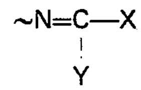

wherein X and Y are defined by the publication; species wherein X is H or lower alkyl and Y is $NH_2$ are especially preferred.

The following examples, illustrate the product, process, compositional or method of treatment aspects of the present invention. All reaction temperatures are in °C.

Example 1

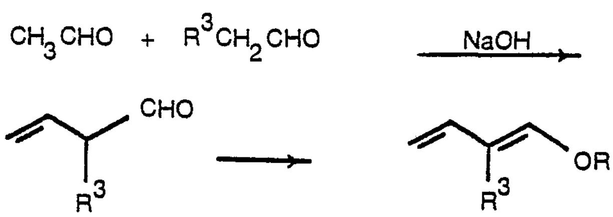


C

1′

The $\alpha,\beta$-unsaturated aldehydes (C) are prepared by modified procedures reported by M. B. Green and W. J. Hickinbottom in *J. Chem. Soc.* 3262 (1957); and W. J. Bailey and R. Barclay Jr., *J. Org. Chem., 21,* 328 (1956).

Acetaldehyde (1 eq.) and propionaldehyde ($R^3$=$CH_3$) (1 eq.) are placed in a three-necked round-bottom flask which is equipped with a mechanical stirrer, a dry-ice condenser, and a pressure equalized dropping-funnel. To the solution is added dropwise 1 eq. of 1*N* NaOH through the dropping funnel with constant stirring. After completion of the mixing, the mixture is stirred for 10 min, then poured into a beaker containing crushed ice. Extraction of the mixture with ether gives crude product. The desired product (C) is obtained by fractional distillation through a Widmer column.

Isopropenyl acetate (2 eq), cupric acetate (0.002 eq) p-toluenesulfonic acid (0.008 eq.) and the $\alpha,\beta$-unsaturated aldehyde C (1 eq.) are placed in a three-necked round-bottom flask equipped with a thermometer, a nitrogen inlet tube and a Widmer column which is attached with a distillation head. The mixture is heated at 93—110°C until quantitative acetone is collected. The mixture is then allowed to cool to r.t. and filtered from solids. The dark brown filtrate is mixed with triethanolamine in water at 0°C. The two layer mixture is distilled quickly under reduced pressure. The organic layer of the distillate is separated. The aqueous layer is extracted with 200 ml ether. The combined organic layer is washed with 10% $K_2CO_3$, dried over $Na_2SO_4$, and evaporated *in vacuo*. The residue so obtained is mixed with 2.0 g N-phenyl-$\beta$-naphthamine and distilled under reduced pressure to give the desired 2-substituted 1-acetoxy-1,3-butadiene (1′).

Following the procedure of Example 1, the following $R^3$ substituted species are obtained. (Table I).

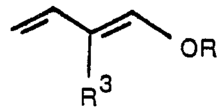

TABLE I

| $R^3$ | R |
|---|---|
| 1. $CH_3$ | 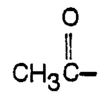 |
| 2. $CH_3CH_2$ | 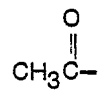 |
| 3. $CH_3CH_2CH_2$ | 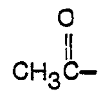 |
| 4. 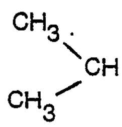 | 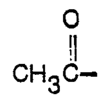 |
| 5. 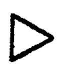 | 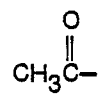 |
| 6. Ph (Ph = phenyl) | 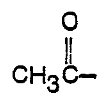 |
| 7. $PhCH_2$ | 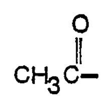 |

Example 2

Preparation of 6-(1-hydroxyethyl)-1-methyl-2-(2-amino-1-methylethylthio)carbapen-2-em-3-carboxylic acid

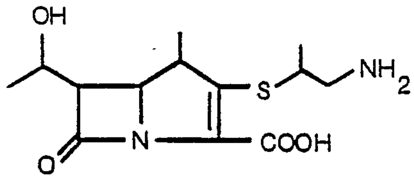

*Step A*

Preparation of 1′

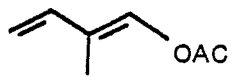

1′

Isopropenyl acetate (182 g), cupric acetate (0.40 g), 2-methyl-2-butenal (84 g) and p-toluenesulfonic acid (1.52 g) are placed in a 1.0—1, three-necked flask equipped with a thermometer, a nitrogen inlet tube and a 10-in. Widmer column which is attached with a distillation head. The mixture is heated at 93—110°C until 73 ml of acetone is collected. After cooling to r.t. (25°C) the mixture is filtered from solids. The dark brown filtrate is cooled in an ice-bath and mixed with 3.4 g triethanolamine in 200 ml water. The two layer mixture is distilled quickly at 53 mm (b.p. 54°C). The organic layer of the distillate is separated. The aqueous layer is extracted with 200 ml ether. The organic layers are combined and washed with 10% $K_2CO_3$, dried over $Na_2SO_4$, and evaporated *in vacuo*. The residue so obtained is mixed with 2.0 g N-phenyl-β-naphthamine and distilled under reduced pressure to give 1′ (97 g), b.p. 81—91° (66 mm).

*Step B*

Preparation of 2′ and 3′

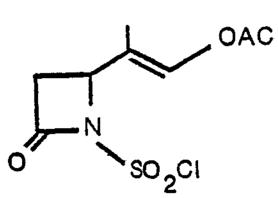

2′

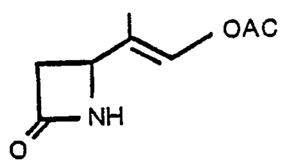

3′

Chlorosulfonylisocyanate (CSI) (6.5 ml) is placed in a three-necked, 100-ml flask equipped with a thermometer, a magnetic stirring bar, a nitrogen inlet tube and a 25-ml pressure-equalized dropping funnel. The CSI is chilled to −50°C and mixed with 12.5 ml ether through the dropping funnel. The etheral solution of CSI is allowed to warm up to −25°C; to the solution is added dropwise 1-acetoxyl-2-methyl-1,3-butadiene (1′) (5.9 ml in 12.5 ml ether) in 30 min. The mixture is then stirred for 20 min at −20 ± 3°C. The white precipitate formed initially is redissolved at the end of the reaction.

In a 500-ml round bottom flask, a solution of 10 g sodium sulfite and 25 g potassium hydrogen phosphate in 100 ml water is prepared and is cooled in an ice bath. Ether (100 ml) and crushed ice (100 g) are added and the mixture is vigorously stirred in an ice bath. At the end of 20 minutes reaction time, the reaction mixture which contains 2′ is transferred into the dropping funnel and added dropwise to the hydrolysis mixture in 5 minutes. The hydrolysis is allowed to continue for an additional 30 minutes at 3°C. The organic layer is separated and the aqueous is extracted with 50 ml ether. The organic layers are combined, dried over $Na_2SO_4$ and evaporated to give crystalline product 3′ (2.3 g), m.p. 77—78,5°; m.s. 169($M^+$); IR 1760 $cm^{-1}$ (β-lactam);

NMR (300 MHz, $CDCl_3$): 1.70 (d), 2.16 (s), 2.84 (qq), 3.18 (qq), 4.20 (m), 5.82 (broad), and 6.26 (s) ppm.

*Step C*

Preparation of 4′:

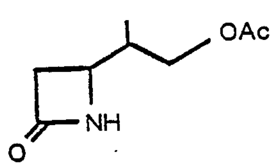

4-(1-methyl-2-acetoxyvinyl)azetidine-2-one (3′) (6.5 g) is hydrogenated on a Parr shaker at r.t.

under 40 psi hydrogen in the presence of 10% Pd/C (0.6 g) in 200 ml ethylacetate for 2 hr. The mixture is filtered from the catalyst and the filtrate is evaporated *in vacuo* to give the crude product. Purification of the crude product by high pressure liquid chromatography (HPLC) (silica gel column, 30% ethylacetate/$CH_2Cl_2$ solvent system) affords a white crystalline product 4′ (6.04 g) after evaporation of solvent. The product shows following physical characteristics: ms 171 ($M^+$); IR(Neat) 1754 $cm^{-1}$; NMR (60 MHz, $CDCl_3$): 0.96 (d), 1.01 (d), 2.06 (d, OAc), 2.75—3.80 (m), 3.99 (d) and 6.80 (broad) ppm.

*Step D*
Preparation of 5′

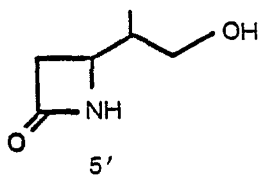

5′

Under $N_2$ at 0°, a solution of 4-(1-methyl-2-acetoxyethyl)-2-azetidinone 4′ (1.2 g) in 10 ml methanol is treated with sodium methoxide (57 mg). After stirring for 1 hr, the solution is neutralized with glacial acetic acid (65 mg). Removal of methanol *in vacuo* gives crude 4-(1-methyl-2-hydroxyethyl)-2-azetidinone (5′) as an oil. The product is purified chromatography on silica gel eluting with ethyl acetate and to give 0.78 g of 5′:

IR (neat): 1740 $cm^{-1}$;

NMR ($CDCl_3$): 0.77 (d), 0.96 (d), 1.90 (m), 2.60—3.30 (m), 3.60 (m), 4.19 (s), and 7.23 (s). The product crystallizes as colorless solids in the refrigerator.

*Step E*
Preparation of 6′

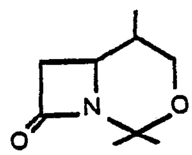

6′

A solution of 4-(1-methyl-2-hydroxyethyl)-2-azetidinone (0.5 g) and 2,2-dimethoxypropane (0.48 g) in 10 ml anhydrous methylene chloride is treated with boron trifluoride (55 mg) at room temperature for 90 min. The mixture is washed with 5 ml saturated $NaHCO_3$. The organic layer is separated, dried over $Na_2SO_4$ and allowed to evaporate *in vacuo* to give crude isomeric mixture of 6′ (0.48 g) as an oil.

Separation of isomers 6′$\alpha$ and 6′$\beta$ is accomplished by high pressure liquid chromatograph (HPLC) (silica gel) eluting with 40% ethylacetate/hexanes. After evaporation of the solvents affords 150 mg of 6′$\beta$ as an oil and 200 mg of 6′$\alpha$ as a white solid.

NMR (300 MHz, $CDCl_3$) of 6′$\alpha$: 0.81 (d), 1.31 (s), 1.68 (s), 1.62 (m), 2.52 (q), 3.05 (m), 3.42 (t), and 3.66 ppm (q),

NMT (300 MHz, $CDCl_3$) of 6′$\beta$: 1.10 (d), 1.38 (s), 1.67 (s), 1.90 (m), 2.80 (q), 2.86 (q), 3.62 (q), 3.78 (m) and 3.98 (q) ppm.

*Step Fa*
Preparation of 7′$\alpha$

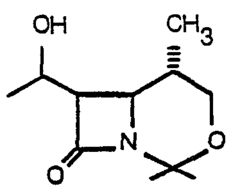

7′a

At −78°C, diisopropylamine (2.2 g) in 20 ml of anhydrous tetrahydrofuran is treated with n-butyllithium (1.6M in n-hexane, 14 ml) for 5 min. To the solution is added 8-oxo-5$\alpha$,2,2-trimethyl-1-3-oxa azabicyclo[4.2.0]octane (6′$\alpha$) (3.4 g) and the mixture is stirred for 10 min. The resulting lithium enolate is treated with acetaldehyde (1.68 ml). The mixture is stirred for 1 min. then is quenched with 24 ml saturated ammonium chloride at −78°C, then allowed to warm to room temperature (25°C). The

mixture is extracted with ethylacetate (2 x 100 ml). The organic layer is separated, dried over $Na_2SO_4$ and allowed to evaporate *in vacuo* to give 4.5 g of the crude product 7'α.

The crude isomeric mixture of 7'α is purified and separated by HPLC (silica gel) eluting with 50% ethylacetate/methylene chloride to give 3.5 g of *trans*-7'α and 0.5 g of *cis*-7'α. Both isomers are crystalline solids.

*Step Fb*
Preparation of 7'β

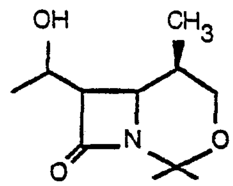

7'β

Following the procedure of Step Fa, except replacing the starting material 6'α with 6'β isomer, the products, trans-7'β (4.0 g) and *cis*-7'β (0.1 g), are obtained.

*Step Fc*
Preparation of 7''β

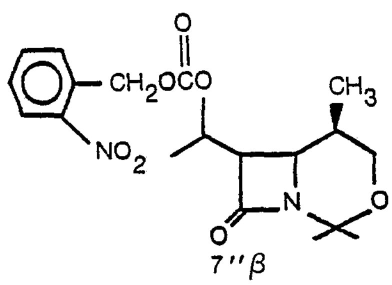

7''β

Under anhydrous conditions at 0°C a solution of R enriched trans-7'β (2.90 g) in 60 ml methylene chloride is treated 4-dimethylaminopyridine (3.32 g) and p-nitrobenzylchloroformate (5.88 g). The mixture is allowed to warm to room temperature and stirred for 1 hr. The resulting mixture is washed with 0.1*N* Hcl, water, brine and water. The organic layer is separated, dried over $Na_2SO_4$ and allowed to evaporate *in vacuo* to give crude products. The crude products dissolved in 20 ml ether and chilled at −5°C give the o-nitrobenzyl alcohol (0.5 g) which is separated by filtration. The isomeric mixture trans-7''β is purified and separated by HPLC (silica gel) eluting with 40% ethylacetate/cyclohexane to give 1.2 g of S-trans-7''β and 1.0 g of R-trans-7''β.

The spectra data of R-trans-7''β:

NMR (300 MHz, $CDCl_3$): 1.12 (d), 1.40 (s), 1.46 (d), 1.73 (s), 1.95 (m), 3.20 (q), 3.60 (q), 3.74 (q), 3.95 (q), 5.07 (m), 5.58 (q), 7.56 (t), 7.70 (m) and 8.19 (d) ppm.

The spectra data of S-trans-7''β:

NMR (300 MHZ, $CDCl_3$): 1.10 (d), 1.40 (s), 1.43 (d), 1.72 (s), 1.94 (m), 3.34 (q), 3.61 (q), 3.67 (q), 3.96 (q), 5.13 (m), 5.64 (d), 7.53 (m), 7.68 (m), and 8.17 (d) ppm.

*Step Fd*
Preparation of 7''α

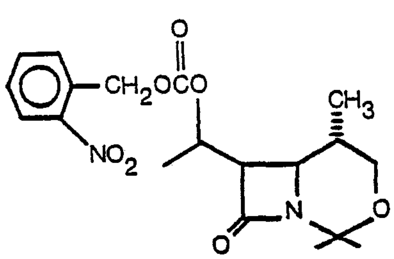

7''α

Following the procedure of Step Fc; except replacing the starting material trans-7'β with trans-7'α isomer, the products R-trans-7''α and S-trans-7''α are obtained.

*Step Ga*

Preparation of R-trans-9'β:

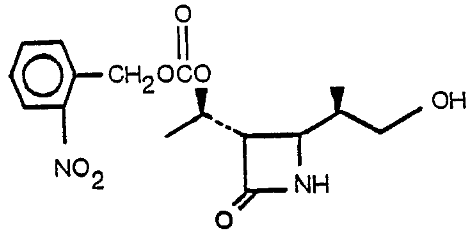

R-trans-9'β

8-Oxo-3-oxa-5β-2,2-trimethyl-7α-(1R - *o* - nitrobenzylcarbonyldioxyethyl) - 1 - azabicyclo[4.2.0]-octane (R-trans-7''β) (2.1 g) is dissolved in 4 ml trifluoroacetic acid and 4 ml water at room temperature and the mixture is stirred for 10 minutes. The resulting homogeneous solution is slowly poured into a vigorously stirred saturated solution of potassium bicarbonate (30 ml) in a 200-ml beaker. The mixture is extracted with methylene chloride (200 ml). The organic layer is separated, dried over $Na_2SO_4$ and allowed to evaporate *in vacuo* to give crude product 9' which is purified by a silica gel column eluting with 40% ethylacetate/cyclohexane to afford product R-trans-9'β as an oil.

NMR (300 MHz, $CDCl_3$): 0.98 (d), 1.28 (d), 2.85 (m), 3.20 (q), 3.62 (m), 5.12 (m), 5.57 (q), 6.40 (s), (7.53) (t), 7.66 (m) and 8.14 (d).

*Steps H—K*

Steps H, I, J and K complement Steps E, Fa-d, and G for the preparation of 3-(1-*p*(and *o*)-nitrobenzylcarbonyldioxyethyl)-4-(1-methyl-2-hydroxyethyl)azetidinone

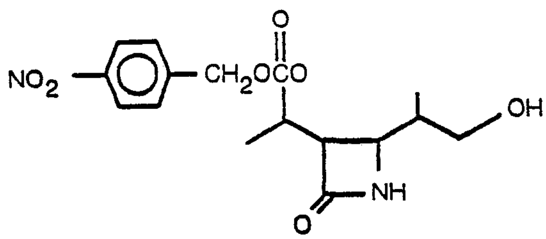

*Step H*

Preparation of 1-(2-Tetrahydropyranyl)-4-[1-methyl-2-(2-tetrahydropyranyl)oxyethyl]-2-azetidinone

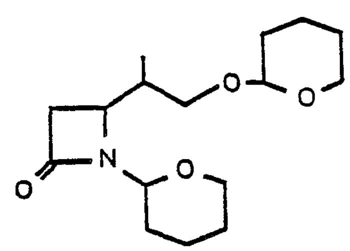

Under nitrogen and at 25°C, a solution of 4-(1-methyl-2-hydroxyethyl)-2-azetidinone (62 mg), in 0.5 ml of anhydrous *p*-dioxane is treated with 2,3-dihydropyran (.98 ml) and *p*-toluenesulfonic acid monohydrate (19 mg). The resulting solution is stirred for a period of 60 minutes and then partitioned between 10 ml of .5M pH 7 phosphate buffer and 10 ml of ethyl acetate. The aqueous phase is extracted a second time with ethyl acetate. The combined ethyl acetate solutions are washed with brine, dried over magnesium sulfate and filtered. The filtrate is evaporated under reduced pressure to give 216 mg of crude product. Purification by preparative thick-layer chromatography developing with ethyl acetate, gives 80 mg of 1 - (2 - tetrahydropyranyl) - 4 - [1 - methyl - 2 - (2 - tetrahydropyranyl) - oxyethyl] - 2 - azetidinone as an oil.

*Step I*
Preparation of *Cis* and *Trans*-1-(2-tetrahydropyranyl)-3-(1-hydroxyethyl)-4-[1-methyl-2-(2-tetrahydropyranyl)-oxyethyl]-2-azetidinone

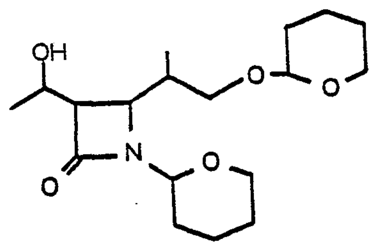

Following the procedure described for the preparation of 8-oxo-2,2,5-trimethyl-7$\alpha$ and $\beta$-(1-hydroxyethyl)-3-oxa-1-azabicyclo[4.2.0]octane from 8-oxo-2,2,5-trimethyl-3-oxa-1-azabicyclo[4.2.0]-octane and using 1 - (2 - tetrahydropyranyl) - 4 - [1 - methyl - 2 - (2 - tetrahydropyranyl)-oxyethyl] - 2 - azetidinone one obtains a diastereomeric mixture of both *cis* and *trans* - 1 - (2 - tetrahydropyranyl) - 3 - (1 - hydroxyethyl) - 4 - [1 - methyl - 2 - (2 - tetrahydropyranyl)oxyethyl] - 2 - azetidinone.

*Step J*
Preparation of *Cis* and *Trans*-1-(2-tetrahydropyranyl)-3-(1-p-nitrobenzylcarbonyldioxyethyl)-4-[1-methyl-2-(2-tetrahydropyranyl)oxyethyl]-2-azetidinone

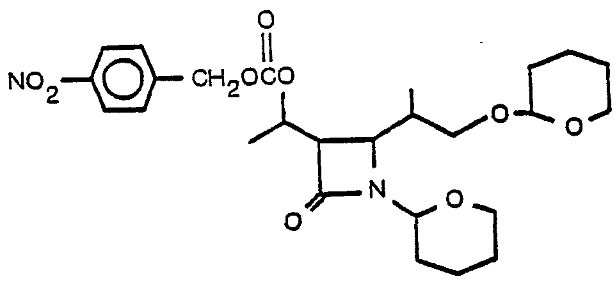

Following the procedure described for the preparation of 8 - oxo - 2,2,5 - trimethyl - 7$\alpha$ - (1 - p - nitrobenzylcarbonyldioxyethyl) - 3 - oxa - 1 - azabicyclo[4.2.0]octane from 8 - oxo - 2,2,5 - trimethyl - 7$\alpha$ - (1 - hydroxyethyl) - 3 - oxa - 1 - azabicyclo[4.2.0]octane and using *trans* - 1 - (2 - tetrahydropyranyl) - 3 - (1 - hydroxyethyl) - 4 - [1 - methyl - 2 - (2 - tetrahydropyranyl)-oxyethyl] - 2 - azetidinone there is obtained *trans* - 1 - (2 - tetrahydropyranyl) - 3 - (1 - p - nitrobenzylcarbonyldioxyethyl) - 4 - [1 - methyl - 2 - (2 - tetrahydropyranyl)oxyethyl] - 2 - azetidinone. The *cis* diastereoisomers are obtained in an analogous manner.

*Step K*
Preparation of *Cis* and *Trans*-3-(1-p-nitrobenzylcarbonyldioxyethyl)-4-(1-methyl-2-hydroxyethyl)-2-azetidinone

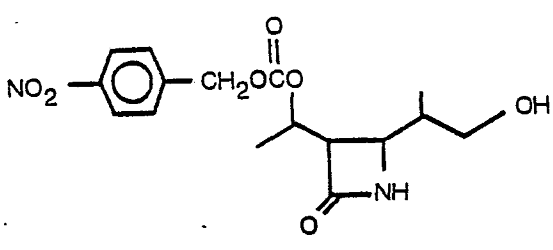

A solution of *trans* - 1 - (2 - tetrahydropyranyl) - 3 - (1 - p - nitrobenzylcarbonyldioxyethyl) - 4 - [1 - methyl - 2 - (2 - tetrahydropyranyl)oxyethyl] - 2 - azetidinone in methanol at 25°C is treated with .1 molar equivalent of *p*-toluenesulfonic acid monohydrate. The solution is stirred for a period of 2 hours and then neutralized with 1M pH 7 phosphate buffer. The product is extracted into ethyl acetate. The ethyl acetate solution is washed with brine, dried over magnesium sulfate and filtered. The filtrate is evaporated under reduced pressure to give *trans*-3-(1-p-nitrobenzylcarbonyldioxyethyl) - 4 - (1 - methyl - 2 - hydroxyethyl) - 2 - azetidinone. The cis diastereoisomers are obtained in an analogous manner.

*Step L*
Preparation of 12

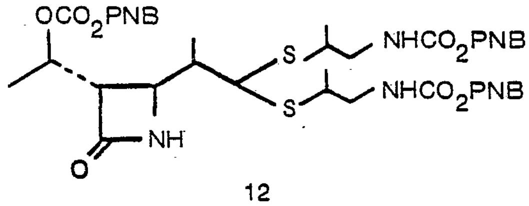

12

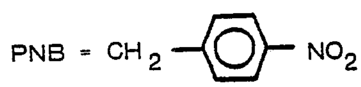

To 6.75 ml anhydrous pyridine (83.9 mmole) in 350 ml anhydrous acetonitrile is added 4.05 g anhydrous powdered chromium trioxide (mw = 100; 40.5 mmole). After stirring at room temperature (25°C) for 30 minutes, 9.6 g dried Supercell (Trade Mark) is added and stirring is continued for 5 additional minutes. A solution of 3.21 g *trans* - 3 - (1 - p - nitrobenzylcarbonyldioxyethyl) - 4 - (1 - methyl - 2 - hydroxyethyl) - 2 - azetidinone in 30 ml anhydrous acetonitrile is added all at once. The reaction mixture is stirred under anhydrous conditions at room temperature (25°C) for one hour. Addition of 9.6 g $NaHSO_3$ is followed by 5 minutes of stirring after which the reaction mixture is filtered through a mixed packed bed of 40 g silica gel and 40 g anhydrous magnesium sulfate. The bed is washed repeatedly with acetonitrile (total volume of filtrate 600 ml). The filtrate is concentrated under a $N_2$ stream to 130 ml volume. To this solution containing crude aldehyde at 0°C under $N_2$ is added 9.46 g 1-(*p*-nitrobenzyloxycarbonylamino)-2-propanethiol as prepared below (Step M). To the stirred reaction mixture is added 8.0 ml boron trifluoride etherate (63.4 mmole). After 1.5 hours at 0°C the reaction mixture is poured into a stirred ice-cold mixture of 69 g $K_2HPO_4$ — 500 ml $H_2O$ and 700 ml ethyl acetate (EA). The layers are separated, and the aqueous one is saturated with NaCl and re-extracted with additional EA. The combined organic layers are washed twice with brine, dried over anhydrous $MgSO_4$ and filtered. The filtrate is concentrated under a $N_2$ stream and then pumped on high vacuum to give crude 12.

The material is chromatographed on 450 g silica gel (column height = 48 cm; diameter = 5.5 cm) packed and applied in $CHCl_3$ and eluted with increasing percentages of MeOH in $CHCl_3$ (0—4% MeOH/$CHCl_3$). Those fractions containing the desired product are combined, concentrated under a $N_2$ stream; and pumped on high vacuum to give 12.

*Step M*
Preparation of 1-(p-Nitrobenzyloxycarbonylamino)-2-propanethiol

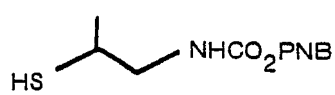

To 600 ml diethyl ether ($Et_2O$) — 75 ml $H_2O$ in an ice bath with stirring is added 3.2 g 1-amino-2-propanethiol hydrochloride (28.1 mmole). A solution of 7.14 g $NaHCO_3$ (85 mmole) in 75 ml $H_2O$ is added. The ice bath is removed, and at room temperature a solution of 6.75 g p-nitrobenzylchloroformate (31.3 mmole) in 270 ml $Et_2O$ is added dropwise over a period of one hour. After 10 additional minutes, the layers are separated. The ether layer is extracted with 150 ml 0.25N HCl, and then with 200 ml brine. Each aqueous layer is then backwashed successively with 100 ml $Et_2O$. The combined $Et_2O$ layers are dried over anhydrous $MgSO_4$, filtered, and concentrated under a $N_2$ stream. The crystalline residue is slurried in a small amount of ether, filtered, and the pale yellow crystals are dried under high vacuum to give 4.7 g product.

*Step N*
Preparation of 14

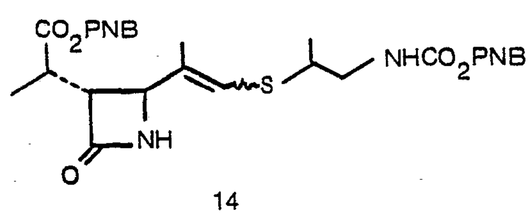

14

To 14.2 ml pentane (dried over 4H Linde molecular sieves) is added 0.5 ml $Br_2$. To 5 g of 12 in 58 ml tetrahydrofuran (THF) (freshly distilled from lithium aluminum hydride (LAH) and 65 ml $Et_2O$ (dried over 3A 1/16″ Linde molecular sieves) at 0°C under $N_2$ with stirring is added dropwise 10 ml of the

above 0.66M $Br_2$ solution. After 10 minutes at 0°C, 0.67 ml cyclohexene is added. After 5 minutes at 0°C., 1.7 ml triethylamine is added immediately followed by 40 ml ice-cold dimethylformamide (DMF) (distilled from anhydrous $CaSO_4$ at 40 mm and stored over 4A Linde molecular sieves). The ice bath is removed, and stirring is continued for 2 1/4 hours at room temperature. The reaction mixture is poured into a stirred ice-cold mixture of 12.6 ml 1M$KH_2PO_4$ 160 ml $H_2O$—500 ml (EA). After separation of the layers, the aqueous one is saturated with sodium chloride and re-extracted with EA. The combined organic layers are extracted once with brine, dried over anhydrous $MgSO_4$, filtered and concentrated under a $N_2$ stream followed by pumping under high vacuum to provide crude 14.

The material is chromatographed on 250 g silica gel (height = 45 cm; diameter = 4.5 cm) packed and applied in $CHCl_3$ and eluted with increasing percentages of MeOH in $CHCl_3$ (0—3% MeOH/$CHCl_3$). Those fractions containing clean product are combined, concentrated under a $N_2$ stream, and pumped on high vacuum to give 14.

*Step O*

Preparation of 15

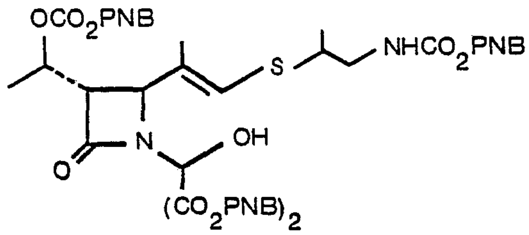

To a stirred solution of 2.48 g di(p-nitrobenzyl)ketomalonate (Step P) in 400 ml hot anhydrous toluene is added a solution of 2.52 g of 14 in 20 ml THF (distilled from LAH) and 40 ml anhydrous toluene. After some of the solvent is boiled off, additional anhydrous toluene is added, and the azeotropic drying process is repeated three times. The solution is then refluxed under $N_2$ for 30 minutes. Additional toluene is then allowed to boil off yet the volume is not allowed to diminish so much that precipitation occurs. Total heating time is approximately 2 1/2 hours. The clear yellow reaction mixture is removed from the oil bath and placed under a stream of $N_2$. After concentration to an oil, the residue is dissolved in $CH_2Cl_2$, dried over anhydrous $MgSO_4$, filtered, and concentrated under a $N_2$ stream to give crude 15.

The material is chromatographed on 250 g silica gel packed and applied in $CHCl_3$ (height = 43 cm; diameter = 4.5 cm). Elution with 500 ml 0.5% MeOH/$CHCl_3$ is followed by continued elution with 1% MeOH/$CHCl_3$ for the remainder of the chromatography. After the emergence of excess reagent, those fractions containing pure 15 are combined, concentrated under a $N_2$ stream and then on high vacuum to give 15.

*Step P*

Preparation of di-p-Nitrobenzyl Ketomalonate

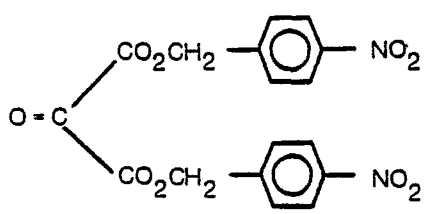

A mixture of 100 g *p*-nitrobenzyl bromide (0.46 mole), 28.6 g malonic acid (0.275 mole) and 750 ml ethanol (EtOH) is stirred and warmed on the steam bath until solution is achieved. A solution of 33 g KOH in 200 ml of water is added carefully with swirling. An additional 200 ml of water is added, and the two-phase system is refluxed for 1.8 hours. The lighter color homogeneous solution is cooled in ice for 1 hour and the crude product isolated by filtration, washed twice with a minimum of cold EtOH, and dried by pulling dry $N_2$ through the cake; 33.7 g of solid is obtained. If, during the refluxing stage the reaction mixture is slowly concentrated to ca. half volume by allowing refluxing solvent to distill off, the crude product yield rises to 77 g. The material is recrystallized from methanol to give pure di-p-nitrobenzyl malonate.

A mixture of 23.4 of di-p-nitrobenzyl malonate 10 g $SeO_2$, and 30—40 ml of xylene is stirred in a flask immersed in an oil bath. The bath temperature is raised over 1 hour to 130—135°. A gradual darkening of the reaction mixture is noted, and after a total of 4 hours at 130—135°, most of the insoluble residue is black Se°. The mixture is cooled, $MgSO_4$ is added to remove the water, and Celite (Trade Mark) is added to aid in filtration. The mixture is filtered through Celite and the cake washed with

xylene and a small portion of EtOAc. Final volume: 60 ml. A 100 g column of Baker silica gel is prepared in benzene and 10 ml of filtrate applied, then eluted with increasing amount of EtOAc in benzene, 500 ml fractions being collected. After one 2% ethyl acetate (EtOAc)/ØH, and two 10% EtOAc/ØH fractions, the third 10% and first 20% EtOAc/ØH provide the bulk of the product (1.6 g from 10 ml filtrate) and judged by tlc (20% EtOAc/$CHCl_3$; silica gel GF). Recrystallization from benzene, (1 g in ca. 50 ml concentrated to 1/3 volume and "spiked" with 1 ml of $H_2O$ saturated benzene): provides di-p-nitrobenzyl ketomalonate.

*Step Q*

Preparation of 17:

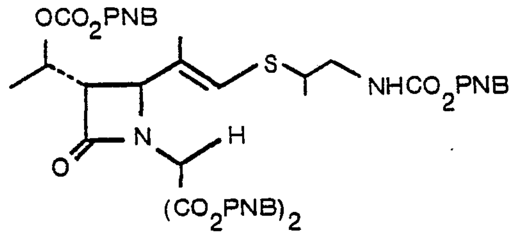

17

A solution of 1.468 g of 15 in $CH_2Cl_2$ is dried over anhydrous $MgSO_4$, filtered, and concentrated under a $N_2$ stream, and dried further under high vacuum just prior to the following reaction. To a solution of 15 in 24 ml THF (freshly distilled from LAH) at −20°C is added 0.206 ml anhydrous pyridine. With stirring under $N_2$, 294 mg of freshly distilled thionyl chloride in 5 ml THF is added dropwise. The reaction mixture is stirred for 10 minutes at −20°C., then 1/2 hour at 0°C and finally 1 hour at 25°C. The pyridine hydrochloride is filtered under $N_2$ and washed with 20 ml THF. The filtrate is concentrated under $N_2$ stream followed by pumping on high vacuum.

To this freshly prepared chloro compound is added with stirring a freshly shaken suspension of 678 mg tributylphosphine in 36.5 ml 9:1 DMF-$H_2O$ followed by 294 mg $K_2HPO_4$. The reaction mixture is stirred at 25°C., for 35 minutes. After dilution with 120 ml EA and 60 ml brine, the layers are separated, and the aqueous one is extracted two times with EA. The combined organic layers are washed one time with brine, dried over anhydrous $MgSO_4$, filtered and concentrated under a $N_2$ stream followed by pumping on high vacuum to give crude 17.

The material is chromatographed on 100 g silica gel (height = 28.5 cm; d = 4 cm) packed and applied in $CHCl_3$ and eluted with 0.5% MeOH in $CHCl_3$. Those fractions containing clean product are combined, concentrated under a $N_2$ stream and then on high vacuum to give 17. (EA = ethyl acetate.)

*Step R*

Preparation of 19:

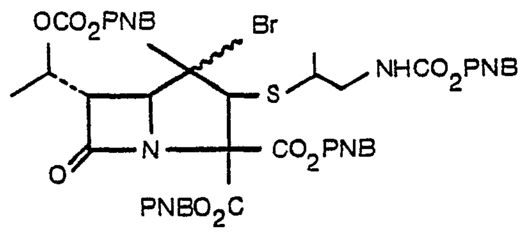

19

To 8.5 ml pentane (dried over 4A Linde molecular sieves) is added 0.2 ml $Br_2$. To 0.706 g of 17 in 18 ml THF (freshly distilled from LAH) and 5.7 ml $Et_2O$ (dried over 3A 1/16" Linde molecular sieves) at 0°C under $N_2$ with stirring is added dropwise 1.8 ml of the above 0.45M $Br_2$ solution (0.81 mmole) to give dibromide 18. After 15 minutes at 0°C., 0.42 ml triethylamine is added immediately followed by 10.5 ml ice-cold DMF (distilled from $CaSO_4$ at 40 mm and stored over 4A Linde molecular sieves). The ice-bath is removed, and stirring at room temperature is continued for 2 hours. The reaction mixture is poured into a stirred, ice-cold mixture of 2.1 ml 1M $KH_2PO_4$-70 ml $H_2O$-100 ml EA. The layers are separated, and the aqueous one is saturated with NaCl and re-extracted with EA. The combined organic layers are washed once with brine, dried over anhydrous $MgSO_4$, and filtered. The filtrate is concentrated under a $N_2$ stream and then pumped on high vacuum to give crude 19.

The material is chromatographed on 60 g silica gel (diameter = 2.8 cm) packed and applied in $CHCl_3$ and is eluted with 100 ml-2% EA/$CHCl_3$; 100 ml-4% EA/$CHCl_3$ and then 5% EA/$CHCl_3$ for the remainder of the chromatography. The fractions containing pure 19 are combined, concentrated under a $N_2$ stream, and pumped on high vacuum to give 19.

*Step S*

Preparation of 20:

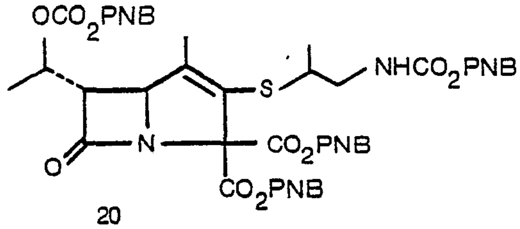

20

To 29 mg anhydrous silver fluoride is added a solution of 146 mg of 19 in 3.5 ml anhydrous pyridine. The stoppered reaction mixture is stirred at room temperature in the dark for one hour and then poured into 20 ml cold water — 30 ml EA. After separation of the layers, the aqueous one is extracted two times with EA and one time with $CHCl_3$. Each organic layer is extracted one time with $H_2O$ and one time with brine. The combined organic layers are dried over anhydrous $MgSO_4$, filtered, and concentrated under a $N_2$ stream followed by pumping on high vacuum to give crude 20.

Preparative thin layer chromatography (eluant = 40% acetone/hexane; repeated extraction of desired u.v. band with a large volume of $CHCl_3$) yields slightly contaminated 20. Re-chromatographing on silica using EA in $CHCl_3$ as an eluting system gives pure 20.

*Step T*

Preparation of 21:

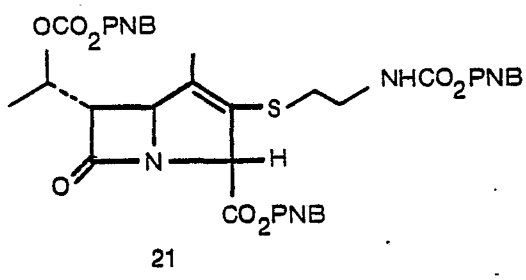

21

A solution of 77 mg of 20 in 0.9 ml S-collidine (distilled from powdered KOH) is added to 13.4 mg anhydrous LiI (dried for few hours at 100°C over $P_2O_5$ under vacuum). With stirring under $N_2$, the reaction mixture is heated in an oil bath at 120°C. After a total of 30 minutes, the reaction mixture is cooled to 25°C., diluted with $CH_2Cl_2$, and transferred to a round bottom flask for concentration under a $N_2$ stream and then on high vacuum. Partitioning the residue between EA-$H_2O$ and 1 ml 1M $KH_2PO_4$ is followed by extraction of the aqueous layer two additional times with EA and one time with $CHCl_3$. Each organic layer is then backwashed with brine. The combined organic layers are dried over anhydrous $MgSO_4$ filtered, concentrated under a $N_2$ stream and then on high vacuum to give crude 21.

Preparative thin layer chromatography on silica gel yields 21.

*Step U*

Preparation of 22:

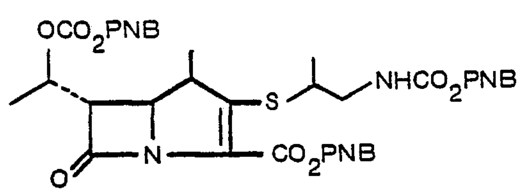

22

To 49 mg of 21 in 0.7 ml DMSO (distilled from $CaH_2$ at 8 mm and stored over 4A Linde molecule sieves) is added 100$\mu$l diisopropylamine (distilled from NaH under $N_2$ and stored over 4A Linde molecular sieves). The stoppered reaction mixture is stirred for a few minutes and then allowed to stand for 2 hours. The amine and most of the DMSO are then concentrated off under high vacuum with no external heating. The residue is passed quickly through a column of silica gel (packed, applied and eluted with EA) to remove residual DMSO. After concentration under a $N_2$ stream of all fractions having u.v. absorbance, the material is chromatographed on a thin layer silica gel plate (eluant = 50% EA/$CHCl_3$; repeated extraction of desired u.v. bands with a large volume of chloroform) to give 22.

*Step V*
Preparation of I:

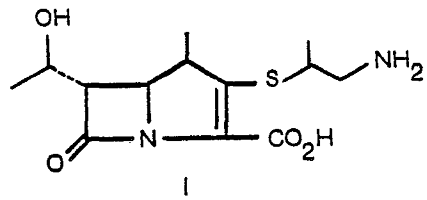

I

To 5.2 mg 22 is added 0.60 ml dioxane, 0.05 ml ethanol, 0.35 ml deionized water and 0.01 ml of 1.0M $K_2HPO_4$. The the resultant clear solution is added 5 mg of 10% Pd/C. The suspension is flushed with $N_2$, then 5—6 times alternately with 50 psi $H_2$ atmosphere for 30—40 minutes. After centrifugation, the Pd/C is washed and centrifuged 2—3X with 0.5 ml portions of deionized water. The combined centrifugates are extracted 5 × 1 – 2 ml ether. Residual ether is removed under vacuum and the aqueous solution is chromatographed on an XAD-2 column (20 × 140 mm) which is eluted with water to give the desired product I.

EXAMPLE 3

Preparation of Bis-(p-Nitrobenzyloxycarbonylamino-2-propyl)disulfide

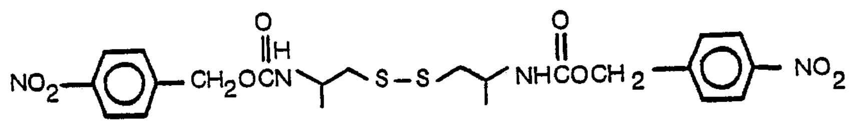

Under nitrogen at —20°C, bromide (1.21 ml, .022 mmole) is added to a solution of p-nitrobenzyloxycarbonylamino-2-propanethiol (11.28 g), in 100 ml of anhydrous tetrahydrofuran. The cooling bath is removed, and the cold solution is stirred for 15 minutes. The solution is then diluted with 400 ml ethyl acetate and washed with 200 ml 1M pH7 phosphate buffer, 200 ml 1M dibasic potassium phosphate, water (2 x 200 ml, 100 ml) and 200 ml brine. It is dried over anhydrous magnesium sulfate and filtered. The filtrate is evaporated *in vacuo* giving a yellow solid residue. This material is chromatographed on silica gel eluting with 5% ethyl acetate/chloroform to give crystalline product.

## EXAMPLE 4

Following the procedure of Example 2 Steps A—$G_a$, the following substituted azetidinones 10 are obtained when an equivalent amount of the appropriately substituted butadiene reagent is substituted for the 1-acetoxyl-2-methyl-1,3-butadiene of Example 2, Step A. The following chart gives the final axetidinone product, the necessary reagents and any pertinent remarks:

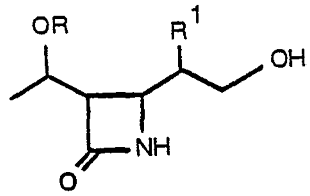

| Compound | R | $R^1$ | Butadiene used in Example 2, Step A |
| --- | --- | --- | --- |
| 1.) | 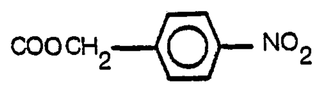 | $CH_3CH_2$– | 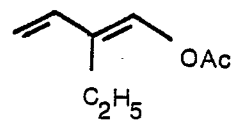 |
| 2.) | 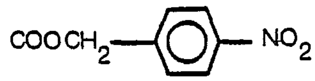 | $CH_3CH_2CH_2$ | 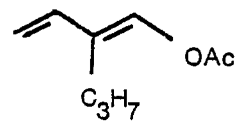 |
| 3.) | 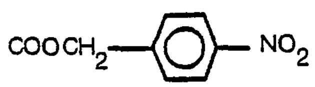 | 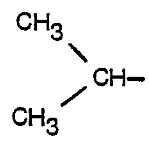 | 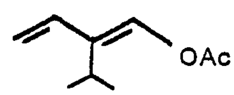 |
| 4.) | 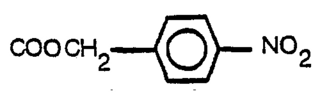 | 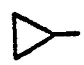 | 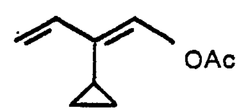 |
| 5.) | 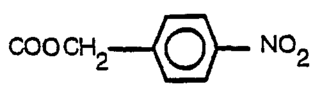 | Ph– | 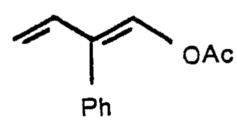 |
| 6.) | 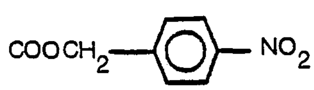 | $PhCH_2$– | 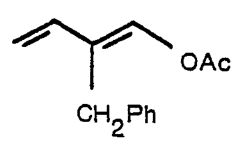 |

## EXAMPLE 5

Following the procedure of Example 2, Steps I—V, the following species of the present invention are obtained when the azetidinones 10 of Example 4 are substituted in equivalent amounts, respectively for the azetidinone of Example 2, Step A. The products of this Example are presented in tabular fashion below:

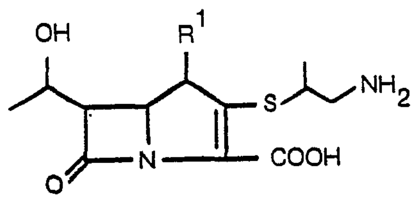

| Compound | $R^1$ |
|---|---|
| 1.) | $CH_3CH_2-$ |
| 2.) | $CH_3CH_2CH_2-$ |
| 3.) | 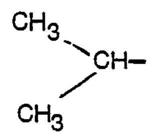 |
| 4.) | 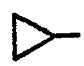 |
| 5.) | 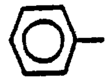 |
| 6.) | 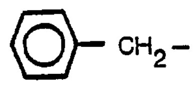 |

## EXAMPLE 6

Preparation of 8-oxo-2,2,5,7-tetramethyl-3-oxa-1-azabicyclo[4.2.0]octane

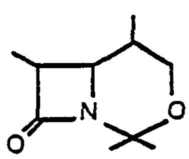

THF, (20 ml) is placed under $N_2$ treated with 1.54 ml diisopropylamine and cooled to −78°C. A solution of n-butyl lithium 1.97M in hexane 5.6 ml is added dropwise over 5 min. The reaction mixture is stirred at −78°C for 10 min and then treated with 8-oxo-2,2,5-trimethyl-3-oxa-1-azabicyclo[4.2.0]octane (1.55 g) in 15 ml THF added dropwise over 5 min. After another 10 min hexamethylphosphoramide 1.97 ml is added. The mixture is stirred another 10 min, then treated with 2 ml of methyl iodide. The reaction mixture is stirred at −78° for 15 min and allowed to warm to 25°C and stirred for 15 min. The reaction mixture is diluted with EtOAc, washed once with pH 7 phosphate buffer and dried and evaporated. The residue is chromatographed on silica gel using 25% EtOAc/$C_6H_6$ as eluant to give 8-oxo-2,2,5,7-tetramethyl-3-oxa-1-azabicyclo[4.2.0]octane.

## EXAMPLE 7

Preparation of 8-oxo-2,2,5,7-tetramethyl-7-(hydroxy-methyl)-3-oxa-1-azabicyclo[4.2.0]octane

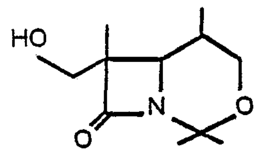

To a solution of 1.1 equivalents of freshly prepared lithium diisopropylamide in anhydrous tetrahydrofuran under a nitrogen atmosphere at −78° is added a solution of 8-oxo-2,2,5,7-tetramethyl-3-oxa-1-azabicyclo[4.2.0]octane in anhydrous tetrahydrofuran which has been cooled to −78°C. After two minutes, the resulting lithium enolate is treated with excess formaldehyde, introduced as a gas just above the surface of the stirred solution. The solution is stirred for 30 minutes at −78° and then poured into water. The aqueous phase is saturated with sodium chloride and extracted with ethyl acetate. The combined ethyl acetate solutions are dried over magnesium sulfate and filtered. The filtrate is evaporated under reduced pressure to give the crude product. Purification by chromatography on silica gel using ethyl acetate/benzene gives 8-oxo-2,2,5,7-tetramethyl-7-(hydroxymethyl)-3-oxa-1-azobicyclo[4.2.0]octane.

## EXAMPLE 8

Preparation of 8-Oxo-2,2,5,7-tetramethyl-7-(p-nitrobenzylcarbonyldioxymethyl)3-oxa-1-azabicyclo[4.2.0]octane

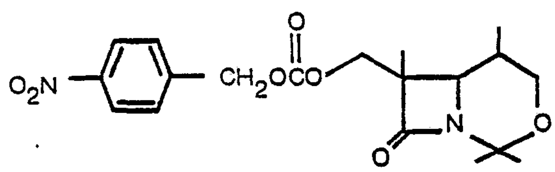

Under anhydrous conditions at 0°C. a solution of 8-oxo-2,2,5,7-tetramethyl-7-(hydroxymethyl)-3-oxa-1-azabicyclo[4.2.0]octane (60 mg) in 0.6 ml ether is treated with powdered potassium hydroxide (19 mg). After a period of 15 minutes, *p*-nitrobenzyl chloroformate (65 mg) is added to the reaction mixture. Stirring is continued at 25°C for an additional 15 hours. The mixture is partitioned between 1M pH7 phosphate buffer and more ether. The ether phase is washed with water and brine, dried over magnesium sulfate and filtered. Evaporation of the filtrate under reduced pressure gives 67 mg of a colorless oil. Purification by preparative thick-layer chromatography on silica gel developing with 1:9 ethyl acetate/benzene gives 8-oxo-2,2,5,7-tetramethyl-7-(p-nitrobenzylcarbonyldioxymethyl)-3-oxa-1-azabicyclo[4.2.0]octane as a mixture of diastereomers.

## EXAMPLE 9

Perparation of 3-methyl-3-(p-nitrobenzylcarbonyldioxymethyl)-4-(1-methyl-2-hydroxyethyl)-2-azetidinone

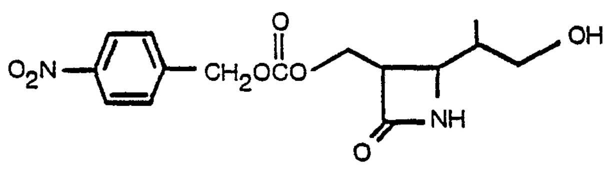

8-Oxo-3-oxa-2,2,5,7-tetramethyl-7-(1-p-nitrobenzylcarbonyldioxymethyl)-1-azabicycle[4.2.0]octane (1.0 g) is dissolved in 8 ml trifluoroacetic acid and 8 ml water and heated at 25°C for 15 min. The reaction mixture is neutralized with $KHCO_3$, and then extracted with EtOAc. The organic layer is separated, dried over $MgSO_4$ and evaporated *in vacuo*. The residue is taken up in benzene and evaporated to give 3-methyl-3-(p-nitrobenzylcarbonyldioxymethyl)-4-(1-methyl-2-hydroxyethyl)-2-azetidinone as a mixture of diastereoisomers.

EXAMPLES 10—13

Examples 10, 11, 12 and 13 complement Examples 6, 7, 8 and 9 for the preparation of 3 - methyl - 3 - (p - nitrobenzylcarbonyldioxymethyl) - 4 - (1 - methyl - 2 - hydroxyethyl) - 2 - azetidinone

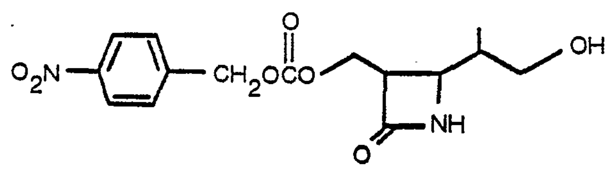

EXAMPLE 10

Preparation of 1 - (2 - Tetrahydropyranyl) - 3 - methyl - 4 - [1 - methyl - 2 - (2 - tetrahydropyranyl)oxyethyl] - 2 - acetidinone

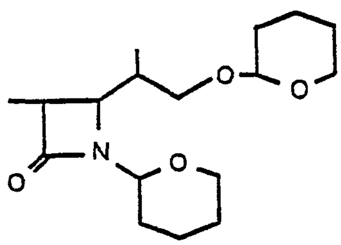

Under nitrogen and at 25°C, a solution of 3-methyl-4(1-methyl-2-hydroxyethyl)-2-azetidinone (62 mg) in 0.5 ml of anhydrous *p*-dioxane is treated with 2,3-dihydropyran (.98 ml) and *p*-toluenesulfonic acid monohydrate (19 mg). The resulting solution is stirred for a period of 60 minutes and then partitioned between 10 ml of 0.5M pH7 phosphate buffer and 10 ml of ethyl acetate. The aqueous phase is extracted a second time with ethyl acetate. The combined ethyl acetate solutions are washed with brine, dried over magnesium sulfate and filtered. The filtrate is evaporated under reduced pressure to give 216 mg of crude product. Purification by preparative thick-layer chromatography developing with ethyl acetate gives 80 ml of 1 - (2 - tetrahydro - pyranyl) - 3 - methyl - 4 - [1 - methyl - 2 - (2 - tetrahydropyranyl) - oxyethyl] - 2 - azetidinone as an oil.

EXAMPLE 11

Preparation of 1 - (2 - tetrahydropyranyl) - 3 - methyl - 3 - (1 - hydroxymethyl) - 4 - [1 - methyl - 2 - (2-tetrahydropyranyl) - oxyethyl] - 2 - azetidinone

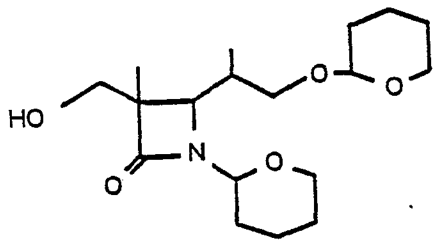

Following the procedure described for the preparation of 8-oxo-2,2,5,7 - tetramethyl - 7 - (hydroxymethyl) - 3 - oxa - 1 - azabicyclo[4.2.0]octane from 8 - oxo - 2,2,5,7-tetramethyl - 3 - oxa - 1 - azabicyclo[4.2.0]octane (Example 6, above) and using 3 - methyl - 1 - (2 - tetrahydropyranyl) - 4-[1 - methyl - 2 - (2 - tetrahydropyranyl) - oxyethyl] - 2 - azetidinone one obtains a diastereomeric mixture of 1 - (2 - tetrahydropyranyl) - 3 - methyl - 3 - (hydroxymethyl) - 4[1 - methyl - 2 - (2 - tetrahydropyranyl)oxyethyl] - 2 - azetidinone.

EXAMPLE 12

Preparation of 3 - Methyl - 1 - (2 - tetrahydropyranyl) - 3 - (1 - p - nitrobenzylcarbonyldioxymethyl) - 4- [1 - methyl - 2 - (2 - tetrahydropyranyl)oxyethyl] - 2 - azetidinone

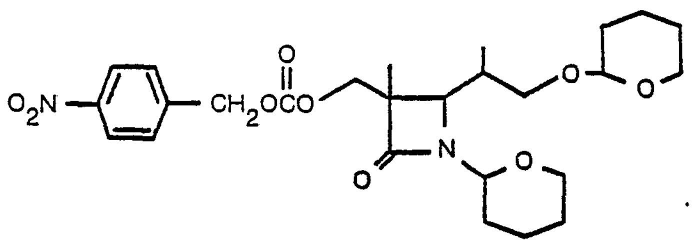

Following the procedure described for the preparation of 8 - oxo - 2,2,5,7 - tetramethyl - 7 - (1 - p - nitrobenzylcarbonyldioxymethyl) - 3 - oxa - 1 - azabicyclo[4.2.0]octane from 8 - oxo - 2,2,5,7 - tetramethyl - (1 - hydroxymethyl) - 3 - oxa - 1 - azabicyclo[4.2.0]octane and using 3 - methyl-1 - (2 - tetrahydropyranyl) - 3 - (hydroxymethyl) - 4 - [1 - methyl - 2 - (2 - tetrahydropyranyl)oxyethyl]-2 - azetidinone there is obtained 3 - methyl - 1 - (2 - tetrahydropyranyl) - 3 - (p - nitrobenzylcarbonyldioxymethyl) - 4 - [1 - methyl - 2 - (2 - tetrahydropyranyl)oxyethyl] - 2 - azetidinone.

EXAMPLE 13

Preparation of 3 - Methyl - 3 - (p - nitrobenzylcarbonyldioxymethyl) - 4 - (1 - methyl - 2 - hydroxyethyl) - 2 - azetidinone

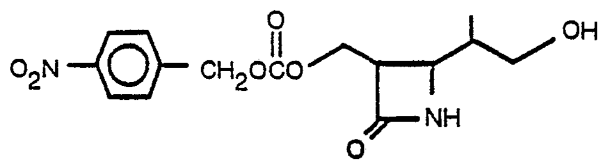

A solution of 3-methyl - 1 - (2 - tetrahydropyranyl) - 3 - (*p* - nitrobenzylcarbonyldioxymethyl)-4- [1 - methyl - 2 - (2 - tetrahydropyranyl)oxyethyl] - 2 - azetidinone in methanol at 25°C is treated with .1 molar equivalent of *p*-toluenesulfonic acid monohydrate. The solution is stirred for a period of 2 hours and then neutralized with 1M pH7 phosphate buffer. The product is extracted into ethyl acetate. The ethyl acetate solution is washed with brine, dried over magnesium sulfate and filtered. The filtrate is evaporated under reduced pressure to give 3 - methyl - 3 - 3(p - nitrobenzylcarbonyldioxymethyl) - 4 - (1 - methyl - 2 - hydroxyethyl) - 2 - azetidinone.

EXAMPLE 14

Preparation of 1,6 - dimethyl - 2 - (2 - aminoethylthio) - 6 - (hydroxymethyl) - 1 - carbapen - 2em - 3 - carboxylic acid

*Step A:*

Preparation of 12′

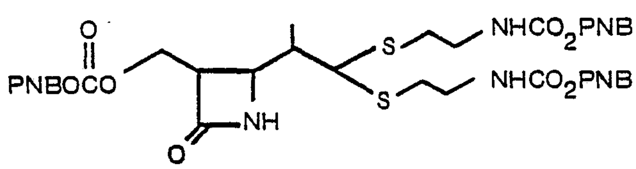

12′

To 6.75 ml anhydrous pyridine in 350 ml anhydrous acetonitrile is added 4.05 g anhydrous powdered chromium trioxide. After stirring at room temperature (25°C) for 30 minutes, 9.6 g dried Supercell is added and stirring is continued for 5 additional minutes. A solution of 3,21 g 3-methyl - 3 - (p - nitrobenzylcarbonyldioxymethyl) - 4 - (1 - methyl - 2 - hydroxyethyl) - 2 - azetidinone in 30 ml anhydrous acetonitrile is added all at once. The reaction mixture is stirred under anhydrous conditions at room temperature (25°C) for one hour. Addition of 9.6 g $NaHSO_3$ is followed by 5 minutes of stirring after which the reaction mixture is filtered through a mixed packed bed of 40 g silica gel and 40 g anhydrous magnesium sulfate. The bed is washed repeatedly with acetontrile (total volume of filtrate 600 ml). The filtrate is concentrated under a $N_2$ stream to 130 ml total volume. To this solution containing crude aldehyde at 0°C under $N_2$ is added 9.64 g *p*-nitrobenzyloxycarbonylaminoethanethiol as prepared below (Example 14, Step B). To the stirred reaction mixture is added 8.0 ml boron trifluoride etherate. After 1.5 hours at 0°C., the reaction mixture is poured into a stirred ice-cold mixture

of 69 g $K_2HPO_4$—500 ml $H_2O$ and 700 ml ethyl acetate (EA). The layers are separated, and the aqueous one is saturated with NaCl and re-extracted with additional EA. The combined organic layers are washed twice with brine, dried over anhydrous $MgSO_4$ and filtered. The filtrate is concentrated under a $N_2$ stream and then pumped on high vacuum to give crude 12′.

The material is chromatographed on 450 g silica gel (column height = 48 cm; diameter = 5.5 cm) packed and applied in $CHCl_3$ and eluted with increasing percentages of MeOH in $CHCl_3$ (0—4% MeOH/$CHCl_3$). Those fractions containing the desired product are combined, concentrated under a $N_2$ stream; and pumped on high vacuum to give 12′.

*Step B:*
Preparation of p-Nitrobenzyloxycarbonylaminoethanethiol

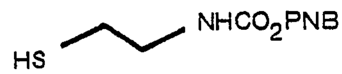

To 600 ml diethyl ether ($Et_2O$)-75 ml $H_2O$ in an ice bath with stirring is added 3.2 g cysteamine hydrochloride (mw = 114; 28.1 mmole). A solution of 7.14 g $NaHCO_3$ (mw = 84; 85 mmole) in 75 ml $H_2O$ is added. The ice bath is removed and at room temperature a solution of 6.75 g p-nitrobenzylchloroformate (mw = 216; 31.3 mmole) in 270 ml $Et_2O$ is added dropwise over a period of one hour. After 10 additional minutes, the layers are separated. The ether layer is extracted with 150 ml 0.25N HCl, and then with 200 ml brine. Each aqueous layer is then backwashed successively with 100 ml $Et_2O$. The combined $Et_2O$ layers are dried over anhydrous $MgSO_4$, filtered, and concentrated under a $N_2$ stream. The crystalline residue is slurried in a small amount of ether, filtered, and the pale yellow crystals are dried under high vacuum to give 4.7 g *p*-nitrobenzyloxycarbonylaminoethanethiol (65% yield).

NMR ($CDCl_3$) 8.18 (*d*, J = 8Hz, aromatic protons ortho to nitro), 7.47 (*d*, J = 8Hz, aromatic protons meta to nitro), 5.27 (—N$\underline{H}$—), 5.20 (*s*, —$CH_2$— Ø-p$NO_2$), 3.40 (*m*, —C$\underline{H}_2$—NH—), 2.67 (*m*, —C$\underline{H}_2$—SH), 1.35) (*t*, J = 8.5Hz, —S$\underline{H}$) in ppm downfield from TMS.
IR($CHCl_3$ solution) carbonyl- 1725 $cm^{-1}$

M.S.—molecular ion-256, (M-47) at 209, (m-136) at 120 +$CH_2$Øp$NO_2$ at 136.

*Step C:*
Preparation of 14′

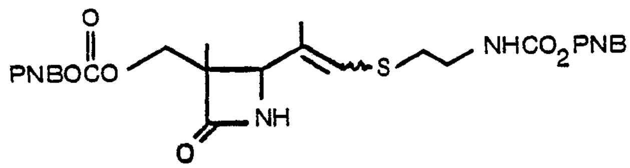

14′

To 14.2 ml pentane (dried over 4A Linde molecular sieves) is added 0.5 ml $Br_2$. To 5 g of 12′ in 58 ml tetrahydrofuran (THF) (freshly distilled from lithium aluminium hydride) (LAH) and 65 ml $Et_2O$ (dried over 3A 1/16″ Linde molecular sieves) at 0°C under $N_2$ with stirring is added dropwise 10 ml of the above 0.66M $Br_2$ solution. After 10 minutes at 0°C, 0.67 ml cyclohexene is added. After 5 minutes at 0°C., 1.7 ml triethylamine is added immediately followed by 40 ml ice-cold dimethylformamide (DMF) (distilled from anhydrous $CaSO_4$ at 40 mm and stored over 4A Linde molecular sieves). The ice bath is removed, and stirring is continued for 2 1/4 hours at room temperature. The reaction mixture is poured into a stirred ice-cold mixture of 12.6 ml 1M $KH_2PO_4$ 160 ml $H_2O$—500 ml (EA). After separation of the layers, the aqueous one is saturated with sodium chloride and re-extracted with EA. The combined organic layers are extracted once with brine, dried over anhydrous $MgSO_4$, filtered and concentrated under $N_2$ stream followed by pumping under high vacuum to provide crude 14′.

The material is chromatographed on 250 g silica gel (height = 45 cm; diameter = 4.5 cm) packed and applied in $CHCl_3$ and eluted with increasing percentages of MeOH in $CHCl_3$ (0—3% MeOH/$CHCl_3$). Those fractions containing clean product are combined, concentrated under a $N_2$ stream, and pumped on high vacuum to give 14′.

*Step D:*
Preparation of 15′

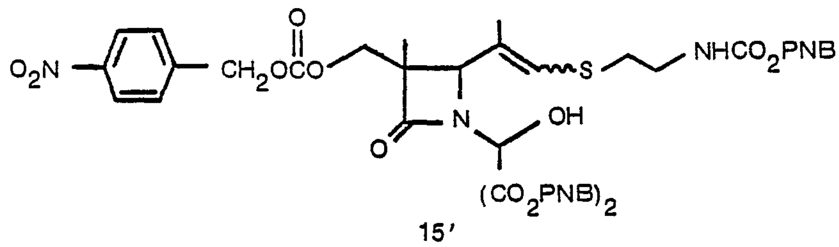

15′

To a stirred solution of 2.48 g di(p-nitrobenzyl)ketomalonate in 400 ml hot anhydrous toluene is added a solution of 2.52 g of 14′ in 20 ml THF (distilled from LAH) and 40 ml anhydrous toluene. After some of the solvent is boiled off, additional anhydrous toluene is added, and the azeodrying process is repeated three times. The solution is then refluxed under $N_2$ for 30 minutes. Additional toluene is then allowed to boil off yet the volume is not allowed to diminish so much that precipitation occurs. Total heating time is approximately 2 1/2 hours. The clear yellow reaction mixture is removed from the oil bath and placed under a stream of $N_2$ which instantaneously causes clouding. After concentration to a yellow oil, the residue is dissolved in $CH_2Cl_2$, dried of anhydrous $MgSO_4$, filtered, and concentrated under a $N_2$ stream to give crude 15′.

The material is chromatographed on 250 g silica gel packed and applied in $CHCl_3$ (height = 43 cm; diameter = 4.5 cm). Elution with 500 ml 0.5% MeOH/$CHCl_3$ is followed by continued elution with 1% MeOH/$CHCl_3$ for the remainder of the chromatography. After the emergence of excess reagent, those fractions containing pure 15′ are combined, concentrated under a $N_2$ stream and then on high vacuum to give 15′.

*Step E:*
Preparation of 17′

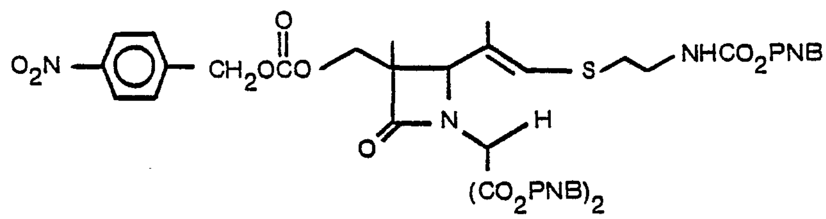

A solution of 1.468 g of 15′ in $CH_2Cl_2$ is dried over anhydrous $MgSO_4$, filtered, concentrated under a $N_2$ stream, and dried further under high vacuum just prior to the following reaction. To a solution of 15′ in 24 ml THF (freshly distilled from LAH) at −20°C is added 0.206 ml anhydrous pyridine. With stirring under $N_2$ 294 mg of freshly distilled thionyl chloride in 5 ml THF is added dropwise. The reaction mixture is stirred for 10 minutes at −20°C., the 1/2 hour at 0°C and finally 1 hour at 25°C. The pyridine hydrochloride is filtered under $N_2$ and washed with 20 ml THF. The filtrate is concentrated under $N_2$ stream followed by pumping on high vacuum.

To this freshly prepared chloro compound is added with stirring a freshly shaken suspension of 678 mg tributylphosphine in 36.5 ml 9:1 DMF—$H_2O$ followed by 294 mg $K_2HPO_4$. The reaction mixture is stirred at 25°C., for 35 minutes. After dilution with 120 ml EA and 60 ml brine, the layers are separated, and the aqueous one is extracted two times with EA. The combined organic layers are washed one time with brine, dried over anhydrous $MgSO_4$, filtered and concentrated under a $N_2$ stream followed by pumping on high vacuum to give crude 17′.

The material is chromatographed on 100 g silica gel (height = 28.5 cm; d = 4 cm) packed and applied in $CHCl_3$ and eluted with 0.5% MeOH in $CHCl_3$. Those fractions containing clean product are combined, concentrated under a $N_2$ stream and then on high vacuum to give 17′.

*Step F:*
Preparation of 19′

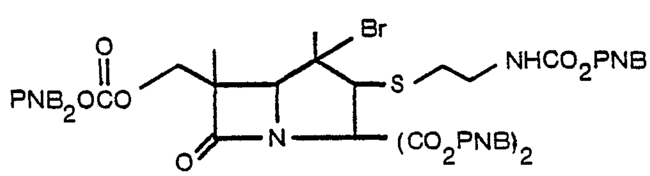

19′

To 8.5 ml pentane (dried over 4A Linde molecular sieves) is added 0.2 ml $Br_2$. To 0.706 g of 17′ in

18 ml THF (freshly distilled from LAH) and 5.7 ml $Et_2O$ (dried over 3A 1/16" Linde molecular sieves) at 0°C under $N_2$ with stirring is added dropwise 1.8 ml of the above 0.45M $Br_2$ solution. After 15 minutes at 0°C., 0.42 ml triethylamine is added immediately followed by 10.5 ml ice-cold DMF distilled from $CaSO_4$ at 40 mm and stored over 4A Linde molecular sieves). The ice-bath is removed, and stirring at room temperature is continued for 2 hours. The reaction mixture is poured into a stirred ice-cold mixture of 3.1 ml 1M $KH_2PO_4$—70 ml $H_2O$—100 ml EA. The layers are separated, and the aqueous one is saturated with NaCl and re-extracted with EA. The combined organic layers are washed once with brine, dried over anhydrous $MgSO_4$, and filtered. The filtrate is concentrated under a $N_2$ stream and then pumped on high vacuum to give crude 19'.

The material is chromatographed on 60 g silica gel (diameter = 2.8 cm) packed and applied in $CHCl_3$ and is eluted with 100 ml—2% EA/$CHCl_3$; 100 ml—4% EA/$CHCl_3$ and then 5% EA/$CHCl_3$ for the remainder of the chromatography. The fractions containing pure 19' are combined, concentrated under a $N_2$ stream, and pumped on high vacuum to give 19'.

*Step G:*
Preparation of 20'

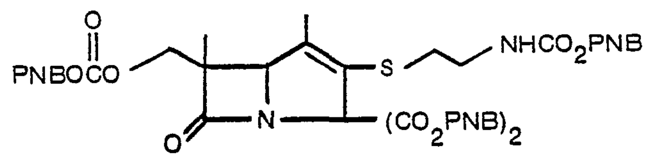

20'

To 29 mg anhydrous silver fluoride is added a solution of 146 mg of 19' in 3.5 ml anhydrous pyridine. The stoppered reaction mixture is stirred at room temperature in the dark for one hour and then poured into 20 ml cold water — 30 ml EA. After separation of the layers, the aqueous one is extracted two times with EA and one time with $CHCl_3$. Each organic layer is extracted one time with $H_2O$ and one time with brine. The combined organic layers are dried over anhydrous $MgSO_4$, filtered, and concentrated under a $N_2$ stream following by pumpiing on high vacuum to give crude 20'.

Preparative thin layer chromatography (eluant = 40% acetone-hexane; repeated extraction of desired u.v. band with a large volume of $HCCl_3$) yields slightly contaminated 20'. Re-chromatographing on silica gel using EA in $CHCl_3$ as an eluing system gives 20'.

*Step H:*
Preparation of 21'

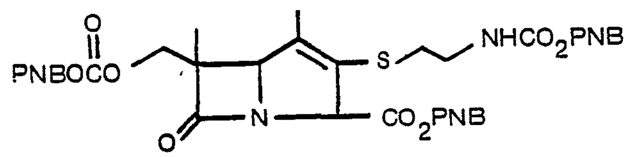

21'

A solution of 77 mg of 20' in 0.9 ml S-collidine (distilled from powdered KOH) is added to 13.4 mg anhydrous LiI (dried for few hours at 100°C over $P_2O_5$ under vacuum). With stirring under $N_2$, the reaction mixture is heated in an oil bath at 120°C. After a total of 30 minutes, the reaction mixture is cooled to 25°C., diluted with $CH_2Cl_2$, and transferred to a round bottom flask for concentration under a $N_2$ stream and then on high vacuum. Partitioning the residue between EA—$H_2O$ and 1 ml 1M $KH_2PO_4$ is followed by extraction of the aqueous layer two additional times with EA and one time with $CHCl_3$. Each organic layer is then backwashed with brine. The combined organic layers are dried over anhydrous $MgSO_4$, filtered, concentrated under a $N_2$ stream and then on high vacuum to give crude 21'.

Preparative thin layer chromatography on silica gel (plate is eluted two times 40% acetone/hexane; repeated extraction of the appropriate u.v. bands with large volume of $CHCl_3$) yields 21'.

*Step I:*
Preparation of 22'

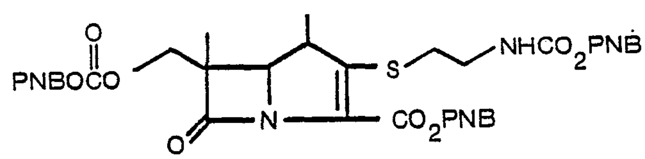

22'

To 49 mg of 21' in 0.7 ml DMSO (distilled from $CaH_2$ at 8 mm and stored over 4A Linde molecular

sieves) is added 100 $\mu$l diisopropylamine (distilled from NaH under $N_2$ and stored over 4A Linde molecular sieves). The stoppered reaction mixture is stirred for a few minutes and then allowed to stand for 2 hours. The amine and most of the DMSO are then concentrated off under high vacuum with no external heating. The residue is passed quickly through a column of silica gel (packed, applied, and eluted with EA) to remove residual DMSO. After concentration under a $N_2$ stream of all fractions having u.v. absorbance, the material is chromatographed on a thin layer silica gel plate (eluant = 50% EA/$CHCl_3$; repeated extraction of desired u.v. bands with a large volume of chloroform) to give 22′.

*Step J:*
Preparation of (I)

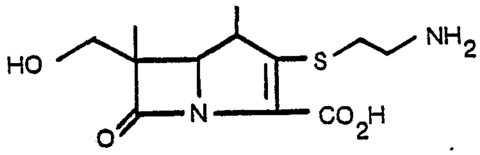

(I)

To 5.2 mg 22′ is added 0.60 ml dioxane, 0.05 ml ethanol, 0.35 ml deionized water and 0.01 ml of 1.0M $K_2HPO_4$. To the resultant clear solution is added 5 mg of 10% Pd/C. The suspension is flushed with $N_2$, then 5—6 times alternately with 50 psi $H_2$ and vacuum. Finally, it is shaken under a 50 psi $H_2$ atmosphere for 30—40 min. After centrifugation, the Pd-C is washed and centrifuged 2—3X with 0.5 ml portions of deionized water. The combined centrifugates are extracted 5 x 1 − 2 ml ether. Residual ether is removed under vacuum and the aqueous solution applied to an XAD-2 column (20 x 140 mm). Fractions of 100 drops (6—7 ml) are collected, with continuous UV monitoring, by elution with deionized water to give (I).

Example 15

Preparation of Bis (*p*-Nitrobenzyloxycarbonylaminoethyl)disulfide

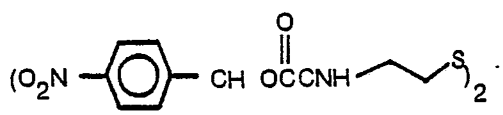

Under nitrogen at −20°C. bromine (1.21 ml., .022 mmole) is added to a solution of *p*-nitrobenzyloxycarbonylaminoethanethiol (11.28 g, .044 mole) in 100 ml of anhydrous tetrahydrofuran. The cooling bath is removed, and the cold solution is stirred for 15 minutes. The solution is then diluted with 400 ml ethyl acetate and washed with 200 ml 1M pH 7 phosphate buffer, 200 ml 1M dibasic potassium phosphate, water (2 x 200 ml, 100 ml) and 200 ml brine. It is dried over anhydrous magnesium sulfate and filtered. The filtrate is evaporation *in vacuo* giving a yellow solid residue. This material is chromatographed on silica gel eluting with 50% ethylacetate/chloroform to give 10.5 g of crystalline bis (*p*-nitrobenzyloxycarbonylaminoethyl)disulfide:

IR ($CH_2Cl_2$) $\mu$ : 3.04NH
5.96 carbonyl
6.22, 6.61 nitro

NMR ($CDCl_3$) : 8.24, 7.54 } d, J=8.5Hz, Ar$\underline{H}$
5.37, broad s, N$\underline{H}$
5.26, s, ArC$\underline{H}_2$O
3.60, q, J=6Hz and 6Hz, NHC$\underline{H}_2$$CH_2$
2.86, t, J=6Hz, $NHCH_2$C$\underline{H}_2$S

Example 16

Preparation of 8-oxo-2,2,5-trimethyl-7$\alpha$ and $\beta$-hydroxymethyl-3-oxa-1-azabicyclo[4.2.0]octane

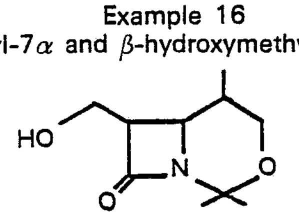

The procedure of Example 7, substituting 8-oxo-2,2,5-trimethyl-3-oxa-1-azabicyclo[4.2.0]octane

for 8-oxo-2,2,5,7-tetramethyl-3-oxa-1-azabicyclo[4.2.0]octane, is carried out. Upon purification by silica gel chromatography, the title compound is obtained.

Example 17

Preparation of 1-methyl-2-(2-aminoethylthio)-6-hydroxymethyl-1-carbadethiapen-2-em-3-carboxylic acid

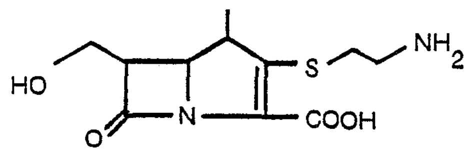

Following the procedure described above for the preparation of 1,6-dimethyl-2-(2-aminoethylthio)-6-hydroxymethyl-1-carbadethiapen-2-em-3-carboxylic acid, except that in Example 8 an equivalent amount of 8-oxo-2,2,5-trimethyl-7-hydroxymethyl-3-oxa-1-azabicyclo[4.2.0]octane rather than the 2,2,5,7-tetramethyl species is taken; the title compound is obtained when the procedures of Examples 8, 9 and 14 are followed.

Example 18

Preparation of 8-oxo-2,2,5-trimethyl-7$\alpha$ and $\beta$-(1-hydroxy-2-phenylethyl)-3-oxa-1-azabicyclo[4.2.0]-octane

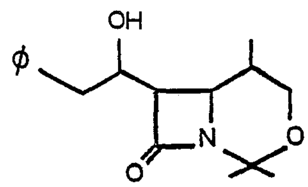

Following the procedure of Example 2, Step Fa except that instead of acetaldehyde, an equivalent amount of phenylacetaldehyde is used, upon purification by silica gel chromatography, the title compound is obtained.

Example 19

Preparation of 1-methyl-(2-aminoethylthio)-6-(1-hydroxy-2-phenylethyl)-1-carbadethiapen-2-em-3-carboxylic acid

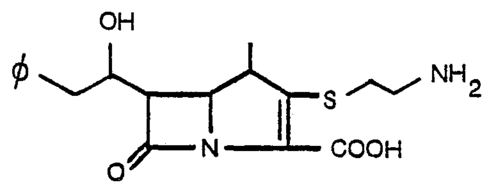

Following the procedure of Example 8 except that 8-oxo-2,2,5-trimethyl-7-(1-hydroxy-2-phenylethyl)-3-oxa-1-azabicyclo[4.2.0]octane is substituted in equivalent amount for its analogous substrate, the title compound is obtained.

Example 20

Preparation of 8-oxo-2,2,5-trimethyl-7$\alpha$-benzyl-3-oxa-1-azabicyclo[4.2.0]octane

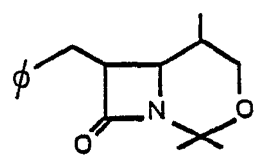

Following the procedure of Example 6 and using benzyl bromide instead of methyl iodide there is obtained 8-oxo-2,2,5-trimethyl-7$\alpha$-benzyl-3-oxa-1-azabicyclo[4.2.0]octane.

Example 21

Preparation of 8-oxo-2,2,5-trimethyl-7$\alpha$ and $\beta$-benzyl-7$\beta$ and $\alpha$-(1-hydroxyethyl)-3-oxa-1-azabicyclo-[4.2.0]octane

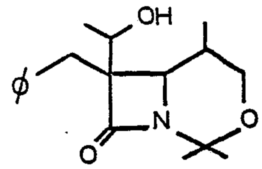

Using the procedure of Example 7 but substituting an equivalent amount of 8-oxo-2,2,5-trimethyl-7$\alpha$-benzyl-3-oxa-1-azabicyclo[4.2.0]octane (Example 20) for 8-oxo-2,2,5,7-tetramethyl-3-oxa-1-azabicyclo-[4.2.0], and an equivalent amount of acetaldehyde for formaldehyde, there is obtained, upon purification by silica gel chromatography, the title compounds.

Example 22

Preparation of 8-oxo-2,2,5-trimethyl-7$\alpha$(1-mesyloxyethyl)-3-oxa-1-azabicyclo[4.2.0]octane

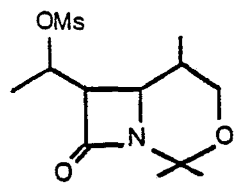

Under nitrogen at 0° a solution of 8-oxo-2,2,5-trimethyl-7,1-hydroxyethyl-3-oxa-1-azabicyclo-[4.2.9]octane )128 mg) and triethylamine (134 $\mu$l) in 5 ml of sieve dried methylene chloride is treated with redistilled methane sulfonyl chloride (55 $\mu$l). The resulting solution is stirred for 30 minutes. It is then washed with 10 ml each of cold water, 1 molar pH 3 phosphate buffer, 5% sodium bicarbonate, water and finally brine. The organic phase is dried over magnesium sulfate and filtered. The filtrate is evaporated *in vacuo* to give the crude product. This material is chromatographed on preparative thick layer silica gel plates developed with acetone/hexane to give a separated diastereomeric mesylate.

Example 23

Preparation of 8-oxo-2,2,5-trimethyl-7$\alpha$-(1-azidoethyl)-3-oxa-1-azabicyclo[4.2.0]octane

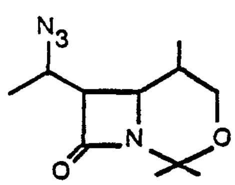

A solution of lithium azide, prepared by stirring over night a mixture of 170 mg sodium azide and 100 mg lithium chloride in 3 ml of anhydrous dimethyl sulfoxide, followed by filtration, is added to 160 mg of 8-oxo-2,2,5-trimethyl-7$\alpha$[1-mesyloxyethyl)-3-oxa-1-azabicyclo[4.2.0]octane and stirred under nitrogen at 25°C for eight hours. The mixture is then poured into 10 ml ice water and extracted with ethyl acetate, the combined extracts washed once with water, once with saturated sodium chloride solution, dried ($MgSO_4$) and evaporated under a nitrogen stream. The crude product is purified by preparative tlc to afford the title compound.

Example 24

Preparation of 8-oxo-2,2,5-trimethyl-7$\alpha$-(1-*p*-nitrobenzyloxycarbonylaminoethyl)-3-oxa-1-azabicyclo-[4.2.0]octane

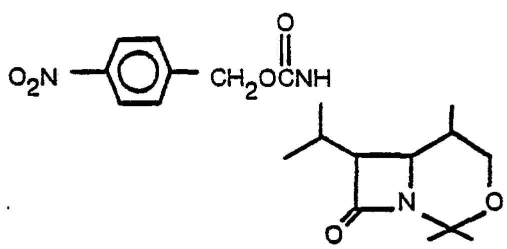

A mixture of 107 mg of 8-oxo-2,2,5-trimethyl-7$\alpha$-(1-azidoethyl)-3-oxa-1-azabicyclo[4.2.0]-octane and 50 mg of 10% Pd/C in 2 ml of anhydrous dioxane is shaken under 3 atmospheres of hydrogen for 1.5 hours. The mixture is filtered and concentrated to 1 ml under a nitrogen stream, then treated with 1 ml of 1M $K_2HPO_4$ and 1 ml methylene chloride. The mixture is stirred vigorously in an ice bath under nitrogen while a solution of 120 mg of p-nitrobenzylchloroformate in 0.5 ml methylene chloride is added dropwise over one minute. The solution is stirred for another 15 minutes, then treated with 0.1 ml pyridine in 2 ml water and stirred vigorously for another 15 minutes. The organic phase is removed and the aqueous phase is extracted several more times with methylene chloride. The combined organic phases are washed twice with water, once with saturated NaCl, dried ($MgSO_4$) and evaporated under reduced pressure. Purification by preparative tlc affords the title compound.

Example 25

Preparation of 8-oxo-2,2,5-trimethyl-7(1-*o*-nitrobenzylthioethyl)-3-oxa-1-azabicyclo[4.2.0]octane

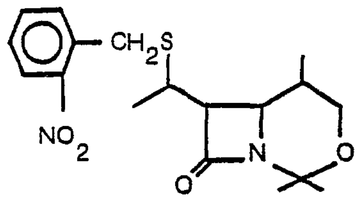

When in Example 23 an equivalent solution of *o*-nitrobenzyl mercaptan in DMF is substituted for the lithium azide solution, the title compound is obtained.

Example 26

Preparation of 4-(1-methyl-2,2-bisbenzylthioethyl)-3-methyl-3(p-nitrobenzylcarbonyldioxymethyl)-2-azetidinone

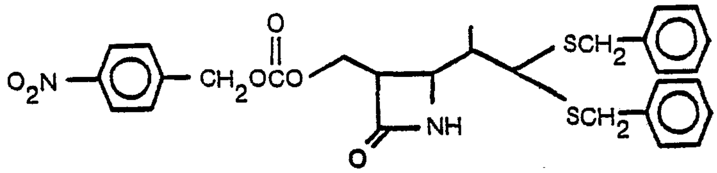

When in Example 14, Step A, an equivalent amount of benzyl mercaptan is substituted for 2-(*p*-nitrobenzyloxycarbonylamino)ethane thiol, the title compound is obtained.

Example 27

Preparation of 3-methyl-4-[1-methyl-2,2-bis-(1′,1′-dimethyl-*p*-nitrobenzoxycarbonylaminoethylthio]-3-(p-nitrobenzylcarbonyldioxymethyl)-2-azetidinone

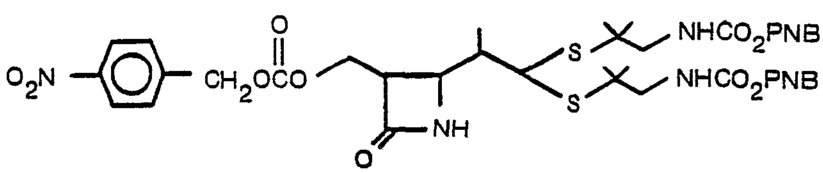

When in Example 14, Step A, an equivalent amount of 2-(p-nitrobenzyloxycarbonylamino)-1,1-dimethylethanethiol is substituted for 2-(p-nitrobenzyloxycarbonylamino) ethane thiol, the title compound is obtained.

Example 28

Preparation of 1,6-dimethyl-2-benzylthiohydroxymethyl-1-carbadethiapen-2-em-3-carboxylic acid

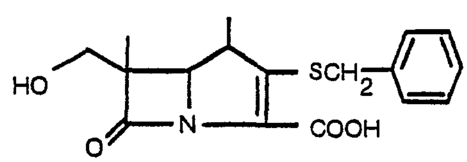

Following the procedure of Example 14, Steps A—K, except substituting for the indicated azetidinone the azetidinone of Example 26, the title compound is obtained.

Example 29

Preparation of 1,6-dimethyl-2-(2-amino-1,1-dimethylethylthio)-6-(1-hydroxymethyl)-1-carbadethiapen-2-em-3-carboxylic acid

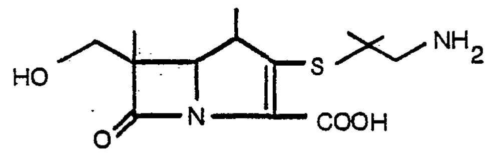

Following the procedure of Example 14, step A—K, except substituting for the indicated azetidinone the azetidinone of Example 27, the title compound is obtained.

## EXAMPLE 30

Following the procedure of the foregoing Examples and text, the following representative compounds of the present invention (Table I) are obtained by analogy.

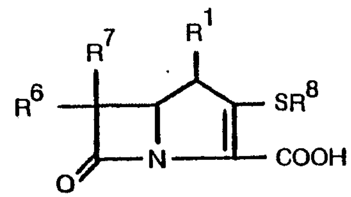

I

| Compound | $R^8$ | $R^6$ | $R^7$ | R′ | Remarks |
|---|---|---|---|---|---|
| 1.) | $-(CH_2)_3NH_2$ | H | $CH_2OH$ | $CH_3$ | $HS(CH_2)_3NHCO_2PNB$, Example 17. |
| 2.) | 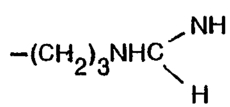 | H | $CH_2OH$ | $CH_3$ | From Compound 1. of Example 30 in reaction with methyl formimidate hydrochloride in water at pH 8.5. |
| 3.) | 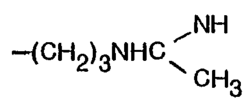 | H | $CH_2OH$ | $CH_3$ | From Compound 1 of Example 30 in reaction with ethyl acetimidate hydrochloride in water at pH 8.5. |
| 4.) | 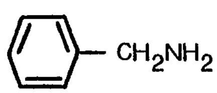 | H | $CH_2OH$ | $CH_3$ | From 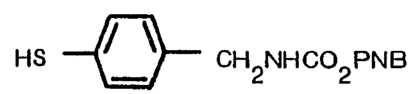 Example 17. |

EXAMPLE 30 (Continued)

| Compound | $R^8$ | $R^6$ | $R^7$ | R′ | Remarks |
|---|---|---|---|---|---|
| 5.) | 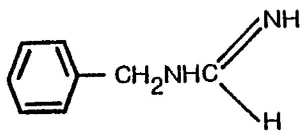 | H | $CH_2OH$ | $CH_3$ | As in 2., above. |
| 6.) | 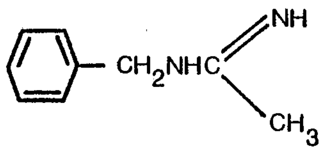 | H | $CH_2OH$ | $CH_3$ | As in 3., above. |
| 7.) | 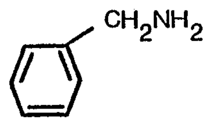 | H | $CH_2OH$ | $CH_3$ | From HS 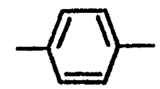 $CH_2NHCO_2PNB$, Example 17. |
| 8.) | 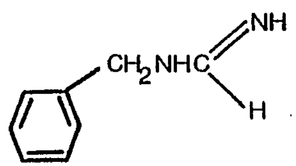 | H | $CH_2OH$ | $CH_3$ | As in 2., above. |
| 9.) | 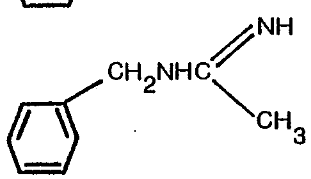 | H | $CH_2OH$ | $CH_3$ | As in 3., above. |

EXAMPLE 30 (Continued)

| Compound | $R^8$ | $R^6$ | $R^7$ | R' | Remarks |
|---|---|---|---|---|---|
| 10.) | 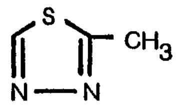 | H | $CH_2OH$ | $CH_3$ | From 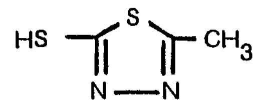<br>Example 17. |
| 11.) | $-CH-CH_2-NH_2$<br>\|<br>$CH_3$ | H | $CH_2OH$ | $CH_3$ | From $HSCH(CH_3)CH_2NHCO_2PNB$, Example 17. |
| 12.) | $-CH_3$ | H | $CH_2OH$ | $CH_3$ | From $HSCH_3$, Example 17. |
| 13.) | 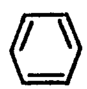 | H | $CH_2OH$ | $CH_3$ | From HSO, Example 17. |
| 14.) | 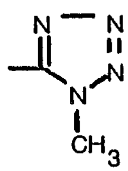 | H | $CH_2OH$ | $CH_3$ | From: 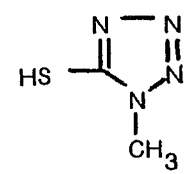<br>Example 17. |

EXAMPLE 30 (Continued)

| Compound | $R^8$ | $R^6$ | $R^7$ | R′ | Remarks |
|---|---|---|---|---|---|
| 15.) | $NH_2$<br>$-CH_2CHCH_3$ | H | $CH_2OH$ | $CH_3$ | From $HSCH_2CH(CH_3)NHCO_2$ PNB, Example 17. |
| 16.) | $NH_2$<br>$-CH_2CHCH_3$ | H | $CH_2OH$ | $CH_3$ | From $HSCH_2CH(CH_3)NHCO_2PNB$ Example 17. |
| 17. | 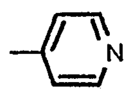 | H | $CH_2OH$ | $CH_3$ | From 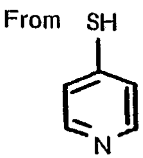<br>Example 17. |
| 18.) | $-SCH_2CH_2NH_2$ | H | $CH_2OH$ | $CH_3$ | Example 17. |
| 19.) | $-SCH_2CH_2NH_2$ | H | $NH_2$<br>$CH_3CH-$ | $CH_3$ | From azetidinone of Example 24, followed the procedure of Example 17. |
| 20.) | $-SCH_2CH_2NH_2$ | H<br>H | Cl<br>$CH_3CH-$ | $CH_3$ | From azetidinone of Example 22, followed the procedure of Example 23 except substituting LiCL for $LiN_3$ and Example 17. |

EXAMPLE 30 (Continued)

| Compound | $R^8$ | $R^6$ | $R^7$ | R' | Remarks |
|---|---|---|---|---|---|
| 21.) | $-SCH_2CH_2NH_2$ | $CH_3$ | OH<br>$CH_3CH-$ | $CH_3$ | From azetidinone of Example 6 followed the procedure of Example 7 except substituting acetaldehyde for formaldehyde and Example 17. |
| 22.) | SØ | $CH_3$ | $NH_2$<br>$CH_3CH-$ | $CH_3$ | From azetidinone of Example 6, followed Example 17 except substituting thiophenol for cysteamine. |
| 23.) | $-SCH_2CH_2CH_2NH_2$ | $CH_3$ | OH<br>$CH_3CH$ | $CH_3$ | Same as compound 21, except using aminopropanethiol instead of aminoethanethiol. |
| 24.) | –SØ | $CH_3$ | OH<br>$CH_2CH$ | $CH_3$ | Same as compound 21, except using thiophenol instead of aminoethanethiol. |
| 25.) | $-SCH_2CH_2NH_2$ | H | $CH_2OH$ | $C_2H_5$ | Same as for (18). except replacing 1-acetoxyl-2-methyl-1,3-butadiene with 1-acetoxyl-2-ethyl-1,3-butadiene in Example 2, Step A. |
| 26.) | $-SCH_2CH_2NH_2$ | $CH_3$ | OH<br>$CH_3CH$ | $CH_3$<br>$CH_3$ CH | Same as for (21), except replacing 1-acetoxyl-2-methyl-butadiene with 1-acetoxyl-2-isopropyl-butadiene. |

Example 31

Following the foregoing procedure, the following species of the present invention are obtained by analogy.

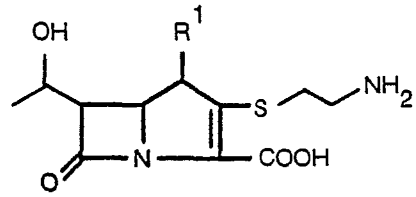

| Compound | $R^1$ |
|---|---|
| 1.) | $CH_3CH_2-$ |
| 2.) | $CH_3CH_2CH_2-$ |
| 3.) | 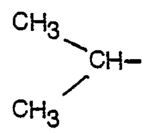 |
| 4.) | 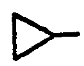 |
| 5.) | 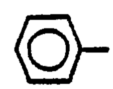 |
| 6.) | 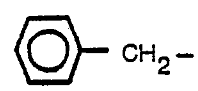 |
| 7.) | $CH_3$ |

Example 32

Preparation of 6-(1-hydroxyethyl)-1-methyl-1-carbadethiapen-2-em-3-carboxylic acid

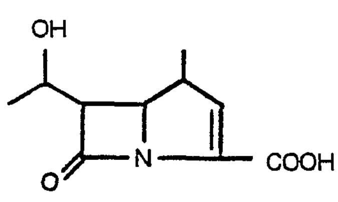

Preparation of R-trans-1β:

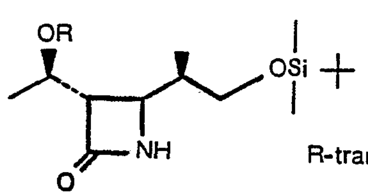

R-trans-1β

The starting material R-trans-9′β (1.58 g, 4.5 mmol) is treated with 5 equivalents of t-butyldimethylchlorosilane, 10 equivalents of imidazole in 5 ml anhydrous N,N-dimethylformamide (DMF) at room temperature for 3 hrs. The mixture is allowed to evaporate *in vacuo* to give crude product. Purification of the crude product by a silica gel eluting with 30% ethylacetate/cyclohexane gives 2.0 g of the product (R-trans-1β),

NMR (300 MHz, $CDCl_3$): 0.04 (s), 0.88 (s), 0.98 (d), 1.26 (d), 1.82 (m), 3.20 (q), 3.60 (m), 5.15 (m), 5.59 (q), 5.94 (s), 7.54 (t), 7.68 (m) and 8.18 (d) ppm. (R is *o*-nitrobenzyloxycarbonyl.)

Preparation of 2

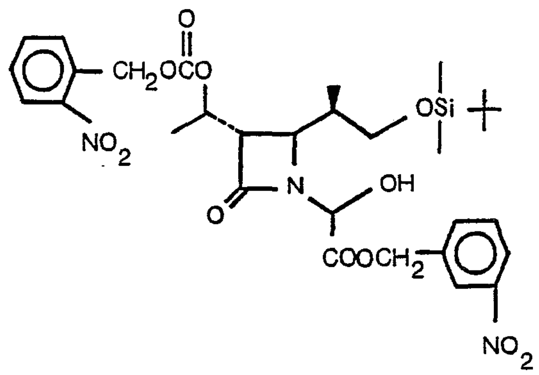

o-Nitrobenzyl-d-tartarate (1.8 g) is oxidized with periodic acid (0.97 g) in 18 ml of anhydrous tetrahydrofuran at 25°C for 30 min. The mixture is filtered from solids and the filtrate is allowed to evaporate *in vacuo* to give o-nitrobenzylglyoxylate which is then taken up in 100 ml benzene and transferred into a 250-ml round bottom flask. To the solution is added *trans*-3-[1(R)-*o*-nitrobenzyloxycarbonyloxyethyl]-4-[1β-methyl-2-t-butyldimethylsilyloxy)ethyl]-2-azetidinone (R-trans-1β) (2.0 g). The mixture is heated at reflux and water removed with a Dean-Stark trap packed with $CaH_2$ (1 g). for 6 hr. The mixture is cooled, filtered, evaporated and chromatographed on silica gel eluting with 30% ethylacetate/cyclohexane to give 2.

Preparation of 3 and 4

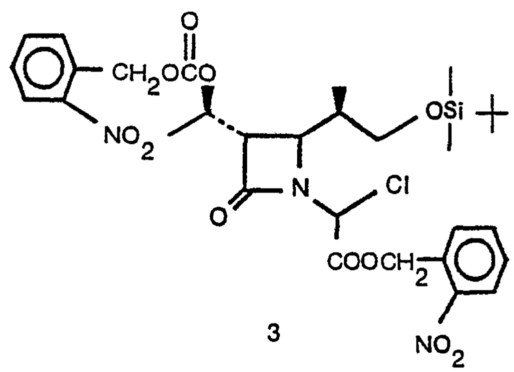

3

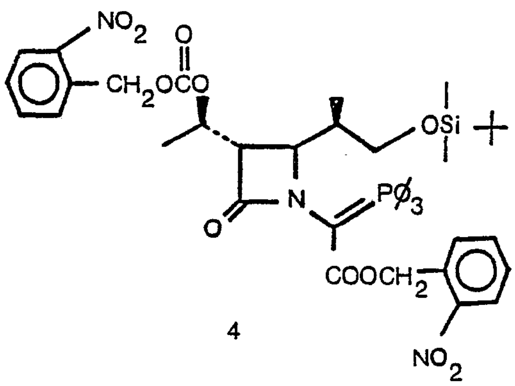

4

*Trans*-1-(o-nitrobenzyloxycarbonylhydroxymethyl)-3-[1(R)-o- nitrobenzyloxycarbonyloxyethyl] - 4-[(1β-methyl-2-t-butyldimethylsilyloxy)ethyl]-2-azetidinone (2) (3.92 g) in 20 ml anhydrous tetrahydrofuran at −20°C is treated with pyridine (0.42 ml) and thionyl chloride (0.37 ml). The mixture is allowed to warm to 25°C with stirring, then filtered from solids. After removal of solvent *in vacuo*, product 3 is obtained. The chloride 3 is redissolved in 25 ml anhydrous DMF and treated with triphenylphosphine (1.1 g) with stirring at 25°C for 1 hr. Solvent is removed *in vacuo* and the residue is dissolved in 100 ml methylene chloride and washed with 0.1N pH 7.2 phosphate buffer 30 ml; chromatographic purification on silica gel, eluting with 40% ethylacetate/cyclohexane, gives product 4 (1.0 g),

IR ($CHCl_3$): 1750 $cm^{-1}$ (β-lactam), 1620 $cm^{-1}$ (ylide ester).

Preparation of 5

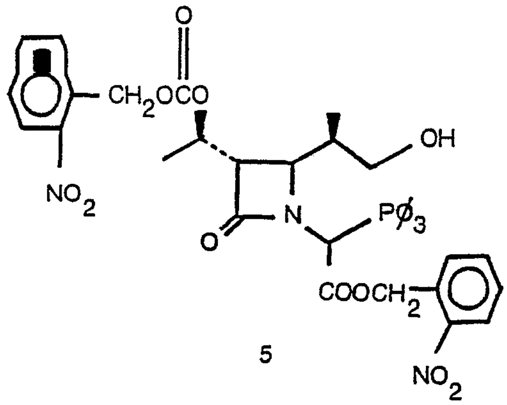

5

*Trans*-1 - (o - nitrobenzyloxycarbonyltriphenylphosphoranylmethyl) - 3 - [1(R) - o - nitrobenzyloxycarbonyloxyethyl]-4-[1β-methyl-2-t-butyldimethylsilyloxy)ethyl]-2-azetidinone (4) (1.0 g) is dissolved in 10 ml tetrahydrofuran and is treated with conc. HCl (0.41 ml) at 25°C for 10 min. The mixture is diluted with 200 ml methylene chloride then washed with 0.1M $Na_2HPO_4$ (50 ml) The organic layer is separated, dried over $Na_2SO_4$ and evaporated *in vacuo* to give crude 5. Chromatographic purification of the crude product eluting with 30% ethylacetate/cyclohexane gives 0.68 g of 5.

IR ($CHCl_3$): 1750 and 1610 $cm^{-1}$.

Preparation of 7

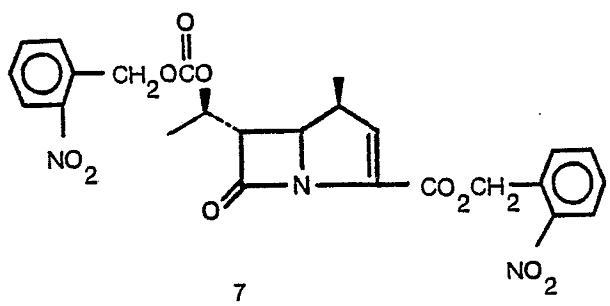

7

*Trans*-1 - (o - nitrobenzyloxycarbonyltriphenylphosphoranylmethyl) - 3 - [1(R) - o - nitrobenzyloxycarbonyloxyethyl]-4-[(1β-methyl-2-hydroxy)ethyl]-2-azetidinone (5) (70 mg) is treated with 1 ml DMSO and 1 ml acetic anhydride at r.t. overnight. After solvents evaporated *in vacuo* the residue is chromatographed on TLC plate (silica gel GF, 500 μ) eluting with 50% ethylacetate/cyclohexane to give 12 mg of 7,

IR ($CHCl_3$): 1770 $cm^{-1}$, and 1740 $cm^{-1}$;

NMR (300 MHZ, $CDCl_3$): 1.15 (d); 2.80 (d), 3.30 (m), 3.53 (q), 4.35 (q), 5.21 (quintet), 4.55 (d), 4.82 (d), 4.55 (d), 4.62 (d), 6.51 (d) 7.49 (q), 7.65 (m), 7.83 (d) and 8.12 (m) ppm.

Preparation of (I)

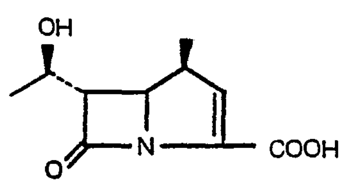

(I)

*Trans*-6 - [1(R) - *o* - nitrobenzyloxycarbonyloxyethyl] - 1β - methyl - 1 - carbadethiapen - 2 - em - 3-carboxylic acid o-nitrobenzyl ester 7 (4 mg) in 4 ml $H_2O$ and 4 ml dioxane is photolysized in a quartz tube under 3500Å in the presence of $NaHCO_3$ (5 mg) at 20°C for 2.5 hr. The mixture is extracted with ether. The aqueous layer is neutralized with HCl then concentrated to 1 ml. Chromatographic purification of the crude product by a XAD—2 resin (1.5 x 5 cm) eluting with water gives (I) (0.13 mg) as sodium salt. Electrophoresis shows single bioactive zone which moves 7.9 cm towards anode at 2KV, for 20 min in 0.05*M* pH 7.0 phosphate buffer.

Example 33

Following the foregoing text and Examples, the following species (I) are obtained by analogy.

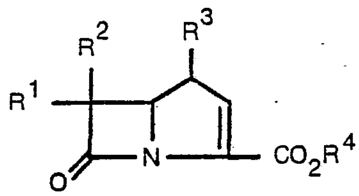

| Compound | $R^1$ | $R^2$ | $R^3$ | $R^4$ |
|---|---|---|---|---|
| 1.) | $HOCH_2$ | H | $-CH_3$ | $Na^+$ |
| 2.) | $-CH_3$ | H | $-CH_3$ | $Na^+$ |
| 3.) | 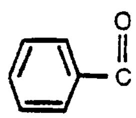 | H | $-CH_3$ | H |
| 4.) | $CH_3\overset{\overset{O}{\|}}{C}$ | H | $-CH_3$ | $K^+$ |
| 5.) | $(CH_3)_2C(OH)-$ | H | $-CH_3$ | $Na^+$ |
| 6.) | $CH_3CH(N_3)-$ | H | $CH_3$ | $Na^+$ |
| 7.) | $CH_3CH(OH)-$ | $-CH_3$ | $CH_3$ | $Na^+$ |
| 8.) | $HOCH_2-$ | $CH_3CH_2-$ | $CH_3$ | H |
| 9.) | $CH_3\overset{\overset{O}{\|}}{C}-$ | $CH_3$ | $CH_3$ | $Na^+$ |
| 10.) | $CH_3CH(OH)-$ | H | $CH_3CH_2$ | $Na^+$ |
| 11.) | $CH_3CH(OH)-$ | $CH_3$ | $CH_3CH_2$ | H |
| 12.) | $HOCH_2-$ | $CH_3$ | 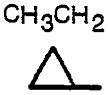 | $Na^+$ |
| 13.) | $CH_3CH(OH)CH_2-$ | H | $CH_3$ | $(C_2H_5)_4N^+$ |
| 14.) | $CH_3CH(OCH_2SCH_3)-$ | H | $CH_3$ | $Na^+$ |
| 15.) | 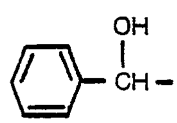 | H | $CH_3$ | $Na^+$ |
| 16.) | 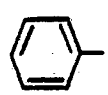 | H | $CH_3CH(CH_3)-$ | $Na^+$ |
| 17.) | 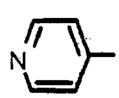 | H | CH | $Na^+$ |
| 18.) | 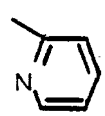 | H | $CH_3$ | $Na^+$ |
| 19.) | $CH_3CH(SH)-$ | H | $CH_3$ | $K^+$ |

Example 34

Preparation of Pharmaceutical Compositions

One such unit dosage form is prepared by mixing 120 mg of 1-methyl-6-(1-hydroxyethyl)-2-(2-aminoethylthio)-1-carbadethiapen-2-em-3-carboxylic acid with 20 mg of lactose and 5 mg of magnesium stearate and placing the 145 mg. mixture into a No. 3 gelatin capsule. Similarly, by employing more of the active ingredient and less lactose, other dosage forms can be put up in No. 3 gelatin capsules and should it be necessary to mix more than 145 mg. of ingredients together larger capsules such as compressed tablets and pills can also be prepared. The following examples are illustrative of the preparation of pharmaceutical formulations:

| TABLET | PER TABLET |
|---|---|
| 1-methyl-6-(1-hydroxyethyl)-2-(2-aminoethylthio)-1-carbadethiapen-2-em-3-carboxylic acid | 125 mg. |
| Dicalcium Phosphate | 192 mg. |
| Cornstarch, U.S.P. | 6 mg. |
| Lactose, U.S.P. | 190 mg. |
| Magnesium Stearate | Balance |

The active ingredient is blended with the dicalcium phosphate, lactose and about half of the cornstarch. The mixture is then granulated with 15% cornstarch paste (6 mg) and rough-screened. It is dried at 45°C and screened again through No. 16 screens. The balance of the cornstarch and the magnesium stearate is added and the mixture is compressed into tablets, approximately 0.5 inch in diameter each weighing 800 mg.

PARENTERAL SOLUTION

| | | |
|---|---|---|
| Ampoule: | | |
| 1-methyl-6-(1-hydroxyethyl)-2-(2-aminoethylthio)-1-carbadethiapen-2-em-3-carboxylic acid | | 500 mg. |
| Diluent: Sterile Water for Injection | | 5 cc |

OPHTHALMIC SOLUTION

| | | |
|---|---|---|
| 1-methyl-6-(1-hydroxyethyl)-2-(2-aminoethylthio)-1-carbadethiapen-2-em-3-carboxylic acid | | 100 mg. |
| Hydroxypropylmethyl cellulose | | 5 mg. |
| Sterile water | to | 1 ml. |

OTIC SOLUTION

| | | |
|---|---|---|
| 1-methyl-6-(1-hydroxyethyl)-2-(2-aminoethylthio)-1-carbadethiapen-2-em-3-carboxylic acid | | 100 mg. |
| Benzalkonium chloride | | 0.1 mg. |
| Sterile water | to | 1 ml. |

TOPICAL OINTMENT

| | |
|---|---|
| 1-methyl-6-(1-hydroxyethyl)-2-(2-aminoethylthio)-1-carbadethiapen-2-em-3-carboxylic acid | 100 mg. |
| Polyethylene Glycol 4000 U.S.P. | 400 mg. |
| Polyethylene Glycol 400 U.S.P. | 1.0 gram. |

The active ingredient in the above formulations may be administered alone or in combination with other biologically active ingredients as, for example, with other antibacterial agents such as lincomycin, a penicillin, streptomycin, novobiocin, gentamicin, neomycin, colistin and kanamycin, or with other therapeutic agents such as probenecid.

**Claims for the Contracting States: BE CH DE FR GB IT LU NL SE**

1. A compound having the structure:

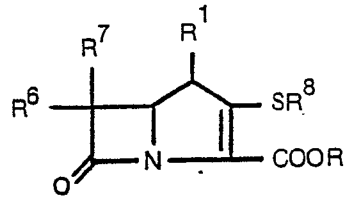

and the pharmaceutically acceptable salts thereof wherein R is H; pharmaceutically acceptable salt cation; or a pharmaceutically acceptable ester moiety; and $R^1$, $R^6$, $R^7$ and $R^8$ are independently selected from hydrogen, ($R^1$ is not H), substituted and unsubstituted: alkyl, alkenyl, and alkynyl, having from 1—10 carbon atoms; cycloalkyl, cycloalkylalkyl, and alkylcycloalkyl, having 3—6 carbon atoms in the cycloalkyl ring and 1—6 carbon atoms in the alkyl moieties, phenyl; aralkyl, aralkenyl, and aralkynyl wherein the aryl moiety is phenyl and the alkyl chain has 1—6 carbon atoms; heterocycyl and heterocyclylalkyl wherein the substituent or substituents relative to the above-named radicals are selected from amino, mono-, di-, and trialkylamino, hydroxyl, alkoxyl, mercapto, alkylthio, phenylthio, sulfamoyl, amidino, guanidino, nitro, chloro, bromo, fluoro, cyano and carboxy; and wherein the hetero atom or atoms in the above-named heterocyclic moieties are selected from 1—4 oxygen, nitrogen or sulphur atoms; and wherein the alkyl moieties of the above-recited substituents have 1—6 carbon atoms.

2. A compound according to Claim 1 wherein $R^1$ is alkyl having from 1—10 carbon atoms, or cycloalkyl having 3—6 carbon atoms.

3. A compound according to Claim 1 wherein $R^6$ is H or methyl and $R^7$ is alkyl having 1 to 10 carbon atoms, phenyl, aralkyl or hydroxyl-substituted alkyl, phenyl or aralkyl wherein the aryl moiety is phenyl and the alkyl chain has 1 to 6 carbon atoms.

4. A compound according to Claim 1 wherein: $R^6$ is hydrogen or methyl and $R^8$ is selected from:

H, $CH_3$, $(CH_2)_2NH_2$, $C(CH_3)_2CH_2NH_2$, 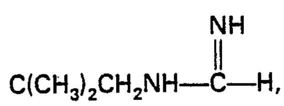, 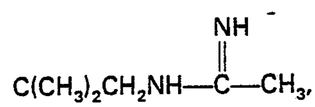,

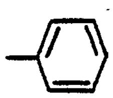, 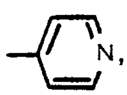, 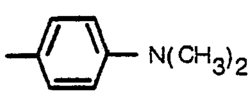 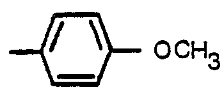 $CH_2CH_2CH_2NH_2$,

$CH_2CH(CH_3)NH_2$, 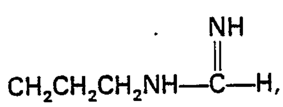, 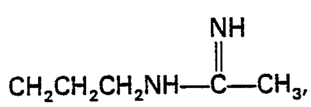, 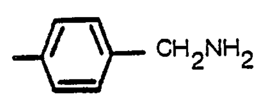

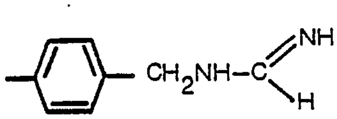 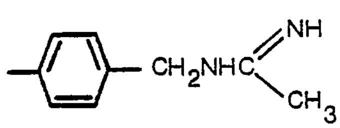 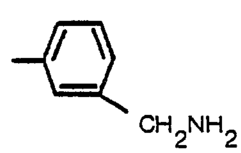

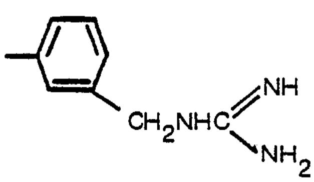 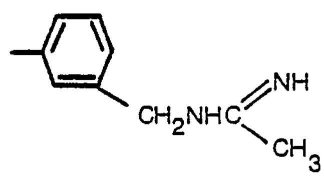 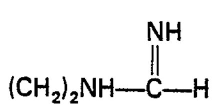 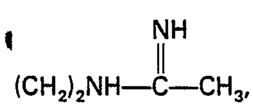,

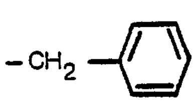 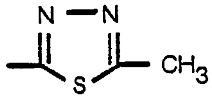 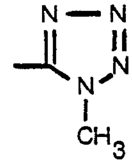 $CH(CH_3)CH_2NH_2$,

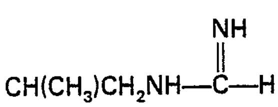 or 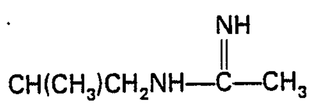

and $R^7$ is selected from:

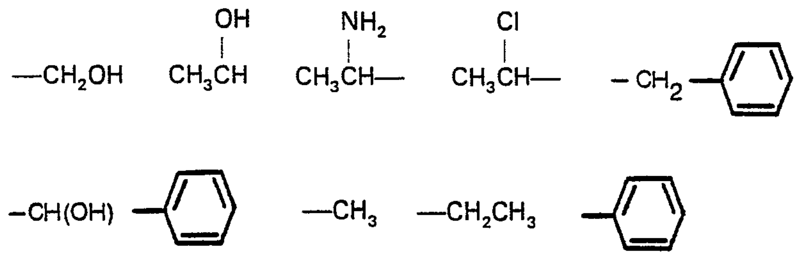

5. A process for preparing a compound according to Claim 1 comprising: cyclizing

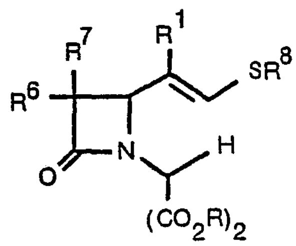

by halogenating and treating the intermediate obtained with a base to form:

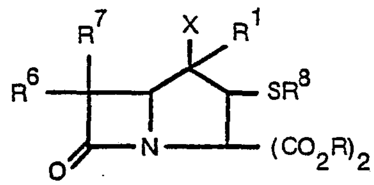

dehydrohalogenating the resulting intermediate in the presence of base to form:

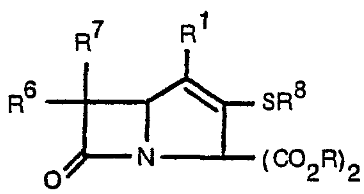

heating the resulting intermediate in the presence of a displacing agent to form:

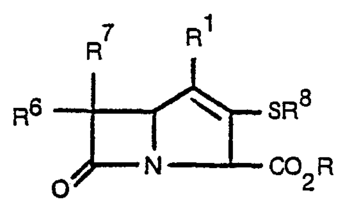

isomerizing the position of the double bond of the resulting intermediate by treating in a solvent with a strong base to form

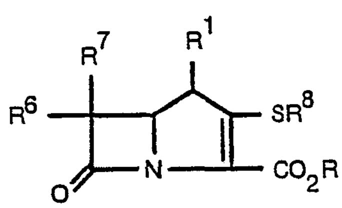

wherein X is halo.

6. A process for preparing a compound according to Claim 1 comprising decarboxylation of:

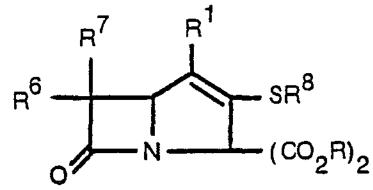

by treating with a displacing agent and further treating with a base capable of isomerizing the double bond to form:

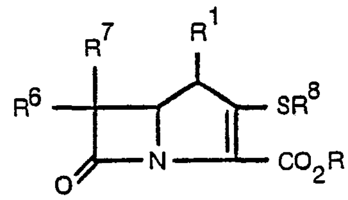

7. A compound according to Claim 1 having the structure:

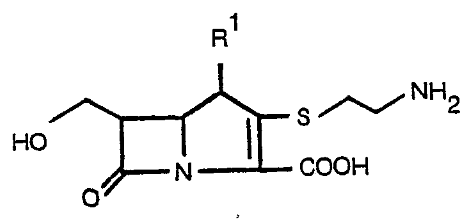

and its pharmaceutically acceptable salts and esters, wherein $R^1$ is defined as in Claim 1.

8. A compound according to Claim 1 having the structure:

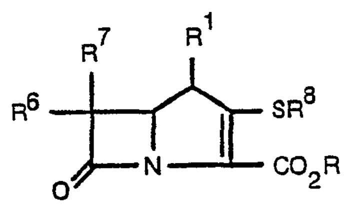

and the pharmaceutically acceptable salts thereof wherein R is H; pharmaceutically acceptable salt cation; or a pharmaceutically acceptable ester moiety; and $R^6$, $R^7$ and $R^8$ are independently selected from hydrogen, substituted and unsubstituted: alkyl, alkenyl, and alkynyl, having from 1—10 carbon cycloalkyl, cycloalkylalkyl, and alkylcycloalkyl, having 3—6 carbon atoms in the cycloalkyl ring and 1—6 carbon atoms in the alkyl moieties, phenyl; aralkyl, aralkenyl, and aralkynyl wherein the aryl moiety is phenyl and the alkyl chain has 1—6 carbon atoms; wherein the substituent or substituents relative to the above-named radicals are selected from amino, mono-, di-, and trialkylamino, hydroxyl, alkoxyl, mercapto, alkylthio, phenylthio, sulfamoyl, amidino, guanidino, nitro, chloro, bromo, fluoro, cyano and carboxy; and wherein the moieties of the above-recited substituents have 1—6 carbon atoms;

$R^1$ is selected from methyl, ethyl, propyl, isopropyl cyclopropyl, phenyl and benzyl.

9. A compound having the structure:

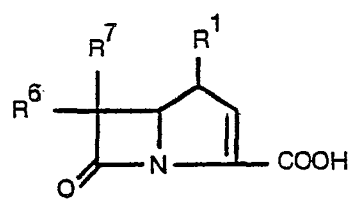

and the pharmaceutically acceptable salts and esters thereof: wherein

a) the substituents $R^1$, $R^6$ and $R^7$ are independently selected from hydrogen ($R^1$ is not H); substituted and unsubstituted: straight and branched loweralkyl having from 1 to 10 carbon atoms; cycloalkyl having from 3 to 6 carbon atoms; cycloalkylalkyl wherein the cycloalkyl moiety comprises 3

to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; phenyl and naphthyl; phenylalkyl; heterocyclyl (saturated and unsaturated) comprising mono- and bicyclic structures having from 5 to 10 ring atoms wherein one or more of the hetero atoms is selected from oxygen, nitrogen or sulphur, heterocyclylalkyl which comprises the immediately preceding heterocyclyl moieties and the alkyl moiety comprises from 1 to 10 carbon atoms; the substituent (or substituents) relative to the above-named radicals is selected from amino, hydroxyl, cyano, carboxyl, nitro, chloro, bromo, fluoro, lower alkoxy having from 1 to 6 carbon atoms, mercapto, perhaloloweralkyl, loweralkylthio, guanidino, amidino, sulfamoyl, and N-substituted: sulfamoyl, amidino and guanidino wherein the N-substituent is loweralkyl having from 1 to 6 carbon atoms or aryl having 6—10 carbon atoms or

b) the substituents $R^1$, $R^6$ and $R^7$ are independently selected from hydrogen ($R^1$ is not hydrogen), substituted and unsubstituted: alkyl, alkenyl, and alkynyl, having from 1—10 carbon atoms; cycloalkyl, cycloalkylalkyl, and alkylcycloalkyl, having 3—6 carbon atoms in the cycloalkyl ring and 1—6 carbon atoms in the alkyl moieties; phenyl; aralkyl, aralkenyl, and aralkynyl wherein the aryl moiety is phenyl and the linear chain has 1—6 carbon atoms; heterocyclyl and heterocyclylalkyl; wherein the substituent or substituents relative to the above-named radicals are selected from: amino, mono, di- and trialkylamino, hydroxyl, alkoxyl, mercapto, alkylthio, phenylthio, sulfamoyl, amidino, guanidino, nitro, chloro, bromo, fluoro, cyano and carboxy; and wherein the hetero atom or atoms in the above-named heterocyclic moieties are selected from 1—4 oxygen, nitrogen or sulphur atoms; and wherein the alkyl moieties of the above-recited substituents have 1—6 carbon atoms.

10. A compound according to Claim 9 wherein $R^1$ is alkyl having from 1—6 carbon atoms, cyclopropyl, benzyl or phenyl; $R^6$ is hydrogen and $R^7$ is alkyl having from 1—6 carbon atoms, or phenylalkyl wherein the alkyl moiety has 1—6 carbon atoms, substituted by hydroxyl or amino.

11. A compound according to Claim 10 wherein $R^1$ is methyl, ethyl, isopropyl, t-butyl or phenyl and $R^7$ is 1-hydroxyethyl, methyl, or hydroxymethyl.

12. A process for preparing a compound according to Claim 9 comprising treating a compound of the formula:

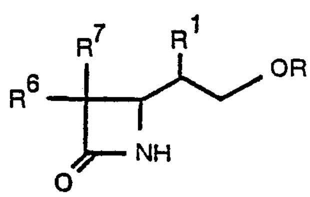

wherein R is a readily removable protecting group with a glyoxylate ester wherein the ester moiety is a pharmaceutically acceptable ester moiety or a readily removable blocking group, R′, to provide a compound having the structure:

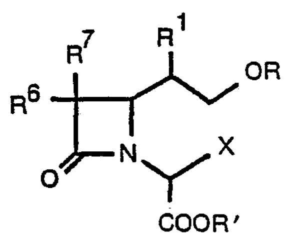

followed by halogenating to provide a compound having the structure:

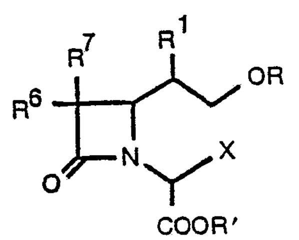

wherein X is halogen followed by treating with triphenylphosphine and mild aqueous hydrolysis to provide:

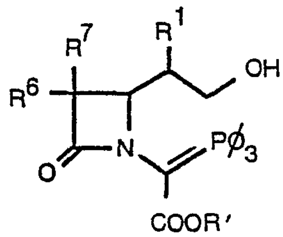

followed by cyclizing the above structure after treating with an oxidizing agent; followed by removing the blocking group.

13. A process for preparing a compound according to Claim 9 comprising cyclizing:

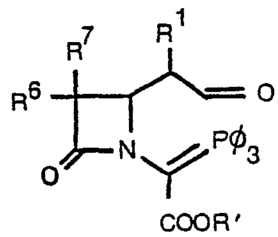

wherein Ø is phenyl, and R′ is a pharmaceutically acceptable ester moiety or a readily removable blocking group; followed by removing the blocking group.

14. A compound according to Claim 9 having the formula:

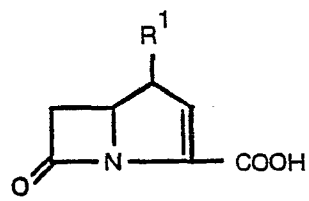

and its pharmaceutically acceptable salts and esters wherein $R^1$ is selected from substituted and unsubstituted loweralkyl having from 1—6 carbon atoms, phenyl, phenylloweralkyl, cycloalkyl having from 3 to 6 carbon atoms, and cycloalkylalkyl having 4 to 7 carbon atoms in the chain and 3—6 carbon atoms in the ring; wherein said substituents on $R^1$ are selected from chloro, bromo, fluoro, hydroxyl, amino, alkoxyl, cyano, carboxyl, mono-, di- and trialkylamino (each alkyl having 1—6 carbon atoms).

15. A compound according to Claim 14 wherein $R^1$ is methyl, phenyl, ethyl, cyclopropyl, propyl and isopropyl.

16. A process for preparing a compound according to Claim 14 comprising treating a compound of the formula:

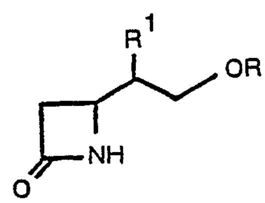

wherein R is a readily removable protecting group with a glyoxylate ester wherein the ester moiety is a pharmaceutically acceptable ester moiety or a readily removable blocking group, $R^2$, to provide a compound having the structure:

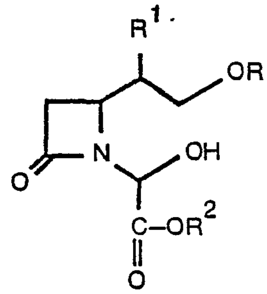

followed by halogenating to provide a compound having the structure;

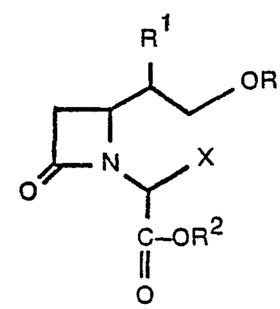

wherein X is halogen followed by treating with triphenylphosphine and mild aqueous hydrolysis to provide:

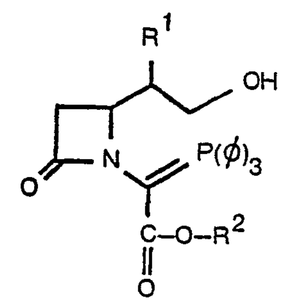

followed by cyclizing the above structure after treating with an oxidizing agent; followed by removing the blocking group.

17. A process for preparing a compound according to Claim 14 comprising cyclizing:

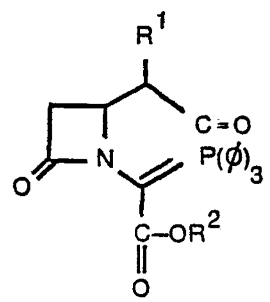

wherein Ø is phenyl, and $R^2$ is a pharmaceutically acceptable ester moiety or a readily removable blocking group; followed by removing the blocking group.

18. A compound according to Claim 1 having the formula:

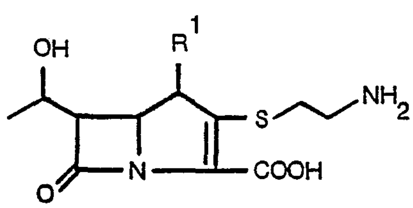

and its pharmceutically acceptable salts and esters wherein $R^1$ is selected from substituted and unsubstituted loweralkyl having from 1—6 carbon atoms, phenyl, phenylloweralkyl, cycloalkyl having from 3 to 6 carbon atoms, and cycloalkylalkyl having 4 to 7 carbon atoms in the chain and 3—6 carbon atoms in the ring; wherein said substituents on $R^1$ are selected from chloro, bromo, fluoro, hydroxyl, amino, alkoxyl having 1—6 carbon atoms, cyano, and carboxyl.

19. A compound according to Claim 18 wherein $R^1$ is methyl, phenyl, benzyl, ethyl, cyclopropyl, propyl and isopropyl.

20. A compound according to Claim 19 wherein $R^1$ is methyl having the structure

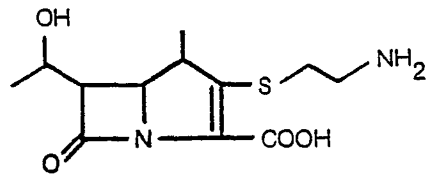

21. A compound according to Claim 1 having the formula:

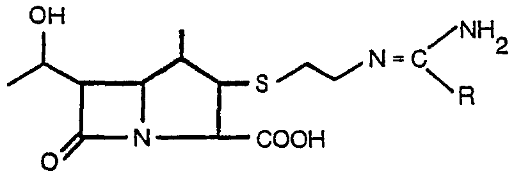

wherein R is H or $CH_3$.

22. A process for preparing a compound according to Claim 1 having the structure:

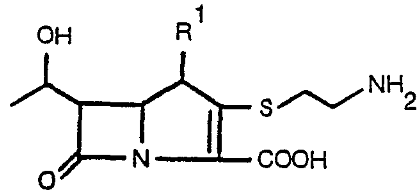

and its pharmaceutically acceptable salts: comprising: cyclizing

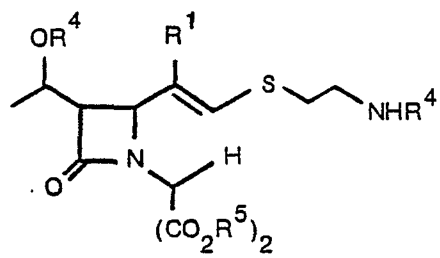

by halogenating and treating the intermediate obtained with a base to form:

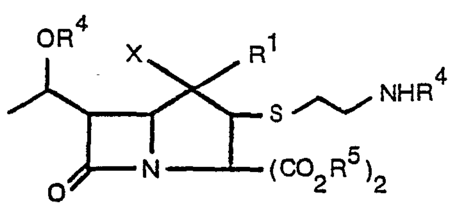

dehydrohalogenating the resulting intermediate in the presence of base to form:

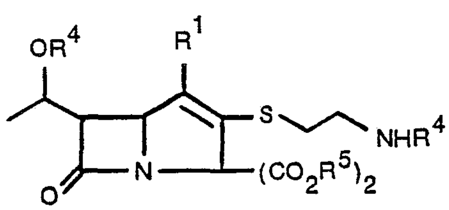

heating the resulting intermediate in the presence of a displacing agent to form:

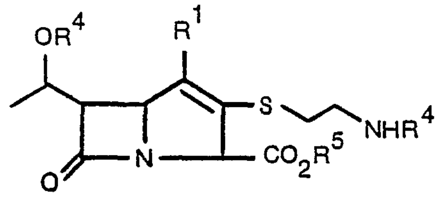

isomerizing the position of the double bond of the resulting intermediate by treating in a solvent with a strong base to form:

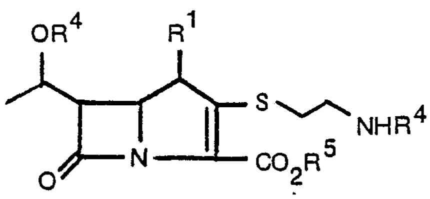 deblocking to form: 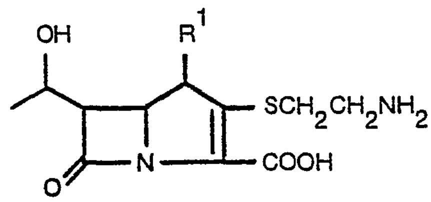

wherein $R^4$ and $R^5$ are blocking groups, and X is halo.

23. A process for preparing a compound according to Claim 18 having the structure:

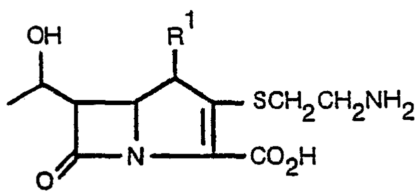 comprising treating: 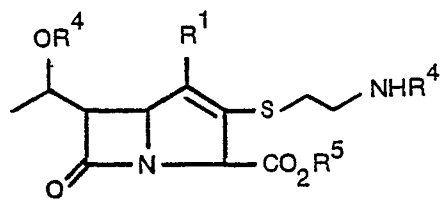

with a base capable of isomerizing the double bond to form:

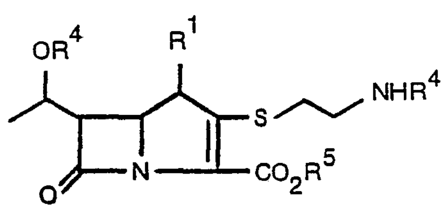

wherein $R^4$ and $R^5$ are blocking groups; followed by removing the blocking group.

24. An antibiotic pharmaceutical composition comprising a therapeutically effective amount of a compound according to Claims 1 to 4, 7 to 11, 14, 15 or 18 to 21 and a pharmaceutically carrier therefor.

25. Compounds according to the formula given in Claim 1 wherein $R^7$ is hydrogen, $R^1$ is selected from substituted and unsubstituted loweralkyl having from 1 to 6 carbon atoms, cyclopropyl, benzyl and phenyl; and $R^6$ is an $\alpha$-substituted alkyl wherein the $\alpha$-substituent is hydroxyl, amino or mercapto and wherein the alkyl moiety is straight or branched and comprises 1 to 6 carbon atoms; the substituent relative to the above radicals for $R^1$ is phenyl.

26. Compounds according to Claims 1, 2, 3, 4 and 8 wherein the pharmaceutically acceptable ester moieties R are pivaloyloxymethyl, allyl, methallyl, (2-methylthio)-ethyl, 3-methyl-2-butenyl, p-t-butylbenzyl, 5-indanyl, 3-phthalidyl.

**Claims for the Contracting State: AT**

1. A process for preparing a compound having the structure:

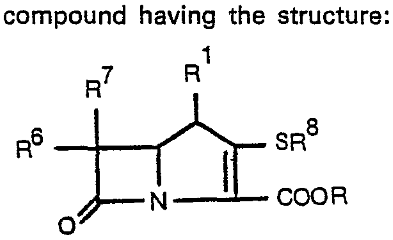

and the pharmaceutically acceptable salts thereof wherein R is H; pharmaceutically acceptable salt cation; or a pharmaceutically acceptable ester moiety; and $R^1$, $R^6$, $R^7$ and $R^8$ are independently selected from hydrogen, ($R^1$ is not H), substituted and unsubstituted: alkyl, alkenyl, and alkynyl, having from 1—10 carbon atoms; cycloalkyl, cycloalkylalkyl, and alkylcycloalkyl, having 3—6 carbon atoms in the cycloalkyl ring and 1—6 carbon atoms in the alkyl moieties; phenyl; aralkyl, aralkenyl, and aralkynyl wherein the aryl moiety is phenyl and the alkyl chain has 1—6 carbon atoms; heterocycyl and heterocyclylalkyl wherein the substituent or substituents relative to the above-named radicals are selected from amino, mono-, di-, and trialkylamino, hydroxyl, alkoxyl, mercapto, alkylthio, phenylthio, sulfamoyl, amidino, guanidino, nitro, chloro, bromo, fluoro, cyano and carboxy; and wherein the hetero atom or atoms in the above-named heterocyclic moieties are selected from 1—4 oxygen, nitrogen or sulphur atoms; and wherein the alkyl moieties of the above-recited substituents have 1—6 carbon atoms comprising: cyclizing:

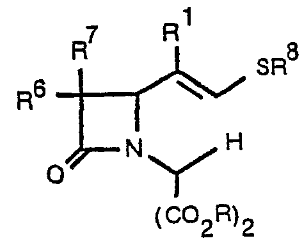

by halogenating and treating the intermediate obtained with a base to form:

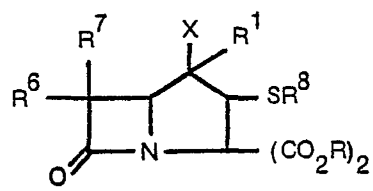

dehydrohalogenating the resulting intermediate in the presence of base to form:

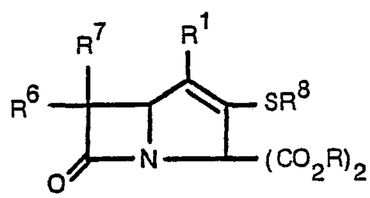

heating the resulting intermediate in the presence of a displacing agent to form:

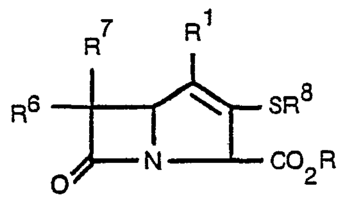

isomerizing the position of the double bond of the resulting intermediate by treating in a solvent with a strong base to form

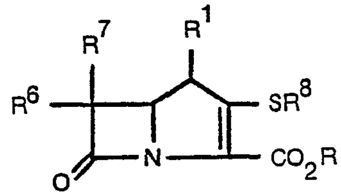

wherein X is halo.

2. A process for preparing a compound according to Claim 1 comprising decarboxylation of:

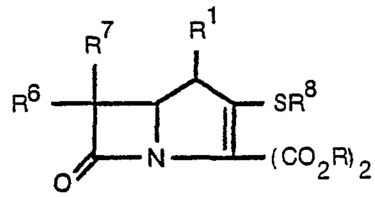

by treating with a displacing agent and further treating with a base capable of isomerizing the double bond to form:

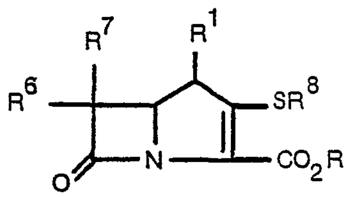

3. A process for preparing a compound having the structure:

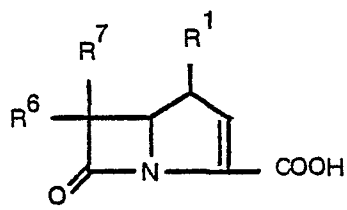

and the pharmaceutically acceptable salts and esters thereof; wherein

a) the substituents $R^1$, $R^6$ and $R^7$ are independently selected from hydrogen ($R^1$ is not H); substituted and unsubstituted: straight and branched loweralkyl having from 1 to 10 carbon atoms; cycloalkyl having from 3 to 6 carbon atoms; cycloalkylalkyl wherein the cycloalkyl moiety comprises 3 to 6 carbon atoms and the alkyl moiety comprises 1 to 10 carbon atoms; alkylcycloalkyl wherein the alkyl moiety comprises 1 to 6 carbon atoms and the cycloalkyl moiety comprises 3 to 6 carbon atoms; phenyl and naphthyl; phenylalkyl; heterocyclyl (saturated and unsaturated) comprising mono- and bicyclic structures having from 5 to 10 ring atoms wherein one or more of the hetero atoms is selected from oxygen, nitrogen or sulphur, heterocyclylalkyl which comprises the immediately preceding heterocyclyl moieties and the alkyl moiety comprises from 1 to 10 carbon atoms; the substituent (or substituents) relative to the above-named radicals is selected from amino, hydroxyl, cyano, carboxyl, nitro, chloro, bromo, fluoro, lower alkoxy having from 1 to 6 carbon atoms, mercapto, perhaloloweralkyl, loweralkylthio, guanidino, amidino, sulfamoyl, and N-substituted: sulfamoyl, amidino and guanidino wherein the N-substituent is loweralkyl having from 1 to 6 carbon atoms or aryl having 6—10 carbon atoms or

b) the substituents $R^1$, $R^6$ and $R^7$ are independently selected from hydrogen ($R^1$ is not hydrogen), substituted and unsubstituted: alkyl, alkenyl, and alkynyl, having from 1—10 carbon atoms; cycloalkyl, cycloalkylalkyl, and alkylcycloalkyl, having 3—6 carbon atoms in the cycloalkyl ring and 1—6 carbon atoms in the alkyl moieties; phenyl, aralkyl, aralkenyl, and aralkynyl wherein the aryl moiety is phenyl and the linear chain has 1—6 carbon atoms; heterocyclyl and heterocyclylalkyl; wherein the substituent or substituents relative to the above-named radicals are selected from: amino, mono, di- and trialkylamino, hydroxyl, alkoxyl, mercapto, alkylthio, phenylthio, sulfamoyl, amidino, guanidino, nitro, chloro, bromo, fluoro, cyano and carboxy; and wherein the hetero atom or atoms in the above-named heterocyclic moieties are selected from 1—4 oxygen, nitrogen or sulphur atoms; and wherein the alkyl moieties of the above-recited substituents have 1—6 carbon atoms, comprising treating a compound of the formula

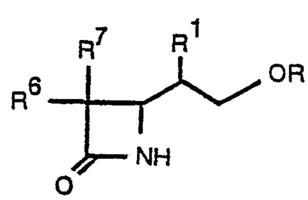

wherein R is a readily removable protecting group with a glyoxylate ester wherein the ester moiety is a

pharmaceutically acceptable ester moiety or a readily removable blocking group, R′, to provide a compound having the structure:

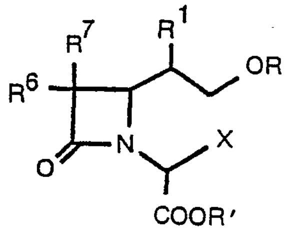

followed by halogenating to provide a compound having the structure:

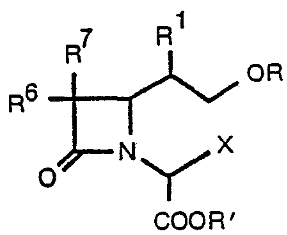

wherein X is halogen followed by treating with triphenylphosphine and mild aqueous hydrolysis to provide:

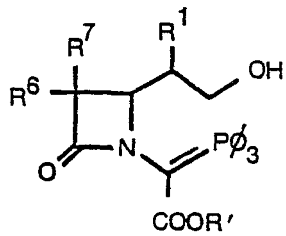

followed by cyclizing the above structure after treating with an oxidizing agent; followed by removing the blocking group.

4. A process for preparing a compound according to Claim 3 comprising cyclizing:

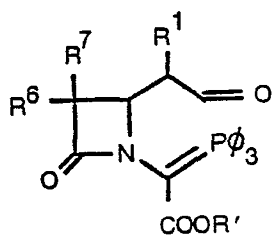

wherein Ø is phenyl, and R′ is a pharmaceutically acceptable ester moiety or a readily removable blocking group; followed by removing the blocking group.

5. A process for preparing a compound having the formula:

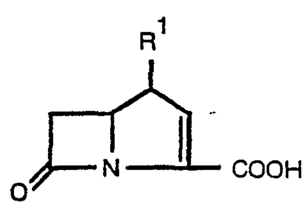

and its pharmaceutically acceptable salts and esters wherein $R^1$ is selected from the group consisting of substituted and unsubstituted loweralkyl having from 1—6 carbon atoms, phenyl, phenylloweralkyl, cycloalkyl having from 3 to 6 carbon atoms, and cycloalkylalkyl having 4 to 7 carbon atoms in the chain and 3—6 carbon atoms in the ring; wherein said substituents on $R^1$ are selected from chloro,

bromo, fluoro, hydroxyl, amino, mono-, di- and trialkylamino (each alkyl having 1 to 6 carbon atoms), alkoxyl, cyano, carboxyl comprising treating a compound of the formula:

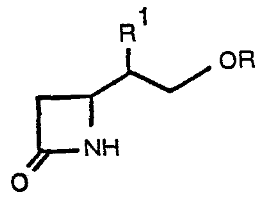

wherein R is a readily removable protecting group with a glyoxylate ester wherein the ester moiety is a pharmaceutically acceptable ester moiety or a readily removable blocking group, $R^2$, to provide a compound having the structure:

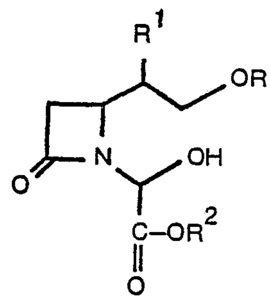

followed by halogenating to provide a compound having the structure;

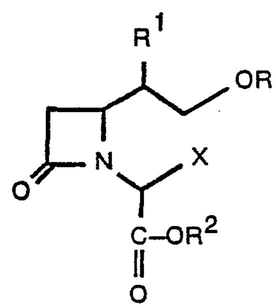

wherein X is halogen followed by treating with triphenylphosphine and mild aqueous hydrolysis to provide:

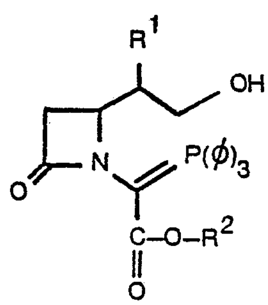

followed by cyclizing the above structure after treating with an oxidizing agent; followed by removing the blocking group.

6. A process for preparing a compound according to Claim 5 comprising cyclizing:

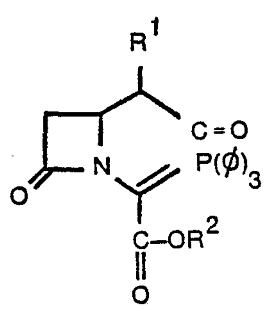

wherein Ø is phenyl, and $R^2$ is a pharmaceutically acceptable ester moiety or a readily removable blocking group; followed by removing the blocking group.

7. A process for preparing a compound according to Claim 1 having the structure:

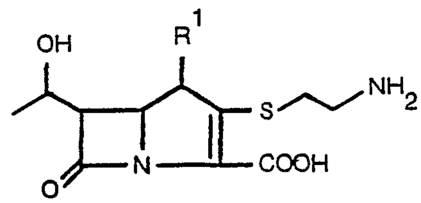

and its pharmaceutically acceptable salts; comprising: cyclizing

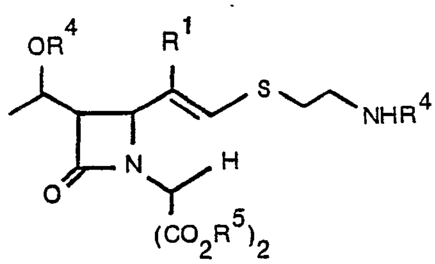

by halogenating and treating the intermediate obtained with a base to form:

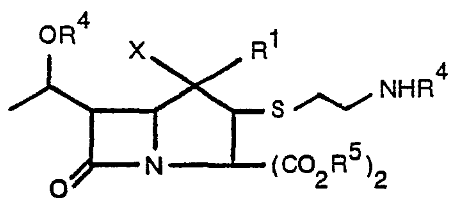

dehydrohalogenating the resulting intermediate in the presence of base to form:

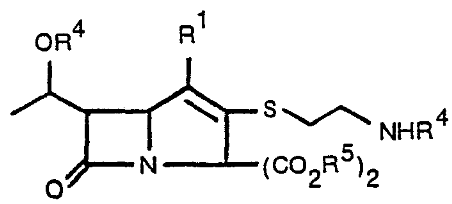

heating the resulting intermediate in the presence of a displacing agent to form:

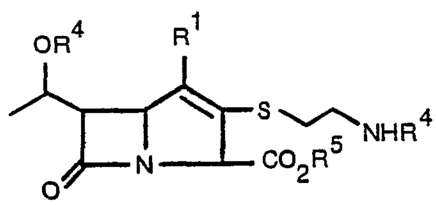

isomerizing the position of the double bond of the resulting intermediate by treating in a solvent with a strong base to form:

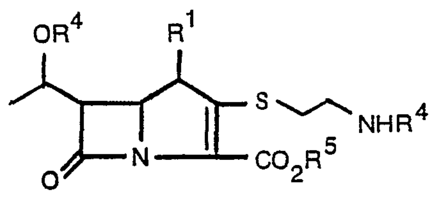 deblocking to form: 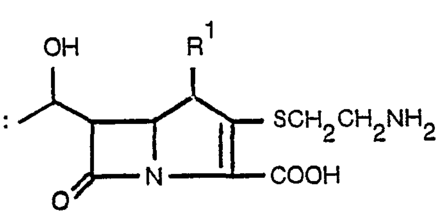

wherein $R^4$ and $R^5$ are blocking groups, and X is halo.

8. A process according to claim 7 for preparing a compound according to claim 1 having the formula:

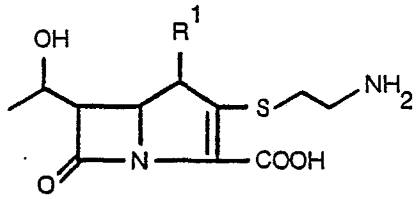

and its pharmaceutically acceptable salts wherein $R^1$ is selected from substituted and unsubstituted loweralkyl having from 1—6 carbon atoms, phenyl, phenylloweralkyl, cycloalkyl having from 3 to 6 carbon atoms, and cycloalkylalkyl having 4 to 7 carbon atoms in the chain and 3—6 carbon atoms in the ring; wherein said substituents on $R^1$ are selected from chloro, bromo, fluoro, hydroxyl, amino, alkoxyl having 1—6 carbon atoms, cyano, and carboxyl.

9. A process for preparing a compound having the formula:

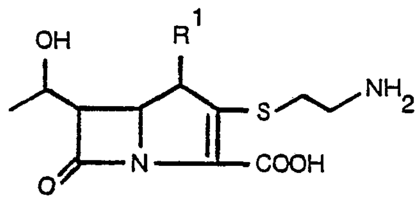

wherein $R^1$ is selected from substituted and unsubstituted loweralkyl having from 1—6 carbon atoms, phenyl, phenylloweralkyl, cycloalkyl having from 3 to 6 carbon atoms, and cycloalkylalkyl having 4 to 7 carbon atoms in the chain and 3—6 carbon atoms in the ring; wherein said substituents on $R^1$ are selected from chloro, bromo, fluoro, hydroxyl, amino, alkoxyl having 1—6 carbon atoms, cyano, and carboxyl comprising treating:

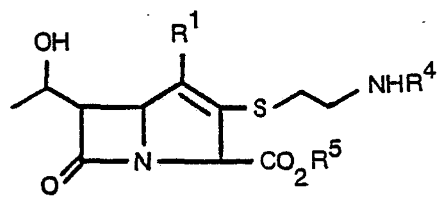

with a base capable of isomerizing the double bond to form:

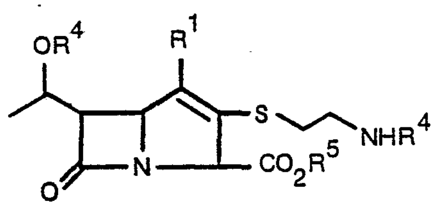

wherein $R^4$ and $R^5$ are blocking groups; followed by deblocking.

10. A process for preparing a compound according to the formula given in Claims 1 and 3 wherein $R^7$ is hydrogen, $R^1$ is selected from substituted and unsubstituted: loweralkyl having from 1 to 6 carbon atoms, cyclopropyl, benzyl and phenyl; and $R^6$ is an $\alpha$-substituted alkyl wherein the $\alpha$-substituent is hydroxyl, amino or mercapto and wherein the alkyl moiety is straight or branched and comprises 1 to 6 carbon atoms; the substituent relative to the above radicals for $R^1$ is phenyl, characterized in that it is carried out as described in Claims 1, 2 or 3, wherein $R^1$, $R^6$ and $R^7$ in the starting compounds are defined as above.

11. A process for preparing a compound according to Claims 1 and 2 wherein the pharmaceutically acceptable ester moieties R and pivaloyloxymethyl, allyl, methallyl, (2-methylthio)-ethyl, 3-methyl-2-butenyl, p-t-butylbenzyl, 5-indanyl, 3-phthalidyl.

**Patentansprüche für die Vertragsstaaten: BE CH DE FR GB IT LU NL SE**

1. Eine Verbindung mit der Struktur:

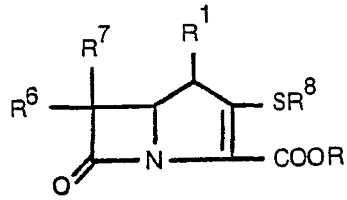

und die pharmazeutisch annehmbaren Salze derselben, worin R H; pharmazeutisch annehmbares Salzkation; oder ein pharmazeutisch annehmbarer Esterrest ist; und $R^1$, $R^6$, $R^7$ und $R^8$ unabhängig voneinander ausgewählt sind aus Wasserstoff ($R^1$ ist nicht Wasserstoff), substituiertem und unsubstituiertem: Alkyl, Alkenyl und Alkinyl mit 1 bis 10 Kohlenstoffatomen; Cycloalkyl, Cycloalkylalkyl und Alkylcycloalkyl mit 3 bis 6 Kohlenstoffatomen im Cycloalkylring und 1 bis 6 Kohlenstoffatomen in den Alkylresten; Phenyl; Aralkyl, Aralkenyl und Aralkinyl, worin der Arylrest Phenyl ist und die Alkylkette 1 bis 6 Kohlenstoffatome hat; Heterocyclyl und Heterocyclylalkyl, worin der Substituent oder die Substituenten in bezug auf die oben genannten Reste ausgewählt sind aus: Amino, Mono-, Di- und Trialkylamino, Hydroxyl, Alkoxyl, Mercapto, Alkylthio, Phenylthio, Sulfamoyl, Amidino, Guanidino, Nitro, Chlor, Brom, Fluor, Cyano und Carboxy; und worin das Heteroatom oder die Heteroatome in den oben genannten heterocyclischen Resten ausgewahlt sind aus 1—4 Sauerstoff-, Stickstoff- oder Schwefelatomen; und worin die Alkylreste der oben genannten Substituenten 1 bis 6 Kohlenstoffatome haben.

2. Eine Verbindung nach Anspruch 1, worin $R^1$ Alkyl mit 1—10 Kohlenstoffatomen oder Cycloalkyl mit 3—6 Kohlenstoffatomen ist.

3. Eine Verbindung nach Anspruch 1, worin $R^6$ H oder Methyl ist und $R^7$ Alkyl mit 1—10 Kohlenstoffatomen, Phenyl, Aralkyl oder hydroxylsubstituiertes Alkyl, Phenyl oder Aralkyl ist, worin der Arylrest Phenyl ist und die Alkylkette 1—6 Kohlenstoffatome hat.

4. Eine Verbindung nach Anspruch 1, worin $R^6$ Wasserstoff oder Methyl ist und $R^8$ aus:

H, $CH_3$, $(CH_2)_2NH_2$, $C(CH_3)_2CH_2NH_2$, 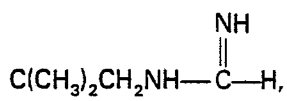, 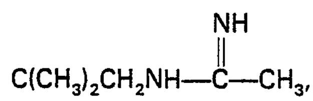,

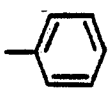, 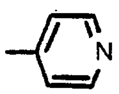, 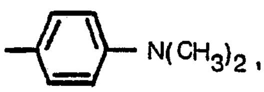, 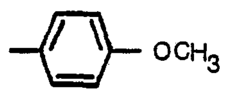, $CH_2CH_2CH_2NH_2$,

$CH_2CH(CH_3)NH_2$, 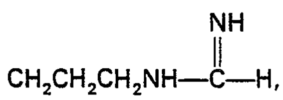, 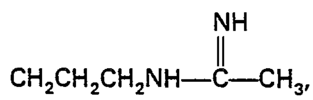, 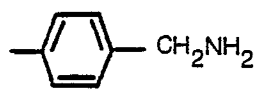,

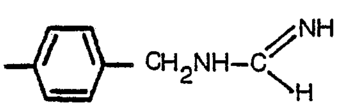, 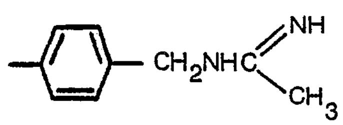, 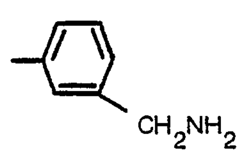,

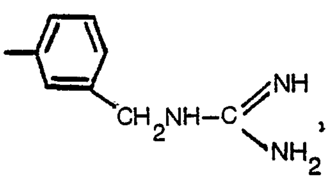, 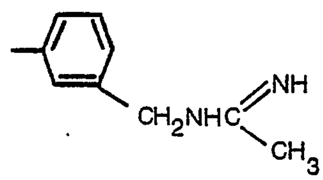, 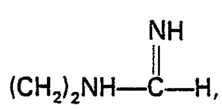, 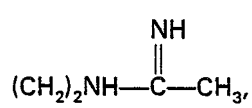,

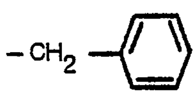, 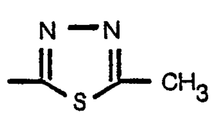, 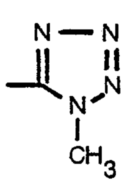, $CH(CH_3)CH_2NH_2$,

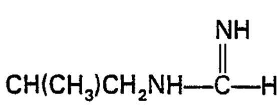 oder 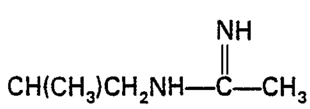

ausgewählt ist und $R^7$ aus: 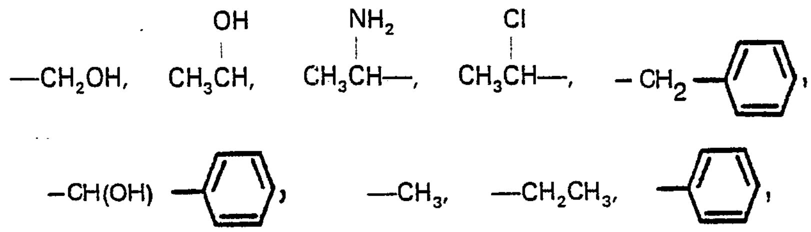

ausgewählt ist.

5. Ein Verfahren zur Herstellung einer Verbindung nach Anspruch 1, umfassend: die Cyclisierung von

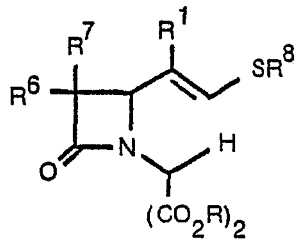

durch Halogenierung und Behandlung des erhaltenen Zwischen-produktes mit einer Base unter Bildung von

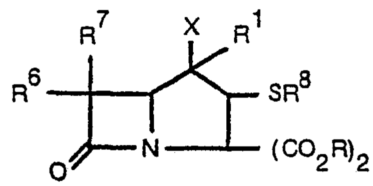

die Dehydrohalogenierung des entstehenden Zwischenproduktes in Gegenwart von Base unter Bildung von

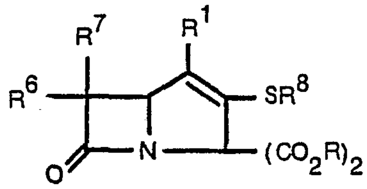

das Erhitzen des entstehenden Zwischenproduktes in Gegenwart eines Verdrängungsmittels unter Bildung von

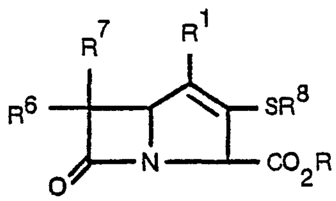

die Isomerisierung der Stellung der Doppelbindung des entstehenden Zwischenproduktes durch Behandlung in einem Lösungsmittel mit einer starken Base unter Bildung von

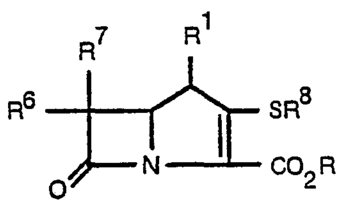

worin X Halogen ist.

6. Ein Verfahren zur Herstellung einer Verbindung nach Anspruch 1, umfassend die Decarboxylierung von

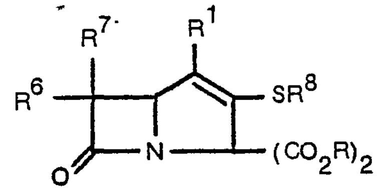

durch Behandlung mit einem Verdrängungsmittel und die weitere Behandlung mit einer zur Isomerisierung der Doppelbindung befähigten Base unter Bildung von

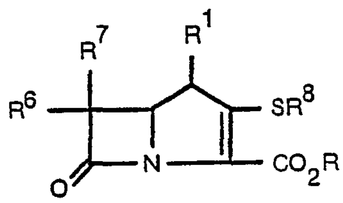

7. Eine Verbindung nach Anspruch 1 mit der Struktur:

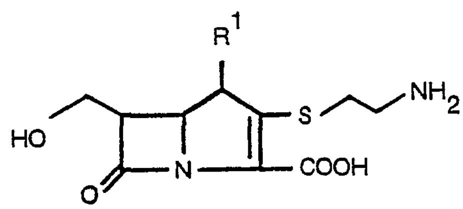

und deren pharmazeutisch annehmbare Salz und Ester, worin $R^1$ wie in Anspruch 1 definiert ist.

8. Eine Verbindung nach Anspruch 1 mit der Struktur:

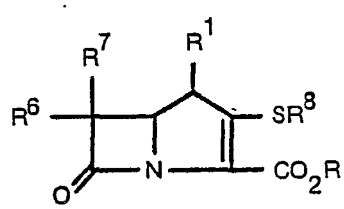

und die pharmazeutisch annehmbaren Salze derselben, worin R H; pharmazeutisch annehmbares Salzkation; oder ein pharmazeutisch annehmbarer Esterrest ist; und $R^6$, $R^7$ und $R^8$ unabhängig voneinander ausgewählt sind aus Wasserstoff, substituiertem und unsubstituiertem: Alkyl, Alkenyl und Alkinyl mit 1 bis 10 Kohlenstoffatomen; Cycloalkyl, Cycloalkylalkyl und Alkylcycloalkyl mit 3 bis 6 Kohlenstoffatomen im Cycloalkylring und 1 bis 6 Kohlenstoffatomen in den Alkylresten; Phenyl; Aralkyl, Aralkenyl und Aralkinyl, worin der Arylrest Phenyl ist und die lineare Kette 1 bis 6 Kohlenstoffatome hat; worin der Substituent oder die Substituenten in bezug auf die oben genannten Reste ausgewählt sind aus: Amino, Mono-, Di- und Trialkylamino, Hydroxyl, Alkoxyl, Mercapto, Alkylthio, Phenylthio, Sulfamoyl, Amidino, Guanidino, Nitro, Chlor, Brom, Fluor, Cyano und Carboxy; und worin die Alkylreste der oben genannten Substituenten 1 bis 6 Kohlenstoffatome haben; wobei $R^1$ aus Methyl, Ethyl, Propyl, Isopropyl, Cyclopropyl, Phenyl und Benzyl ausgewählt ist.

9. Eine Verbindung mit der Struktur:

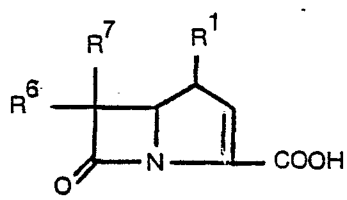

und die pharmazeutisch annehmbaren Salze und Ester derselben; worin

a) die Substituenten $R^1$, $R^6$ und $R^7$ unabhängig voneinander ausgewählt sind aus Wasserstoff ($R^1$ ist nicht H); substituiertem und unsubstituiertem: geradkettigem und verzweigtem Niedrigalkyl mit 1

bis 10 Kohlenstoffatomen; Cycloalkyl mit 3 bis 6 Kohlenstoffatomen; Cycloalkylalkyl, worin der Cycloalkylrest 3 bis 6 Kohlenstoffatome und der Alkylrest 1 bis 10 Kohlenstoffatome umfaßt; Alkylcycloalkyl, worin der Alkylrest 1 bis 6 Kohlenstoffatome und der Cycloalkylrest 3 bis 6 Kohlenstoffatome umfaßt; Phenyl und Naphthyl; Phenylalkyl; Heterocyclyl (gesättigt und ungesättigt), umfassend mono- und bicyclische Strukturen mit 5 bis 10 Ringatomen, worin eines oder mehrere der Heteroatome aus Sauerstoff, Stickstoff oder Schwefel gewählt sind; Heterocyclylalkyl, welches die unmittelbar vorhergehenden Heterocyclylreste und die 1 bis 10 Kohlenstoffatome enthaltenden Alkylreste umfaßt; wobei der Substituent (oder die Substituenten) in bezug auf die oben genannten Reste ausgewählt ist (sind) aus Amino, Hydroxyl, Cyano, Carboxyl, Nitro, Chlor, Brom, Fluor, Niedrigalkoxy mit 1 bis 6 Kohlenstoffatomen, Mercapto, Perhalogenniedrigalkyl, Niedrigalkylthio, Guanidino, Amidino, Sulfamoyl, und N-substituiertem: Sulfamoyl, Amidino und Guanidino, worin der N-Substituent Niedrigalkyl mit 1 bis 6 Kohlenstoffatomen oder Aryl mit 6 bis 10 Kohlenstoffatomen ist, oder

b) die Substituenten $R^1$, $R^6$ und $R^7$ unabhängig voneinander ausgewählt sind aus Wasserstoff ($R^1$ ist nicht Wasserstoff), substituiertem und unsubstituiertem: Alkyl, Alkenyl und Alkinyl mit 1 bis 10 Kohlenstoffatomen; Cycloalkyl, Cycloalkylalkyl und Alkylcycloalkyl mit 3 bis 6 Kohlenstoffatomen im Cycloalkylring und 1 bis 6 Kohlenstoffatomen in den Alkylresten; Phenyl; Aralkyl, Aralkenyl und Aralkinyl, worin der Arylrest Phenyl ist und die lineare Kette 1 bis 6 Kohlenstoffatome hat; Heterocyclyl und Heterocyclylalkyl; worin der Substituent oder die Substituenten in bezug auf die oben genannten Reste ausgewählt sind aus: Amino, Mono-, Di- und Trialkylamino, Hydroxyl, Alkoxyl, Mercapto, Alkylthio, Phenylthio, Sulfamoyl, Amidino, Guanidino, Nitro, Chlor, Brom, Fluor, Cyano und Carboxy; und worin das Heteroatom oder die Heteroatome in den oben gennanten heterocyclischen Resten ausgewählt sind aus 1—4 Sauerstoff-, Stickstoff- oder Schwefelatomen; und worin die Alkylreste der oben genannten Substituenten 1 bis 6 Kohlenstoffatome haben.

10. Eine Verbindung nach Anspruch 9, worin $R^1$ Alkyl mit 1 bis 6 Kohlenstoffatomen, Cyclopropyl, Benzyl oder Phenyl ist; $R^6$ Wasserstoff ist und $R^7$ Alkyl mit 1 bis 6 Kohlenstoffatomen, oder Phenylalkyl, worin der Alkylrest 1 bis 6 Kohlenstoffatome hat, substituiert durch Hydroxyl oder Amino ist.

11. Eine Verbindung nach Anspruch 10, worin $R^1$ Methyl, Ethyl, Isopropyl, tert.Butyl oder Phenyl ist und $R^7$ 1-Hydroxyethyl, Methyl oder Hydroxymethyl ist.

12. Ein Verfahren zur Herstellung einer Verbindung nach Anspruch 9, umfassend die Behandlung einer Verbindung der Formel:

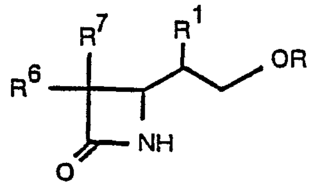
,

worin R eine leicht entfernbare Schutzgruppe ist, mit einem Glyoxylatester, worin der Esterrest ein pharmazeutisch annehmbarer Esterrest oder eine leicht entfernbare blockierende Gruppe R′ ist, unter Bildung einer Verbindung der Struktur:

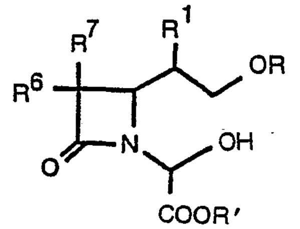
,

gefolgt von der Halogenierung unter Bildung einer Verbindung der Struktur:

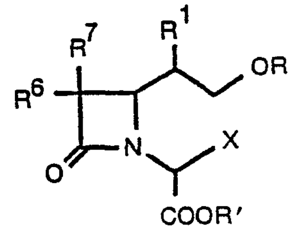
,

worin X Halogen ist, gefolgt von der Behandlung mit Triphenylphosphin und milder wässeriger Hydrolyse unter Bildung von

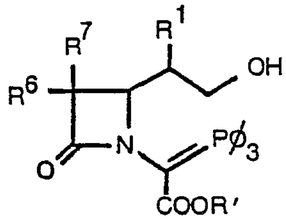

gefolgt von der Cyclisierung obiger Struktur nach Behandlung mit einem Oxydationsmittel; gefolgt von der Entfernung der blockierenden Gruppe.

13. Ein Verfahren zur Herstellung einer Verbindung nach Anspruch 9, umfassend die Cyclisierung von

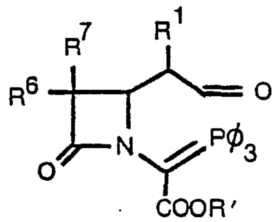

worin Ø Phenyl ist und R' ein pharmazeutisch annehmbarer Esterrest oder eine leicht entfernbare blockierende Gruppe ist, gefolgt von der Entfernung der blockierenden Gruppe.

14. Eine Verbindung nach Anspruch 9 mit der Formel:

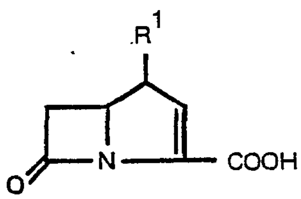

und deren pharmazeutisch annehmbare Salze und Ester, worin $R^1$ aus substituiertem und unsubstituiertem Niedrigalkyl mit 1 bis 6 Kohlenstoffatomen, Phenyl, Phenylniedrigalkyl, Cycloalkyl mit 3 bis 6 Kohlenstoffatomen und Cycloalkylalkyl mit 4 bis 7 Kohlenstoffatomen in der Kette und 3 bis 6 Kohlenstoffatomen im Ring ausgewählt ist; worin diese Substituenten an $R^1$ ausgewählt sind aus Chlor, Brom, Fluor, Hydroxyl, Amino, Alkoxyl, Cyano, Carboxyl, Mono-, Di- und Trialkylamino (jedes Alkyl hat 1 bis 6 Kohlenstoffatome).

15. Eine Verbindung nach Anspruch 14, worin $R^1$ Methyl, Phenyl, Ethyl, Cyclopropyl, Propyl und Isopropyl ist.

16. Ein Verfahren zur Herstellung einer Verbindung nach Anspruch 14, umfassend die Behandlung einer Verbindung der Formel:

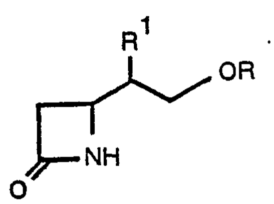

worin R eine leicht entfernbare Schutzgruppe ist, mit einem Glyoxylatester, worin der Esterrest ein pharmazeutisch annehmbarer Esterrest oder eine leicht entfernbare blockierende Gruppe $R^2$ ist, unter Bildung einer Verbindung der Struktur:

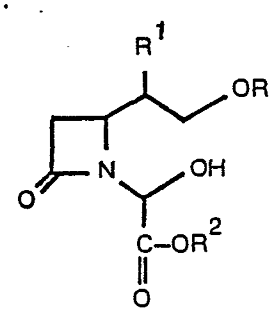

gefolgt von einer Halogenierung unter Bildung einer Verbindung der Struktur:

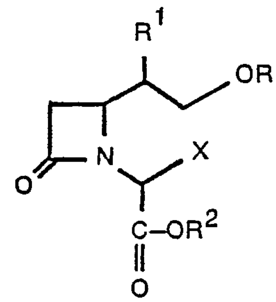

worin X Halogen ist, gefolgt von der Behandlung mit Triphenylphosphin und milder, wässeriger Hydrolyse unter Bildung von:

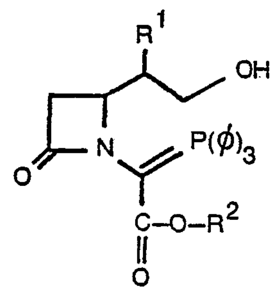

gefolgt von der Cyclisierung der obigen Struktur nach Behandlung mit einem Oxydationsmittel; gefolgt von der Entfernung der blockierenden Gruppe.

17. Ein Verfahren zur Herstellung einer Verbindung nach Anspruch 14, umfassend die Cyclisierung von

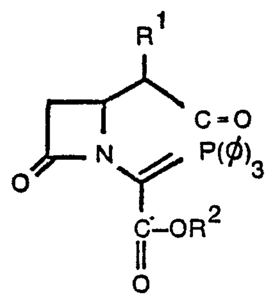

worin Ø Phenyl ist und $R^2$ ein pharmazeutisch annehmbarer Esterrest oder eine leicht entfernbare blockierende Gruppe ist, gefolgt von der Entfernung der blockierenden Gruppe.

18. Eine Verbindung nach Anspruch 1 mit der Formel:

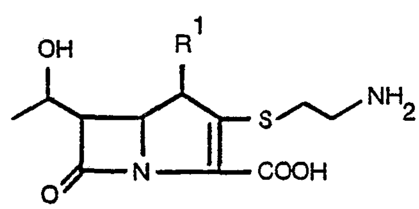

und deren pharmazeutisch annehmbare Salze und Ester, worin $R^1$ ausgewählt ist aus substituiertem und unsubstituiertem Niedrigalkyl mit 1 bis 6 Kohlenstoffatomen, Phenyl, Phenylniedrigalkyl, Cycloalkyl mit 3 bis 6 Kohlenstoffatomen und Cycloalkylalkyl mit 4 bis 7 Kohlenstoffatomen in der Kette und 3 bis 6 Kohlenstoffatomen im Ring; worin diese Substituenten an $R^1$ ausgewählt sind aus Chlor, Brom Fluor, Hydroxyl, Amino, Alkoxyl mit 1 bis 6 Kohlenstoffatomen, Cyano und Carboxyl.

19. Eine Verbindung nach Anspruch 18, worin $R^1$ Methyl, Phenyl, Benzyl, Ethyl, Cyclopropyl, Propyl und Isopropyl ist.

20. Eine Verbindung nach Anspruch 19, worin $R^1$ Methyl ist und welche die Struktur

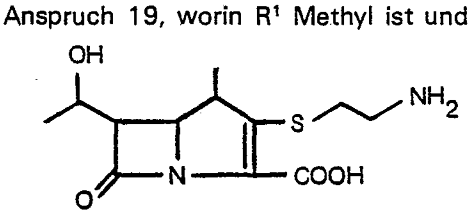

hat.

21. Eine Verbindung nach Anspruch 1 mit der Formel:

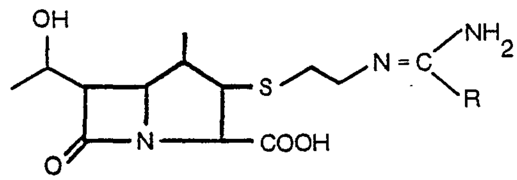

worin R H oder Methyl ist.

22. Ein Verfahren zur Herstellung einer Verbindung nach Anspruch 1 mit der Struktur:

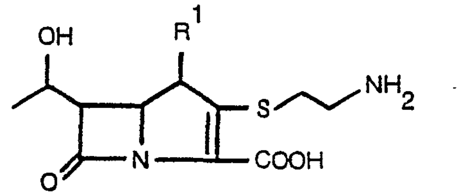

und seiner pharmazeutisch annehmbaren Salze, umfassend die Cyclisierung von

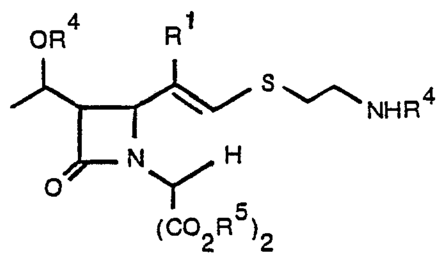

durch Halogenierung und Behandlung des erhaltenen Zwischenproduktes mit einer Base unter Bildung von

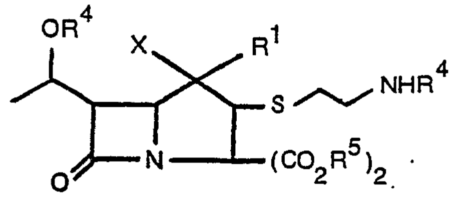

die Dehydrohalogenierung des entstehenden Zwischenproduktes in Gegenwart von Base unter Bildung von:

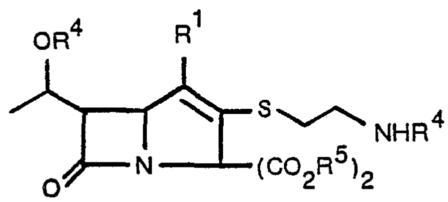

das Erhitzen des entstehenden Zwischenproduktes in Gegenwart eines Verdrängungsmittels unter Bildung von:

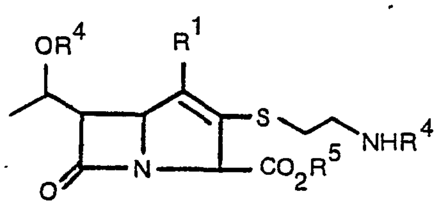

die Isomerisierung der Stellung der Doppelbindung des entstehenden Zwischenproduktes durch Behandlung in einem Lösungsmittel mit einer starken Base unter Bildung von:

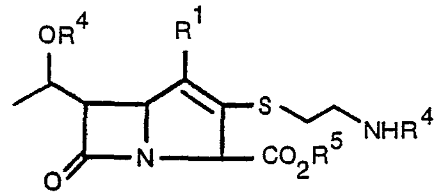

die Entblockierung unter Bildung von:

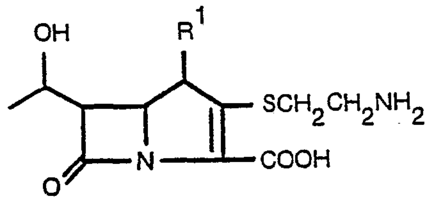

worin $R^4$ und $R^5$ blockierende Gruppen sind und X Halogen ist.

23. Ein Verfahren zur Herstellung einer Verbindung nach Anspruch 18 mit der Struktur:

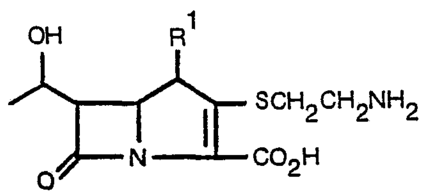

umfassend die Behandlung von:

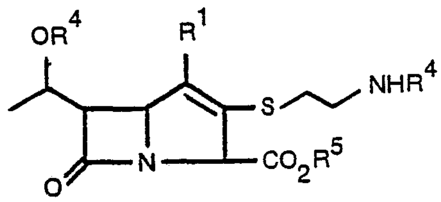

mit einer zur Isomerisierung der Doppelbindung befähigten Base unter Bildung von

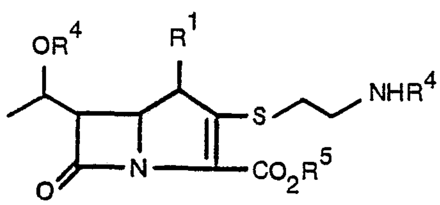

worin $R^4$ und $R^5$ blockierende Gruppen sind; gefolgt von der Entfernung der blockierenden Gruppe.

24. Ein antibiotische, pharmazeutische Zusammensetzung, enthaltend eine therapeutisch wirksame Menge einer Verbindung nach den Ansprüchen 1 bis 4, 7 bis 11, 14, 15 oder 18 bis 21 und einen pharmazeutischen Träger dafür.

25. Verbindung mit der im Anspruch 1 angegebenen Formel, worin $R^7$ Wasserstoff ist, $R^1$ ausgewählt ist aus substituiertem und unsubstituiertem Niedrigalkyl mit 1 bis 6 Kohlenstoffatomen, Cyclopropyl, Benzyl und Phenyl; und $R^6$ ein $\alpha$-substituiertes Alkyl ist, worin der $\alpha$-Substituent Hydroxyl, Amino oder Mercapto ist und worin der Alkylrest gerade oder verzweigt ist und 1 bis 6 Kohlenstoffatome umfaßt; wobei der Substituent in bezug auf die obigen Reste für $R^1$ Phenyl ist.

26. Verbindungen nach den Ansprüchen 1, 2, 3, 4 und 8, worin die pharmazeutisch annehmbaren Esterreste R Pivaloyloxymethyl, Allyl, Methallyl, (2-Methylthio)-ethyl, 3-Methyl-2-butenyl, p-tert. Butylbenzyl, 5-Indanyl, 3-Phthalidyl sind.

**Patentansprüche fur der Vertragsstaat: AT**

1. Ein Verfahren zur Herstellung einer Verbindung mit der Struktur:

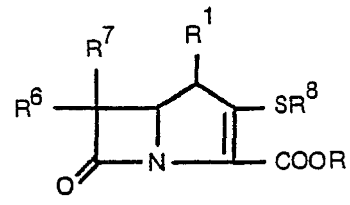

und der pharmazeutisch annehmbaren Salze derselben, worin R H; pharmazeutisch annehmbares Salzkation; oder ein pharmazeutisch annehmbarer Esterrest ist; und $R^1$, $R^6$, $R^7$ und $R^8$ unabhängig voneinander ausgewählt sind aus Wasserstoff ($R^1$ ist nicht Wasserstoff), substituiertem und unsubstituiertem: Alkyl, Alkenyl und Alkinyl mit 1 bis 10 Kohlenstoffatomen; Cycloalkyl, Cycloalkylalkyl und Alkylcycloalkyl mit 3 bis 6 Kohlenstoffatomen im Cycloalkylring und 1 bis 6 Kohlenstoffatomen in den Alkylresten; Phenyl; Aralkyl, Aralkenyl und Aralkinyl, worin der Arylrest Phenyl ist und die Alkylkette 1 bis 6 Kohlenstoffatome hat; Heterocyclyl und Heterocyclylalkyl, worin der Substituent oder die Substituenten in bezug auf die oben genannten Reste ausgewählt sind aus: Amino, Mono-, Di- und Trialkylamino, Hydroxyl, Alkoxyl, Mercapto, Alkthio, Phenylthio, Sulfamoyl, Amidino, Guanidino, Nitro, Chlor, Brom, Fluor, Cyano und Carboxy; und worin das Heteroatom oder die Heteroatome in den oben genannten heterocyclischen Resten ausgewählt sind aus 1—4 Sauerstoff-, Stickstoff- oder Schwefelatomen; und worin die Alkylreste der oben genannten Substituenten 1 bis 6 Kohlenstoffatome haben. umfassend die Cyclisierung von

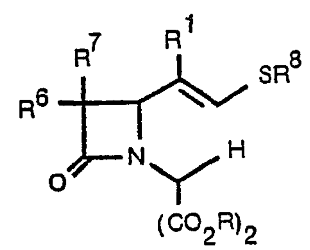

durch Halogenierung und Behandlung des erhaltenen Zwischen produktes mit einer Base unter Bildung von

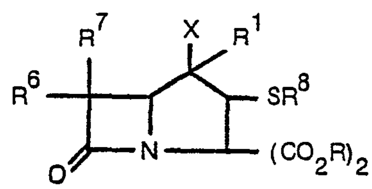

die Dehydrohalogenierung des entstehenden Zwischenproduktes in Gegenwart von Base unter Bildung von

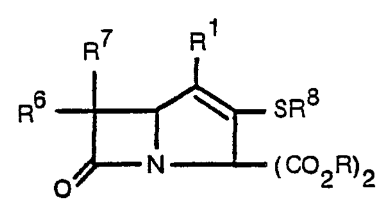

das Erhitzen des entstehenden Zwischenproduktes in Gegenwart eines Verdrängungsmittels unter Bildung von

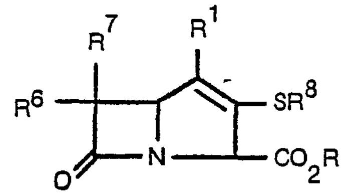

die Isomerisierung der Stellung der Doppelbindung des entstehenden Zwischenproduktes durch Behandlung in einem Lösungsmittel mit einer starken Base unter Bildung von

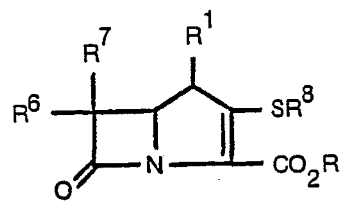

worin X Halogen ist.

2. Ein Verfahren zur Herstellung einer Verbindung nach Anspruch 1, umfassend die Decarboxylierung von

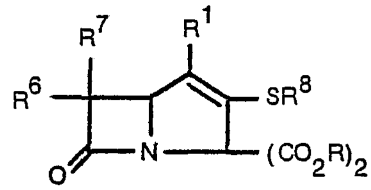

durch Behandlung mit einem Verdrängungsmittel und die weitere Behandlung mit einer zur Isomerisierung der Doppelbindung befähigten Base unter Bildung von

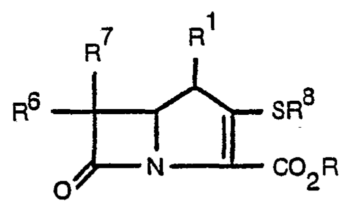

3. Eine Verfahren zur Herstellung einer Verbindung mit der Struktur:

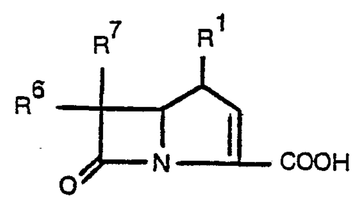

und der pharmazeutisch annehmbaren Salze und Ester derselben; worin

a) die Substituenten $R^1$, $R^6$ und $R^7$ unabhängig voneinander ausgewählt sind aus Wasserstoff ($R^1$ ist nicht H); substituiertem und unsubstituiertem: geradkettigem und verzweigtem Niedrigalkyl mit 1 bis 10 Kohlenstoffatomen; Cycloalkyl mit 3 bis 6 Kohlenstoffatomen; Cycloalkylalkyl, worin der Cycloalkylrest 3 bis 6 Kohlenstoffatome und der Alkylrest 1 bis 10 Kohlenstoffatome umfaßt; Alkylcycloalkyl, worin der Alkylrest 1 bis 6 Kohlenstoffatome und der Cycloalkylrest 3 bis 6 Kohlenstoffatome umfaßt; Phenyl und Naphthyl; Phenylalkyl; Heterocyclyl (gesättigt und ungesättigt), umfassend mono- und bicyclische Strukturen mit 5 bis 10 Ringatomen, worin eines oder mehrere der Heteroatome aus Sauerstoff, Stickstoff oder Schwefel gewählt sind; Heterocyclyalkyl, welches die unmittelbar vorhergehenden Heterocyclylreste und die 1 bis 10 Kohlenstoffatome enthaltenden Alkylrest umfaßt; wobei der Substituent (oder die Substituenten) in bezug auf die oben genannten Reste ausgewählt ist (sind) aus Amino, Hydroxyl, Cyano, Carboxyl, Nitro, Chlor, Brom, Fluor, Niedrigalkoxy mit 1 bis 6 Kohlenstoffatomen, Mercapto, Perhalogenniedrigalkyl, Niedrigalkylthio, Guanidino, Amidino, Sulfamoyl, und N-substituiertem: Sulfamoyl, Amidino und Guanidino, worin der N-Substituent Niedrigalkyl mit 1 bis 6 Kohlenstoffatomen oder Aryl mit 6 bis 10 Kohlenstoffatomen ist, oder

b) die Substituenten $R^1$, $R^6$ und $R^7$ unabhängig voneinander ausgewählt sind aus Wasserstoff ($R^1$ ist nicht Wasserstoff), substituiertem und unsubstituiertem: Alkyl, Alkenyl und Alkinyl mit 1 bis 10 Kohlenstoffatomen; Cycloalkyl, Cycloalkylalkyl und Alkylcycloalkyl mit 3 bis 6 Kohlenstoffatomen im Cycloalkylring und 1 bis 6 Kohlenstoffatomen in den Alkylresten; Phenyl; Aralkyl, Aralkenyl und Aralkinyl, worin der Arylrest Phenyl ist und die lineare Kette 1 bis 6 Kohlenstoffatome hat; Heterocyclyl und Heterocyclylalkyl; worin der Substituent oder die Substituenten in bezug auf die oben genannten Reste ausgewählt sind aus: Amino, Mono-, Di- und Trialkylamino, Hydroxyl, Alkoxyl, Mercapto, Alkylthio, Phenylthio, Sulfamoyl, Amidino, Guanidino, Nitro, Chlor, Brom, Fluor, Cyano und Carboxy; und worin das

Heteroatom oder die Heteroatome in den oben gennanten heterocyclischen Resten ausgewählt sind aus 1—4 Sauerstoff-, Stickstoff- oder Schwefelatomen; und worin die Alkylreste der oben genannten Substituenten 1 bis 6 Kohlenstoffatome haben, umfassend die Behandlung einer Verbindung der Formel:

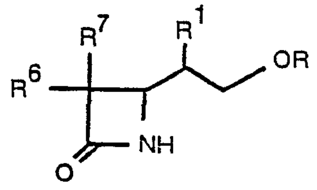

worin R eine leicht entfernbare Schutzgruppe ist, mit einem Glyoxylatester, worin der Esterrest ein pharmazeutisch annehmbarer Esterrest oder eine leicht entfernbare blockierende Gruppe R′ ist, unter Bildung einer Verbindung der Struktur:

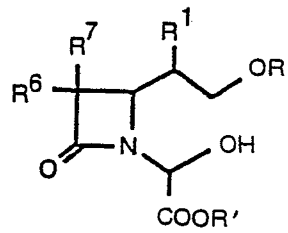

gefolgt von der Halogenierung unter Bildung einer Verbindung der Struktur:

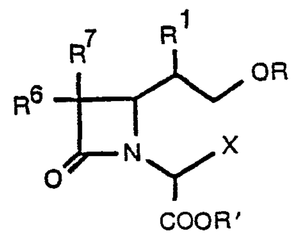

worin X Halogen ist, gefolgt von der Behandlung mit Triphenylphosphin und milder wässeriger Hydrolyse unter Bildung von

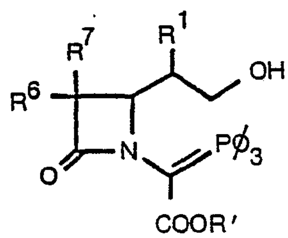

gefolgt von der Cyclisierung obiger Struktur nach Behandlung mit einem Oxydationsmittel; gefolgt von der Entfernung der blockierenden Gruppe.

4. Ein Verfahren zur Herstellung einer Verbindung nach Anspruch 3, umfassend die Cyclisierung von

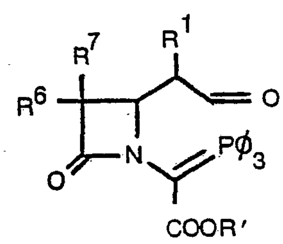

worin Ø Phenyl ist und R′ ein pharmazeutisch annehmbarer Esterrest oder eine leicht entfernbare blockierende Gruppe ist, gefolgt von der Entfernung der blockierenden Gruppe.

5. Ein Verfahren zur Herstellung einer Verbindung mit der Formel

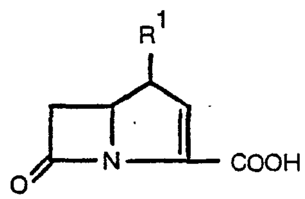

und der pharmazeutisch annehmbaren Salz und Ester derselben, worin $R^1$ aus der aus substituiertem und unsubstituiertem Niedrigalkyl mit 1 bis 6 Kohlenstoffatomen, Phenyl, Phenylniedrigalkyl, Cycloalkyl mit 3 bis 6 Kohlenstoffatomen und Cycloalkylalkyl mit 4 bis 7 Kohlenstoffatomen in der Kette und 3 bis 6 Kohlenstoffatomen im Ring bestehenden Gruppe ausgewählt ist; worin diese Substituenten an $R^1$ ausgewählt sind aus Chlor, Brom, Fluor, Hydroxyl, Amino, Mono-, Di- und Trialkylamino (jedes Alkyl hat 1 bis 6 Kohlenstoffatome), Alkoxyl, Cyano, Carboxyl, umfassend die Behandlung einer Verbindung der Formel:

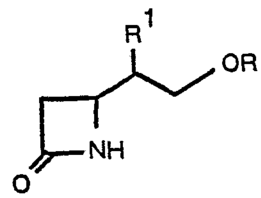

worin R eine leicht entfernbare Schutzgruppe ist, mit einem Glyoxylatester, worin der Esterrest ein pharmazeutisch annehmbarer Esterrest oder eine leicht entfernbare blockierende Gruppe $R^2$ ist, unter Bildung einer Verbindung der Struktur:

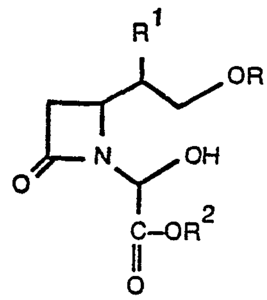

gefolgt von einer Halogenierung unter Bildung einer Verbindung der Struktur:

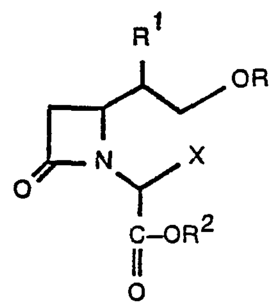

worin X Halogen ist, gefolgt von der Behandlung mit Triphenylphosphin und milder, wässeriger Hydrolyse unter Bildung von:

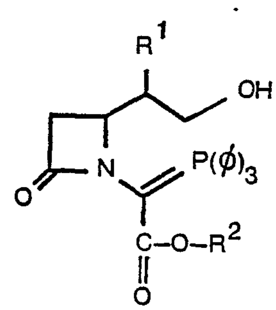

gefolgt von der Cyclisierung der obigen Struktur nach Behandlung mit einem Oxydationsmittel; gefolgt von der Entfernung der blockierenden Gruppe.

6. Ein Verfahren zur Herstellung einer Verbindung nach Anspruch 5, umfassend die Cyclisierung von

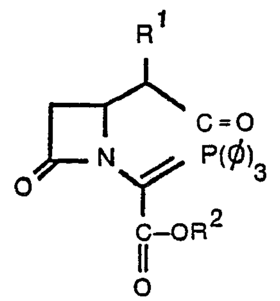

worin Ø Phenyl ist und $R^2$ ein pharmazeutisch annehmbarer Esterrest oder eine leicht entfernbare blockierende Gruppe ist, gefolgt von der Entfernung der blockierenden Gruppe.

7. Eine Verfahren zur Herstellung einer Verbindung nach Anspruch 1 mit der Struktur:

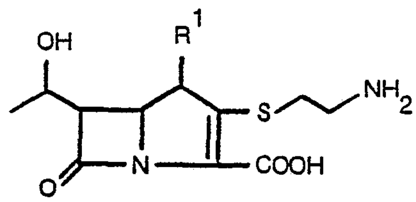

und seiner pharmazeutisch annehmbaren Salze, umfassend die Cyclisierung von

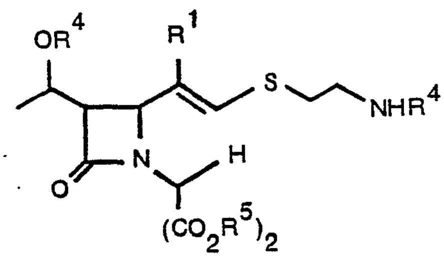

durch Halogenierung und Behandlung des erhaltenen Zwischenproduktes mit einer Base unter Bildung von

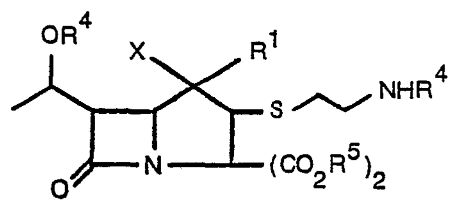

die Dehydrohalogenierung des entstehenden Zwischenproduktes in Gegenwart von Base unter Bildung von:

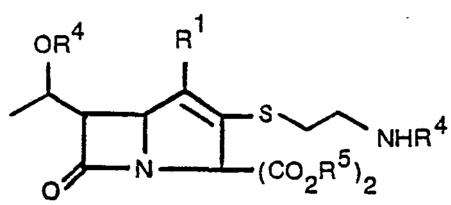

das Erhitzen des entstehenden Zwischenproduktes in Gegenwart eines Verdrängungsmittels unter Bildung von:

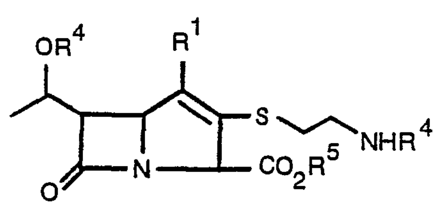

die Isomerisierung der Stellung der Doppelbindung des entstehenden Zwischenproduktes durch Behandlung in einem Lösungsmittel mit einer starken Base unter Bildung von:

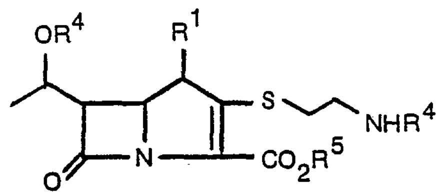

die Entblockierung unter Bildung von:

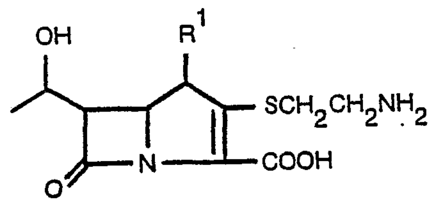

worin $R^4$ und $R^5$ blockierende Gruppen sind und X Halogen ist.

8. Ein Verfahren nach Anspurch 7, zur Herstellung einer Verbindung nach Anspruch 1 mit der Formel

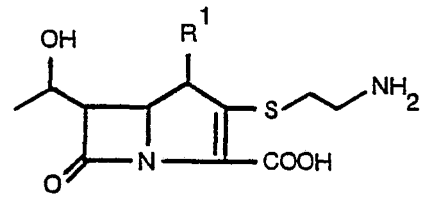

und seiner pharmazeutisch annehmbaren Salze und Ester, worin $R^1$ ausgewählt ist aus substituiertem und unsubstituiertem Niedrigalkyl mit 1 bis 6 Kohlenstoffatomen, Phenyl, Phenylniedrigalkyl, Cycloalkyl mit 3 bis 6 Kohlenstoffatomen und Cycloalkylalkyl mit 4 bis 7 Kohlenstoffatomen in der Kette und 3 bis 6 Kohlenstoffatomen im Ring; worin diese Substituenten an $R^1$ ausgewählt sind aus Chlor, Brom, Fluor, Hydroxyl, Amino, Alkoxyl mit 1 bis 6 Kohlenstoffatomen, Cyano und Carboxyl.

9. Ein Verfahren zur Herstellung einer Verbindung mit der Formel

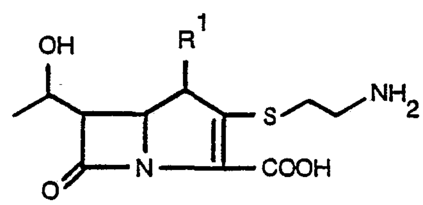

worin $R^1$ ausgewählt ist aus substituiertem und unsubstituiertem niedrigalkyl mit 1 bis 6 Kohlenstoffatomen, Phenyl, Phenylniedrigalkyl, Cycloalkyl mit 3 bis 6 Kohlenstoffatomen und Cycloalkylalkyl mit 4 bis 7 Kohlenstoffatomen in der Kette und 3 bis 6 Kohlenstoffatomen im Ring; worin diese Substituenten an $R^1$ ausgewählt sind aus Chlor, Brom, Fluor, Hydroxyl, Amino, Alkoxy mit 1 bis 6 Kohlenstoffatomen, Cyano und Carboxyl, umfassend die Behandlung von

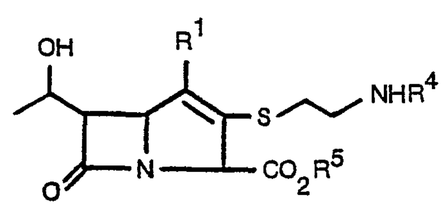

mit einer zur Isomerisierung der Doppelbindung befähigten Base unter Bildung von

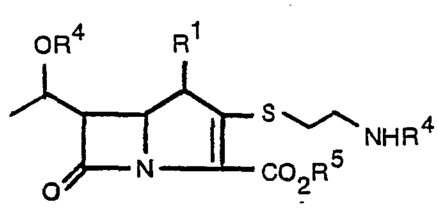

worin $R^4$ und $R^5$ blockierende Gruppen sind; gefolgt von der Entblockierung.

10. Ein Verfahren zur Herstellung einer Verbindung der in den Ansprüchen 1 und 3 angegebenen Formel, worin $R^7$ Wasserstoff ist, $R^1$ ausgewählt ist aus substituiertem und unsubstituiertem Niedrigalkyl mit 1 bis 6 Kohlenstoffatomen, Cyclopropyl, Benzyl und Phenyl; und $R^6$ ein $\alpha$-substituiertes Alkyl ist, worin der $\alpha$-Substituent Hydroxyl, Amino oder Mercapto ist und worin der Alkylrest gerade oder verzweigt ist und 1 bis 6 Kohlenstoffatome umfaßt; wobei der Substituent in bezug auf die obigen Reste für $R^1$ Phenyl ist, dadurch gekennzeichnet, daß es wie in Anspruch 1, 2 oder 3 beschrieben ausgeführt wird, worin $R^1$, $R^6$ und $R^7$ in den Ausgangsverbindungen wie oben definiert sind.

11. Ein Verfahren zur Herstellung einer Verbindung nach den Ansprüchen 1 und 2, worin die pharmazeutisch annehmbaren Esterreste R Pivaloyloxymethyl, Allyl, Methallyl, (2-Methylthio)-ethyl, 3-Methyl-2-butenyl, p-tert.Butylbenzyl, 4-Indanyl, 3-Phthalidyl sind.

**Revendications pour les Etats contractants: BE CH DE FR GB IT LU NL SE**

1. Un composé ayant pour structure:

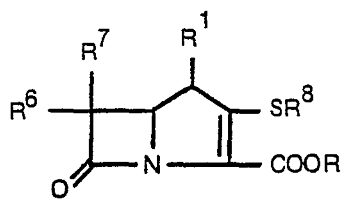

et les sels acceptables en pharmacie correspondants où R est H; un cation de sel acceptable en pharmacie; ou un fragment ester acceptable en pharmacie; et $R^1$, $R^6$, $R^7$ et $R^8$ sont indépendamment choisis parmi un hydrogène, ($R^1$ n'est pas H), et des radicaux substitués et non substitués: alkyle, alcényle et alcynyle, ayant 1 à 10 atomes de carbone; cycloalkyle, cycloalkylalkyle et alkylcycloalkyle ayant 3 à 6 atomes de carbone dans le cycle cycloalkyle et 1 à 6 atomes de carbone dans les fragments alkyles; phényle; aralkyle, aralcényle et aralcynyle dont le fragment aryle est un phényle et la chaîne alkyle a 1 à 6 atomes de carbone; hétérocyclyle et hétérocyclylalkyle dans lesquels le substituant ou les substituants relatifs aux radicaux précités sont choisis parmi: amino, mono-, di- et tri-alkylamino, hydroxy, alcoxy, mercapto, alkylthio, phénylthio, sulfamoyle, amidino, guanidino, nitro, chloro, bromo, fluoro, cyano et carboxy; et où le ou les hétéro-atomes des fragments hétérocycliques précités sont choisis parmi 1 à 4 atomes d'oxygène, d'azote ou de soufre; et où les fragments alkyles des substituants précités ont 1 à 6 atomes de carbone.

2. Un composé selon la revendication 1, où $R^1$ est un alkyle ayant 1 à 10 atomes de carbone ou un cycloalkyle ayant 3 à 6 atomes de carbone.

3. Un composé selon la revendication 1 où $R^6$ est H ou un méthyle et $R^7$ est un alkyle ayant 1 à 10 atomes de carbone, phényle un aralkyle ou un alkyle, phényle ou aralkyle hydroxy-substitués où le fragment aryle est phényle et la chaîne alkyle a 1 à 6 atomes de carbone.

4. Un composé selon la revendication 1 où $R^6$ est un hydrogène ou un méthyle et $R^8$ est choisi parmi:

H, $CH_3$, $(CH_2)_2NH_2$, $C(CH_3)_2CH_2NH_2$, 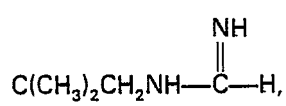, 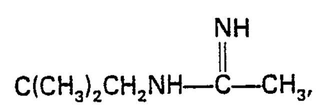,

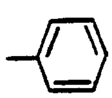, 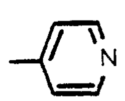, 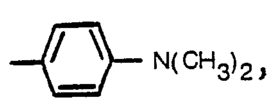, 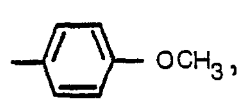, $CH_2CH_2CH_2NH_2$,

$CH_2CH(CH_3)NH_2$, 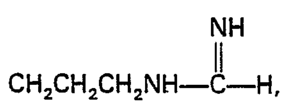, 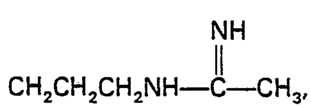, 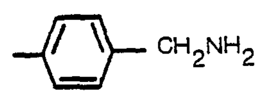,

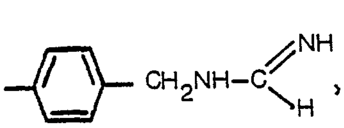, 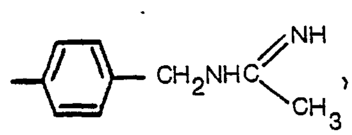, 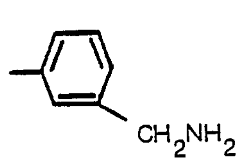,

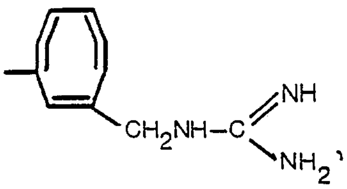, 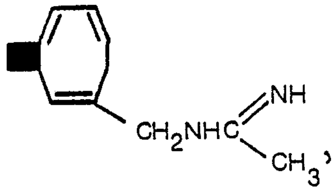, 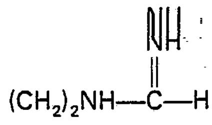, 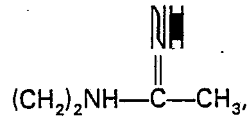,

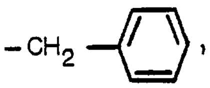, 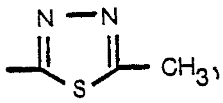 , 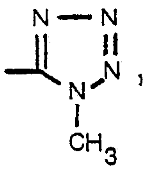, $CH(CH_3)CH_2NH_2$,

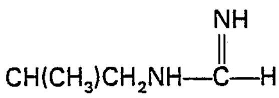 ou 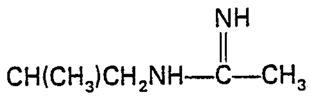

et $R^7$ est choisi parmi: $—CH_2OH$, 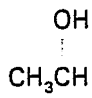, 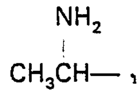, 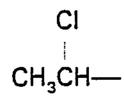, 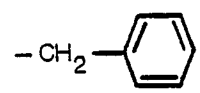,

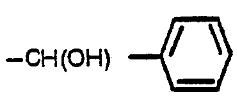, $—CH_3$, $—CH_2CH_3$, 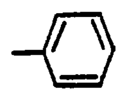

5. Un procédé pour préparer un composé selon la revendication 1, comprenant: la cyclisation de

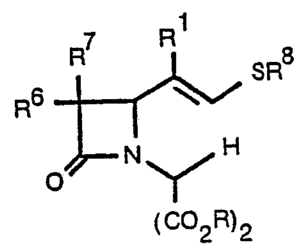

par halogénation et traitement de l'intermédiaire obtenu avec une base pour former:

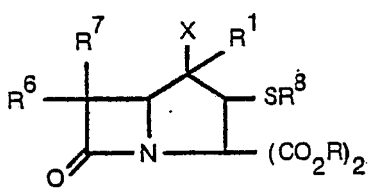

la déshydrohalogénation de l'intermédiaire obtenu en présence d'une base pour former:

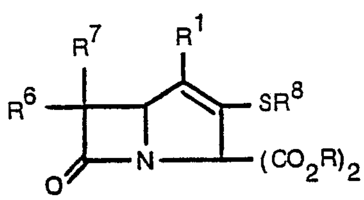

le chauffage de l'intermédiaire obtenu en présence d'un agent de déplacement pour former:

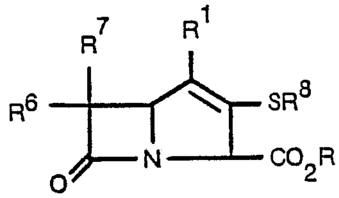

l'isomérisation de la position de la double laision de l'intermédiaire obtenu par traitement dans un solvant avec une base forte pour former:

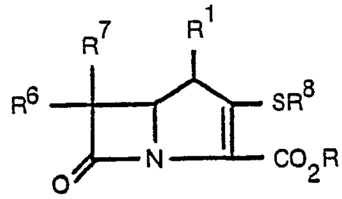

où X est un halogéno.

6. Un procédé pour préparer un composé selon la revendication 1 comprenant la décarboxylation de

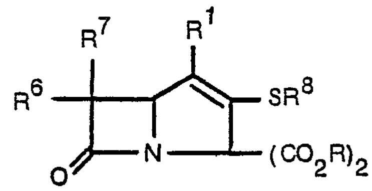

par traitement avec un agent de déplacement et de plus le traitement avec une base capable d'isomériser la double liaison pour former:

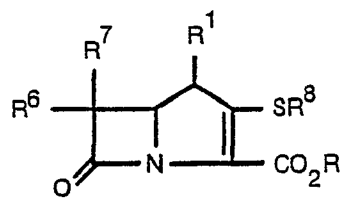

7. Un composé selon la revendication 1, ayant pour structure:

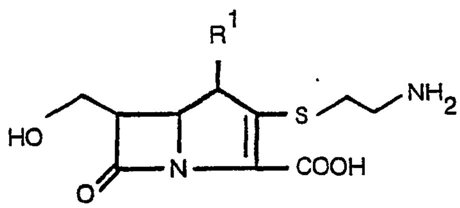

et ses sels et esters acceptables en pharmacie, où $R^1$ est défini comme dans la revendication 1.

8. Un composé selon la revendication 1, ayant pour structure:

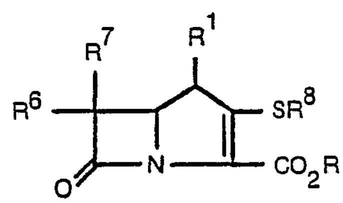

et les sels acceptables en pharmacie correspondants où R est H; un cation de sel acceptable en pharmacie; ou un fragment ester acceptable en pharmacie; et $R^6$, $R^7$ et $R^8$ sont choisis indépendamment parmi un hydrogène, un radical substitué ou non substitué: alkyle, alcényle et alcynyle ayant 1 à 10 atomes de carbone, cycloalkyle, cycloalkylalkyle et alkylcycloalkyle, ayant 3 à 6 atomes de carbone dans le cycle cycloalkyle et 1 à 6 atomes de carbone dans les fragments alkyles; phényle; aralkyle, aralcényle, et aralcynyle dont le fragment aryle est phényle et la chaîne alkyle a 1 à 6 atomes de carbone; où le substituant ou les substituants des radicaux précités sont choisis parmi amino, mono-, di- et trialkylamino, hydroxy, alcoxy, mercapto, alkylthio, phénylthio, sulfamoyle, amidino, guanidino, nitro, chloro, bromo, fluoro, cyano et carboxy; et où les fragments alkyles des substituants précités ont 1 à 6 atomes de carbone;

$R^1$ est choisi parmi méthyle, éthyle, propyle, isopropyle, cyclopropyle, phényle et benzyle.

9. Un composé ayant pour structure:

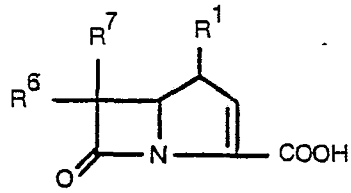

et les sels et esters acceptables en pharmacie correspondants; où

a) les substituants $R^1$, $R^6$ et $R^7$ sont choisis indépendamment parmi un hydrogène ($R^1$ n'est pas H); des radicaux substitués et non substitués: alkyle inférieur droit ou ramifié ayant 1 à 10 atomes de carbone; cycloalkyle ayant 3 à 6 atomes de carbone; cycloalkylalkyle dont le fragment cycloalkyle comprend 3 à 6 atomes de carbone et le fragment alkyle comprend 1 à 10 atomes de carbone; alkylcycloalkyle dont le fragment alkyle comprend 1 à 6 atomes de carbone et le fragment cycloalkyle comprend 3 à 6 atomes de carbone; phényle et naphtyle; phénylalkyle; hétérocyclyle (saturé et non saturé) comprenant des structures mono- et bicycliques ayant 5 à 10 atomes cycliques où un ou plusieurs des hétéroatomes sont choisis parmi l'oxygène, l'azote ou le soufre; hétérocyclylalkyle comprenant les fragments hétérocyclyles juste précités et dont le fragment alkyle comprend 1 à 10 atomes de carbone; le substituant (ou les substituants) des radicaux précités est (sont) choisi(s) parmi amino, hydroxy, cyano, carboxy, nitro, chloro, bromo, fluoro, alcoxy inférieur ayant 1 à 6 atomes de carbone, mercapto, perhalogénoalkyle inférieur, alkylthio inférieur, guanidino, amidino, sulfamoyle et sulfamoyle, amidino et guanidino N-substitués dont le substituant à l'azote est un alkyle inférieur ayant 1 à 6 atomes de carbone ou un aryle ayant 6 à 10 atomes de carbone ou

b) les substituants $R^1$, $R^6$ et $R^7$ sont choisis indépendamment parmi l'hydrogène ($R^1$ n'est pas un hydrogène), les radicaux substitués et non substitués: alkyle, alcényle et alcynyle ayant 1 à 10 atomes de carbone; cycloalkyle, cycloalkylalkyle et alkylcycloalkyle ayant 3 à 6 atomes de carbone dans le cycle cycloalkyle et 1 à 6 atomes de carbone dans les fragments alkyles; phényle; aralkyle, aralcényle et aralcynyle dont le fragment aryle est un phényle et la chaîne linéaire a 1 à 6 atomes de carbone; hétérocyclyle et hétérocyclylalkyle; où le substituant ou les substituants des radicaux précités sont choisis parmi: amino, mono-, di- et trialkylamino, hydroxy, alcoxy, mercapto, alkylthio, phénylthio, sulfamoyle, amidino, guanidino, nitro, chloro, bromo, fluoro, cyano et carboxy; et où le ou les hétéro-atomes des fragments hétérocycliques précités sont choisis parmi 1 à 4 atomes d'oxygène, d'azote ou de soufre; et où les fragments alkyles des substituants précités ont 1 à 6 atomes de carbone.

10. Un composé selon la revendication 9, où $R^1$ est un alkyle ayant 1 à 6 atomes de carbone, cyclopropyle, benzyle ou phényle, $R^6$ est un hydrogène et $R^7$ est un alkyle ayant 1 à 6 atomes de carbone ou un phénylalkyle dont le fragment alkyle a 1 à 6 atomes de carbone, substitué par un hydroxy ou un amino.

11. Un composé selon la revendication 10, où $R^1$ est un méthyle, éthyle, isopropyle, tert-butyle ou phényle et $R^7$ est un 1-hydroxyéthyle, méthyle ou hydroxyméthyle.

12. Un procédé pour préparer un composé selon la revendication 9, comprenant le traitement d'un composé de formule:

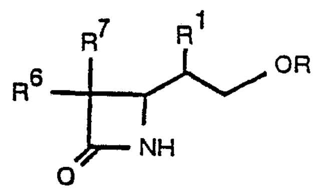

où R est un groupe protecteur facile à éliminer avec un ester glyoxylique dont le fragment ester est un fragment ester acceptable en pharmacie ou un groupe de blocage facile à éliminer, R', pour fournir un composé ayant pour structure:

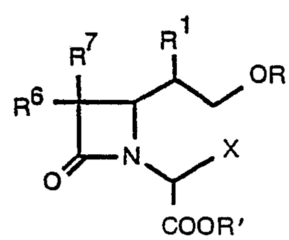

puis une halogénation pour fournir un composé ayant pour structure:

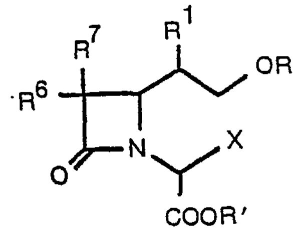

où X est un halogène puis le traitement avec la triphénylphosphine et une hydrolyse aqueuse douce pour fournir:

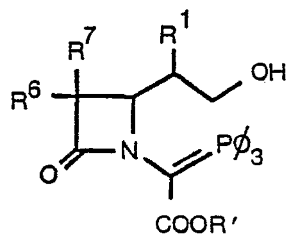

puis la cyclisation de la structure ci-dessus après traitement avec un agent oxydant; suivie de l'élimination du groupe de blocage.

13. Un procédé pour préparer un composé selon la revendication 9, comprenant la cyclisation de:

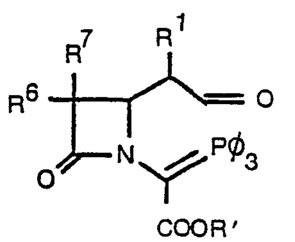

où ∅ est un phényle et R' est un fragment ester acceptable en pharmacie ou un groupe de blocage facile à éliminer; suivie de l'élimination du groupe de blocage.

14. Un composé selon la revendication 9 ayant pour formule:

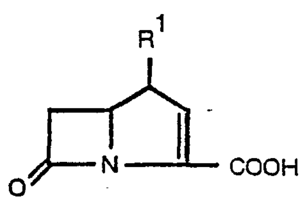

et ses sels et esters acceptables en pharmacie où $R^1$ est choisi parmi les radicaux substitués et non substitués alkyle inférieur ayant 1 à 6 atomes de carbone, phényle, phénylalkyle inférieur, cycloalkyle ayant 3 à 6 atomes de carbone, et cycloalkylalkyle ayant 4 à 7 atomes de carbone dans la chaîne et 3 à 6 atomes de carbone dans le cycle; où lesdits substituants de $R^1$ sont choisis parmi chloro, bromo, fluoro, hydroxy, amino, alcoxy, cyano, carboxy, mono-, di- et trialkylamino (chaque alkyle ayant 1 à 6 atomes de carbone).

15. Un composé selon la revendication 14 où $R^1$ est méthyle, phényle, éthyle, cyclopropyle, propyle et isopropyle.

16. Un procédé pour préparer un composé selon la revendication 14, comprenant le traitement d'un composé de formule:

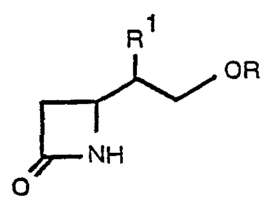

où R est un groupe protecteur facile à éliminer avec un ester glyoxylique dont le fragment ester est un

fragment ester acceptable en pharmacie ou un groupe de blocage facile à éliminer, $R^2$, pour fournir un composé ayant pour structure:

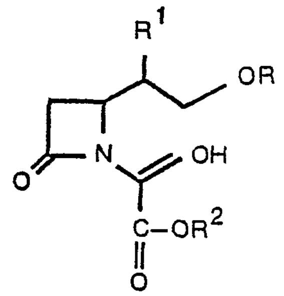

puis une halogénation pour fournir un composé ayant pour structure:

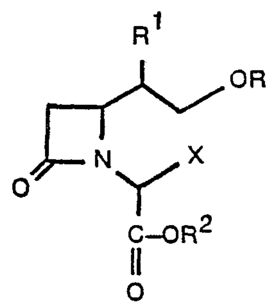

où X est un halogène puis le traitement avec la triphénylphosphine et une hydrolyse aqueuse douce, pour fournir:

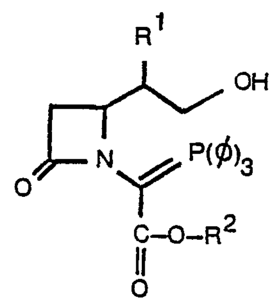

puis la cyclisation de la structure ci-dessus après traitement avec un agent oxydant; puis l'élimination du groupe de blocage.

17. Un procédé pour préparer un composé selon la revendication 14, comprenant la cyclisation de:

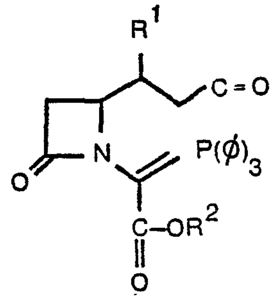

où ∅ est un phényle et $R^2$ est un fragment ester acceptable en pharmacie ou un groupe de blocage facile à éliminer; puis l'élimination du groupe de blocage.

18. Un composé selon la revendication 1, ayant pour formule:

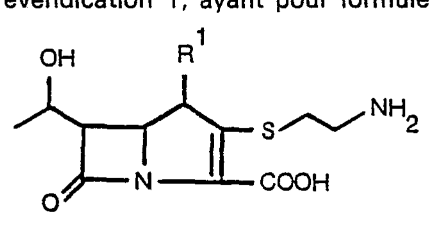

et ses sels et esters acceptables en pharmacie où $R^1$ est choisi parmi les radicaux substitués et non substitués alkyle inférieur ayant 1 à 6 atomes de carbone, phényle, phénylalkyle inférieur, cycloalkyle ayant 3 à 6 atomes de carbone et cycloalkylalkyle ayant 4 à 7 atomes de carbone dans la chaîne et 3 à 6 atomes de carbone dans le cycle; où lesdits substituants de $R^1$ sont choisis parmi chloro, bromo, fluoro, hydroxy, amino, alcoxy ayant 1 à 6 atomes de carbone, cyano et carboxy.

19. Un composé selon la revendication 18, où $R^1$ est méthyle, phényle, benzyle, éthyle, cyclopropyle, propyle et isopropyle.

20. Un composé selon la revendication 19, où $R^1$ est un méthyle ayant pour structure:

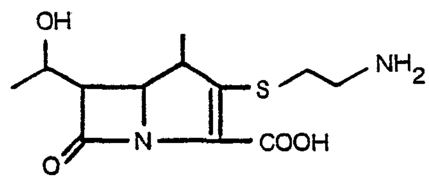

21. Un composé selon la revendication 1 ayant pour formule:

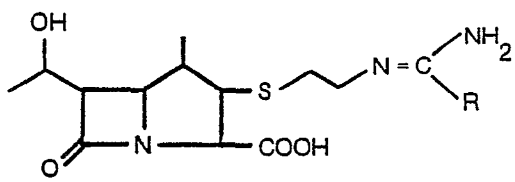

où R est H ou $CH_3$.

22. Un procédé pour préparer un composé selon la revendication 1 ayant pour structure:

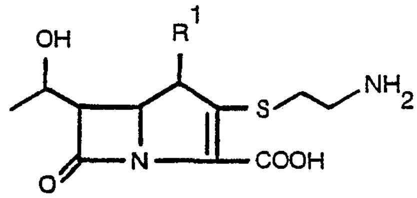

et ses sels acceptables en pharmacie; comprenant la cyclisation de:

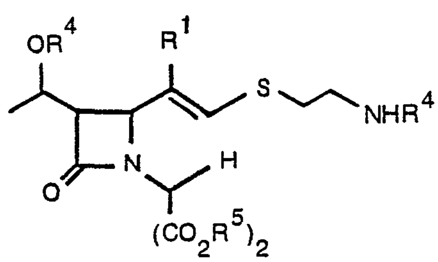

par halogénation et traitement de l'intermédiaire obtenu avec une base pour former:

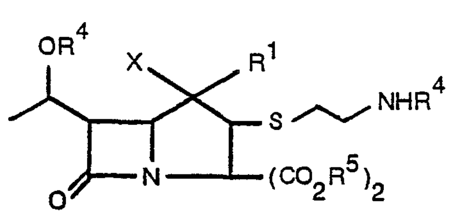

la déshydrohalogénation de l'intermédiaire obtenu en présence d'une base pour former:

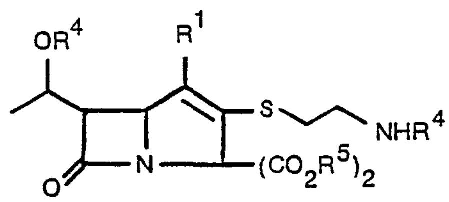

le chauffage de l'intermédiaire obtenu en présence d'un agent de déplacement pour former:

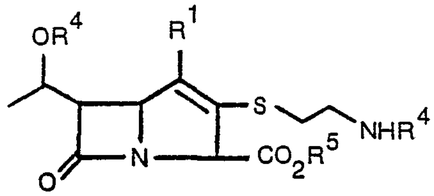

l'isomérisation de la position de la double liaison de l'intermédiaire obtenu par traitement dans un solvant avec une base forte pour former

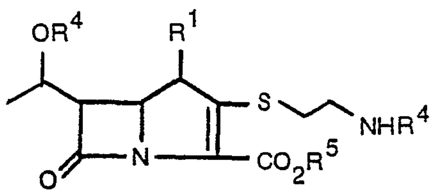 le déblocage pour former: 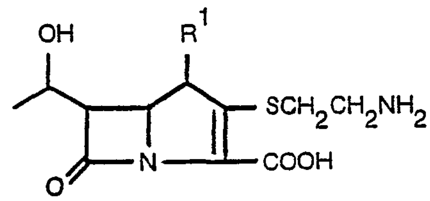

où $R^4$ et $R^5$ sont des groupes de blocage et X est halogéno.

23. Un procédé pour préparer un composé selon la revendication 18 ayant pour structure:

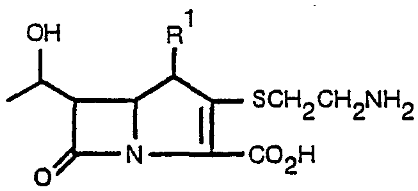 comprenant le traitement de: 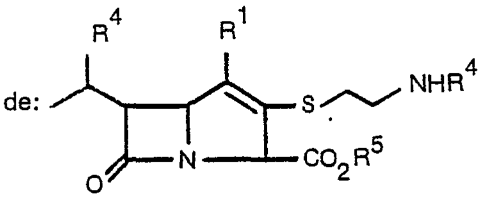

avec une base capable d'isomériser la double liaison pour former:

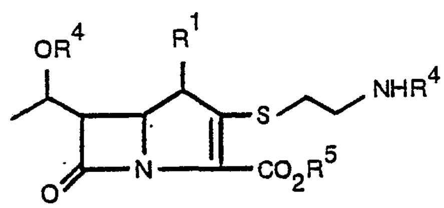

où $R^4$ et $R^5$ sont des groupes de blocage; puis l'élimination du groupe de blocage.

24. Une composition pharmaceutique antibiotique comprenant une quantité thérapeutique efficace d'un composé selon les revendications 1 à 4, 7 à 11, 14, 15 ou 18 à 21 et un de ses supports pharmaceutiques.

25. Composés selon la formule indiquée dans la revendication 1 où $R^7$ est un hydrogène, $R^1$ est choisi parmi les radicaux substitués et non substitués, alkyle inférieur ayant 1 à 6 atomes de carbone, cyclopropyle, benzyle et phényle; et $R^6$ est un alkyle $\alpha$-substitué dont le substituant en $\alpha$ est un hydroxy, un amino, ou un mercapto et où le fragment alkyle est droit ou ramifié et comprend 1 à 6 atomes de carbone; le substituant des radicaux précités pour $R^1$ est un phényle.

26. Composés selon les revendications 1, 2, 3, 4 et 8 où les fragments esters acceptables en pharmacie R sont pivaloyloxyméthyle, allyle, méthallyle, (2-méthylthio)-éthyle, 3-méthyl-2-butényle, p-tert-butylbenzyle, 5-indanyle et 3-phtalidyle.

**Revendications pour l'Etat contractant: AT**

1. Un procédé pour préparer un composé ayant pour structure:

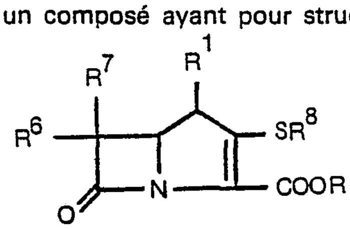

et les sels acceptables en pharmacie correspondants où R est H; un cation de sel acceptable en pharmacie; ou un fragment ester acceptable en pharmacie; et $R^1$, $R^6$, $R^7$ et $R^8$ sont indépendamment choisis parmi un hydrogène, ($R^1$ n'est pas H), et des radicaux substitués et non substitués: alkyle, alcényle et alcynyle, ayant 1 à 10 atomes de carbone; cycloalkyle, cycloalkylalkyle et alkylcycloalkyle ayant 3 à 6 atomes de carbone dans le cycle cycloalkyle et 1 à 6 atomes de carbone dans les fragments alkyles; phényle; aralkyle, aralcényle et aralcynyle dont le fragment aryle est un phényle et la chaîne alkyle a 1 à 6 atomes de carbone; hétérocyclyle et hétérocyclylalkyle dans lesquels le substituant ou les substituants relatifs aux radicaux précités sont choisis parmi: amino, mono-, di- et tri-alkylamino, hydroxy, alcoxy, mercapto, alkylthio, phénylthio, sulfamoyle, amidino, guanidino, nitro, chloro, bromo, fluoro, cyano et carboxy; et où le ou les hétéro-atomes des fragments hétérocycliques précités sont choisis parmi 1 à 4 atomes d'oxygène, d'azote ou de soufre; et où les fragments alkyles des substituants précités ont 1 à 6 atomes de carbone, comprenant: la cyclisation de:

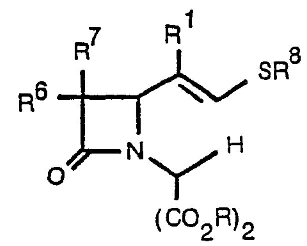

par halogénation et traitement de l'intermédiaire obtenu avec une base pour former:

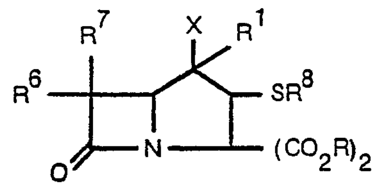

la déshydrohalogénation de l'intermédiaire obtenu en présence d'une base pour former:

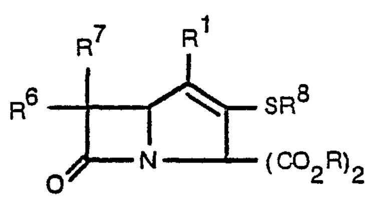

le chauffage de l'intermédiaire obtenu en présence d'un agent de déplacement pour former:

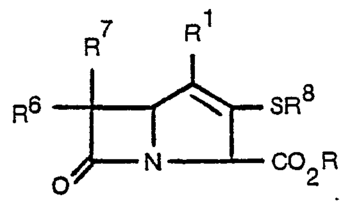

l'isomérisation de la position de la double laision de l'intermédiaire obtenu par traitement dans un solvant avec une base forte pour former:

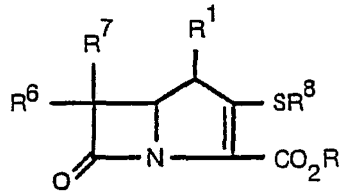

où X est un halogéno.

2. Un procédé pour préparer un composé selon la revendication 1, comprenant la décarboxylation de

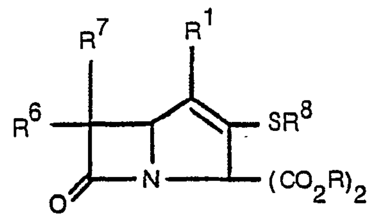

par traitement avec un agent de déplacement et de plus le traitement avec une base capable d'isomériser la double liaison pour former:

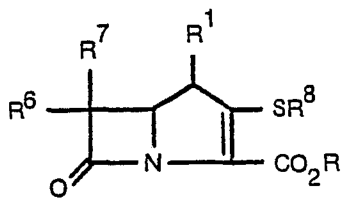

3. Un procédé pour préparer un composé ayant pour structure:

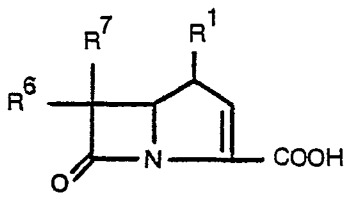

et les sels et esters acceptables en pharmacie correspondants; où

a) les substituants $R^1$, $R^6$ et $R^7$ sont choisis indépendamment parmi un hydrogène ($R^1$ n'est pas H); des radicaux substitués et non substitués: alkyle inférieur droit ou ramifié ayant 1 à 10 atomes de carbone; cycloalkyle ayant 3 à 6 atomes de carbone; cycloalkylalkyle dont le fragment cycloalkyle comprend 3 à 6 atomes de carbone et le fragment alkyle comprend 1 à 10 atomes de carbone; alkylcycloalkyle dont le fragment alkyle comprend 1 à 6 atomes de carbone et le fragment cycloalkyle comprend 3 à 6 atomes de carbone; phényle et naphtyle; phénylalkyle; hétérocyclyle (saturé et non saturé) comprenant des structures mono- et bicycliques ayant 5 à 10 atomes cycliques où un ou plusieurs des hétéroatomes sont choisis parmi l'oxygène, l'azote ou le soufre; hétérocyclylalkyle comprenant les fragments hétérocyclyles justes précités et dont le fragment alkyle comprend 1 à 10 atomes de carbone; le substituant (ou les substituants) des radicaux précités est (sont) choisi(s) parmi amino, hydroxy, cyano, carboxy, nitro, chloro, bromo, fluoro, alcoxy inférieur ayant 1 à 6 atomes de carbone, mercapto, perhalogénoalkyle inférieur, alkylthio inférieur, guanidino, amidino, sulfamoyle et sulfamoyle, amidino et guanidino N-substitués dont le substituant à l'azote est un alkyle inférieur ayant 1 à 6 atomes de carbone ou un aryle ayant 6 à 10 atomes de carbone ou

b) les substituants $R^1$, $R^6$ et $R^7$ sont choisis indépendamment parmi l'hydrogène ($R^1$ n'est pas un hydrogène), les radicaux substitués et non substitués: alkyle, alcényle et alcynyle ayant 1 à 10 atomes de carbone; cycloalkyle, cycloalkylalkyle et alkylcycloalkyle ayant 3 à 6 atomes de carbone dans le cycle cycloalkyle et 1 à 6 atomes de carbone dans les fragments alkyle; phényle; aralkyle, aralcényle et aralcynyle dont le fragment aryle est un phényle et la chaîne linéaire a 1 à 6 atomes de carbone; hétérocyclyle et hétérocyclylalkyle; où le substituant ou les substituants des radicaux précités sont choisis parmi: amino, mono-, di- et trialkylamino, hydroxy, alcoxy, mercapto, alkylthio, phénylthio, sulfamoyle, amidino, guanidino, nitro, chloro, bromo, fluoro, cyano et carboxy; et où le ou les hétéroatomes des fragments hétérocycliques précités sont choisis parmi 1 à 4 atomes d'oxygène, d'azote ou de soufre; et où les fragments alkyles des substituants précités ont 1 à 6 atomes de carbone, comprenant le traitement d'un composé de formule:

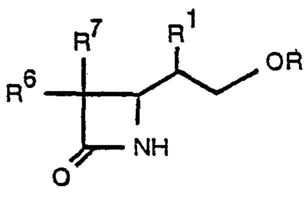

où R est un groupe protecteur facile à éliminer avec un ester glyoxylique dont le fragment ester est un

fragment ester acceptable en pharmacie ou un groupe de blocage facile à éliminer, R', pour fournir un composé ayant pour structure:

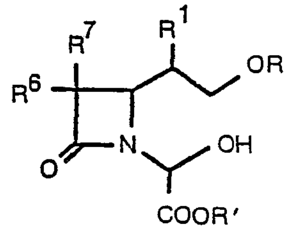

puis une halogénation pour fournir un composé ayant pour structure:

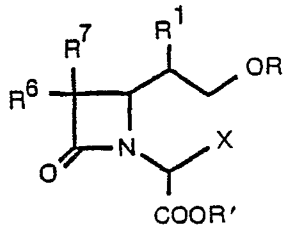

où X est un halogène puis le traitement avec la triphénylphosphine et une hydrolyse aqueuse douce pour fournir:

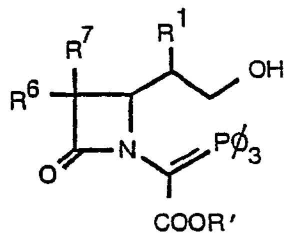

puis la cyclisation de la structure ci-dessus après traitement avec un agent oxydant; puis l'élimination du groupe de blocage.

4. Un procédé pour préparer un composé selon la revendication 3, comprenant la cyclisation de:

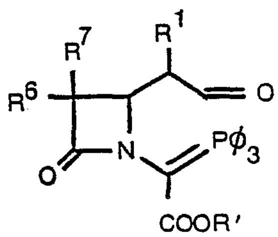

où Ø est phényle et R' est un fragment ester acceptable en pharmacie ou un groupe de blocage facile à éliminer; puis l'élimination du groupe de blocage.

5. Un procédé pour préparer un composé ayant pour formule

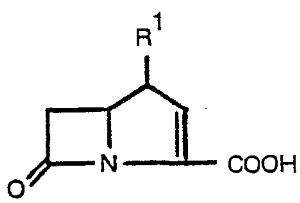

et ses sels et esters acceptables en pharmacie où $R^1$ est choisi parmi le groupe constitué par les radicaux substitués et non substitués alkyle inférieur ayant 1 à 6 atomes de carbone, phényle, phénylalkyle inférieur, cycloalkyle ayant 3 à 6 atomes de carbone, et cycloalkylalkyle ayant 4 à 7 atomes de carbone dans la chaîne et 3 à 6 atomes de carbone dans le cycle; où lesdits substituants de $R^1$ sont choisis parmi chloro, bromo, fluoro, amino, mono-, di- et trialkylamino (chaque alkyle ayant 1 à 6

atomes de carbone), alcoxy, cyano et carboxy, comprenant le traitement d'un composé de formule:

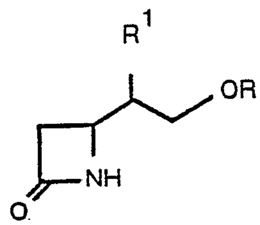

où R est un groupe protecteur facile à éliminer avec un ester glyoxylique dont le fragment ester est un fragment ester acceptable en pharmacie ou un groupe de blocage facile à éliminer, $R^2$, pour fournir un composé ayant pour structure:

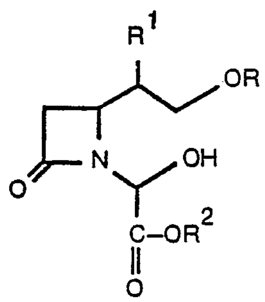

puis une halogénation pour fournir un composé ayant pour structure:

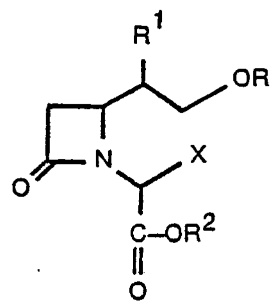

où X est un halogène puis le traitement avec la triphénylphosphine et une hydrolyse aqueuse douce, pour fournir:

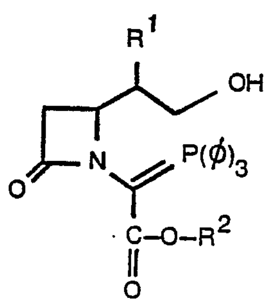

puis la cyclisation de la structure ci-dessus après traitement avec un agent oxydant; puis l'élimination du groupe de blocage.

6. Un procédé pour préparer un composé selon la revendication 5, comprenant la cyclisation de:

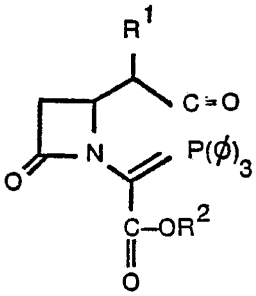

où Ø est un phényle et $R^2$ est un fragment ester acceptable en pharmacie ou un groupe de blocage facile à éliminer; puis l'élimination du groupe de blocage.

7. Un procédé pour préparer un composé selon la revendication 1 ayant pour structure:

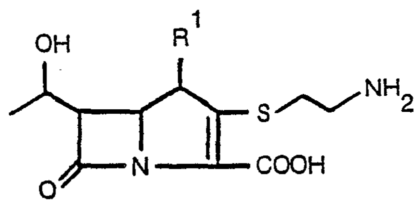

et ses sels acceptables en pharmacie comprenant: la cyclisation de

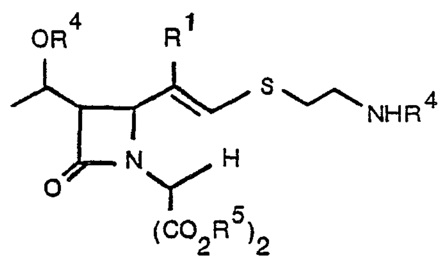

par halogénation et traitement de l'intermédiaire obtenu avec une base pour former:

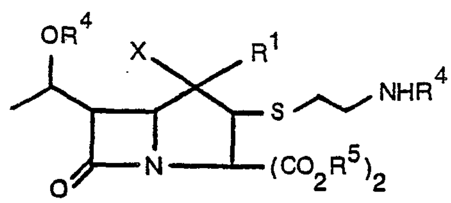

la déshydrohalogénation de l'intermédiaire obtenu en présence d'une base pour former:

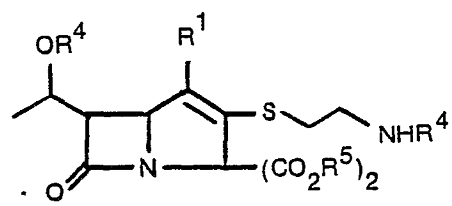

le chauffage de l'intermédiaire obtenu en présence d'un agent de déplacement pour former:

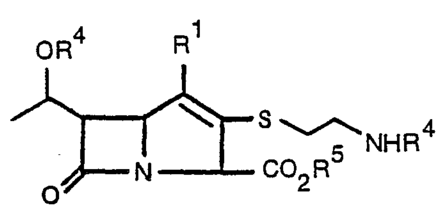

l'isomérisation de la position de la double liaison de l'intermédiaire obtenu par traitement dans un solvant avec une base forte pour former:

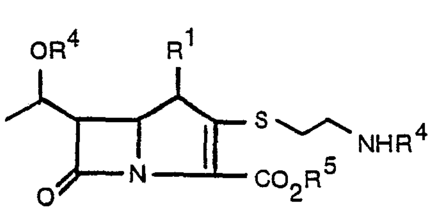 un déblocage pour former: 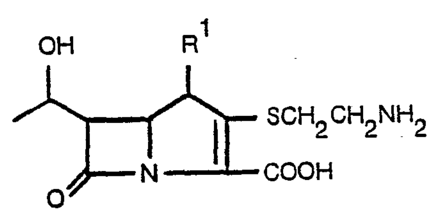

où $R^4$ et $R^5$ sont des groupes de blocage et X est un halogéno.

8. Un procédé selon la revendication 7 pour préparer un composé selon la revendication 1, ayant pour formule:

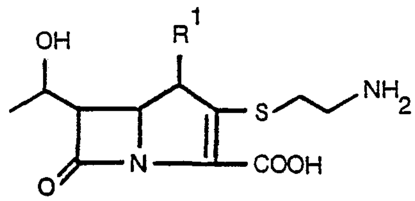

et ses sels acceptables en pharmacie où $R^1$ est choisi parmi les radicaux substitués et non substitués alkyle inférieur ayant 1 à 6 atomes de carbone, phényle, phénylalkyle inférieur, cycloalkyle ayant 3 à 6 atomes de carbone et cycloalkylalkyle ayant 4 à 7 atomes de carbone dans la chaîne et 3 à 6 atomes de carbone dans le cycle; où lesdits substituants de $R^1$ sont choisis parmi chloro, bromo, fluoro, hydroxy, amino, alcoxy ayant 1 à 6 atomes de carbone, cyano et carboxy.

9. Un procédé pour préparer un composé ayant pour formule:

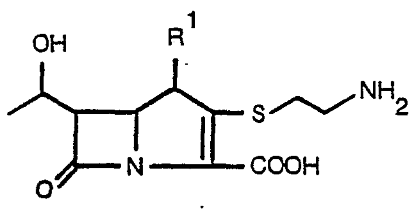

où $R^1$ est choisi parmi les radicaux substitués et non substitués alkyle inférieur ayant 1 à 6 atomes de carbone, phényle, phénylalkyle inférieur, cycloalkyle ayant 3 à 6 atomes de carbone et cycloalkylalkyle ayant 4 à 7 atomes de carbone dans la chaîne et 3 à 6 atomes de carbone dans le cycle; où lesdits substituants de $R^1$ sont choisis parmi chloro, bromo, fluoro, hydroxy, amino, alcoxy ayant 1 à 6 atomes de carbone, cyano et carboxy, comprenant le traitement de:

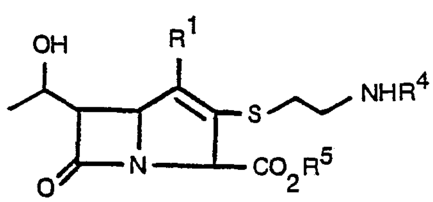

avec une base capable d'isomériser la double liaison pour former:

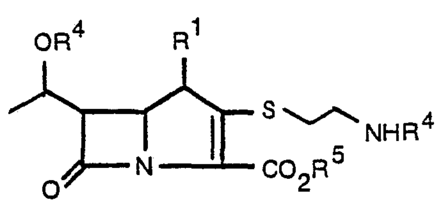

où $R^4$ et $R^5$ sont des groupes de blocage; puis un déblocage.

10. Un procédé pour préparer un composé selon la formule indiquée dans les revendications 1 et 3 où $R^7$ est un hydrogène, $R^1$ est choisi parmi les radicaux substitués et non substitués: alkyle inférieur ayant 1 à 6 atomes de carbone, cyclopropyle, benzyle et phényle; et $R^6$ est un alkyle $\alpha$-substitué dont le substituant en $\alpha$ est un hydroxy, un amino, un mercapto et où le fragment alkyle est droit ou ramifié et comprend 1 à 6 atomes de carbone; le substituant relatif aux radicaux ci-dessus pour $R^1$ est phényle, caractérisé en ce qu'on opère comme décrit dans les revendications 1, 2 ou 3 où $R^1$, $R^6$ et $R^7$ dans les composés de départ sont définis comme ci-dessus.

11. Un procédé pour préparer un composé selon les revendications 1 et 2, où les fragments esters acceptables en pharmacie R sont pivaloyloxyméthyle, allyle, méthallyle, (2-méthylthio)-éthyle, 3-méthyl-2-buténylė, p-tert-butylbenzyle, 5-indanyle et 3-phtalidyle.